# EUROPEAN PATENT SPECIFICATION

(11) **EP 2 766 041 B1**
(45) Date of publication and mention of the grant of the patent: **05.12.2018**
(21) Application number: 12840096.7
(22) Date of filing: 12.10.2012
(51) Int. Cl.: A61K 39/395, A61K 31/7088, C12N 15/11, A61P 7/06

(54) **COMBINATORIAL COMPOSITIONS AND METHODS OF TREATING HEMOGLOBINOPATHIES**
KOMBINATORISCHE ZUSAMMENSETZUNGEN UND VERFAHREN ZUR BEHANDLUNG VON HÄMOGLOBINOPATHIEN
COMPOSITIONS COMBINATOIRES ET MÉTHODES DE TRAITEMENT D'HÉMOGLOBINOPATHIES

(30) Priority: 12.10.2011 US 201161546121 P
(43) Date of publication of application: 20.08.2014
(73) Proprietor: Children's Medical Center Corporation, Boston, MA 02115 (US); Orkin, Stuart H., Brookline, Massachusetts 02445 (US); Xu, Jian, Brookline, Massachusetts 02446 (US); Cong, Peng, Medford, Massachusetts 02155 (US)
(72) Inventor: ORKIN, Stuart H., Brookline Massachusetts 02445 (US); XU, Jian, Brookline Massachusetts 02446 (US); PENG, Cong, Medford Massachusetts 02155 (US)
(74) Representative: Griffin, Philippa Jane
(86) International application number: PCT/US2012/059861
(87) International publication number: WO 2013/055985

(56) References cited:
- WO-A1-2011/072086
- US-A1- 2011 182 867
- XU JIAN ET AL: "Correction of Sickle Cell Disease in Adult Mice by Interference with Fetal Hemoglobin Silencing", SCIENCE (WASHINGTON D C), vol. 334, no. 6058, November 2011 (2011-11), pages 993-996, XP002736464, ISSN: 0036-8075
- XU JIAN; SANKARAN VIJAY G; ESRICK ERICA B; EBERT BENJAMIN L; ORKIN STUART: "Reactivation of Silenced Human HbF In Adult Mice by Inactivation of BCL11A", BLOOD, vol. 116, no. 21, 7 December 2010 (2010-12-07), pages 282-283, XP55172109, US ISSN: 0006-4971
- AKINSHEYE I. ET AL.: 'Fetal hemoglobin in sickle cell anemia.' BLOOD vol. 118, no. 1, 07 July 2011, pages 19 - 27, XP055149541

## Description

### CROSS REFERENCE TO RELATED APPLICATION

This application claims benefit under 35 U.S.C. § 119(e) of U.S. Provisional Application No. 61/546,121 filed October 12, 2011.

### GOVERNMENT SUPPORT

This invention was made with Government support under Grant No.: P01HL032262 and P30DK049216 awarded by the National Institutes of Health. The Government has certain rights in the invention.

### BACKGROUND

Normal adult hemoglobin comprises four globin proteins, two of which are alpha (α) proteins and two of which are beta (β) proteins. During mammalian fetal development, particularly in humans, the fetus produces fetal hemoglobin, which comprises two gamma (γ)-globin proteins instead of the two β-globin proteins. At some point during fetal development or infancy, depending on the particular species and individual, a globin switch occurs, referred to as the "fetal switch", at which point, erythrocytes in the fetus switch from making predominantly γ-globin to making predominantly β-globin. The developmental switch from production of predominantly fetal hemoglobin or HbF (α₂γ₂) to production of adult hemoglobin or HbA (α₂β₂) begins at about 28 to 34 weeks of gestation and continues shortly after birth until HbA becomes predominant. This switch results primarily from decreased transcription of the γ-globin genes and increased transcription of β-globin genes. On average, the blood of a normal adult contains only about 2% HbF.

Sometimes, there are defects in the adult globin proteins expressed or there are defects in expressing all the four adult globin proteins. Hemoglobinopathies encompass a number of anemias of genetic origin in which there is a decreased production and/or increased destruction (hemolysis) of red blood cells (RBCs). These also include genetic defects that result in the production of abnormal hemoglobins with a concomitant impaired ability to maintain oxygen concentration. Some such disorders involve the failure to produce normal β-globin in sufficient amounts, while others involve the failure to produce normal β-globin entirely. These disorders associated with the β-globin protein are referred to generally as β-hemoglobinopathies. For example, β-thalassemias result from a partial or complete defect in the expression of the β-globin gene, leading to deficient or absent HbA. Sickle cell anemia results from a point mutation in the β-globin structural gene, leading to the production of an abnormal (sickled) hemoglobin (HbS). HbS RBCs are more fragile than normal RBCs and undergo hemolysis more readily, leading eventually to anemia.

In addressing these hemoglobinopathies, the current treatment objective is aimed at the reduction of globin chain imbalances in patients with β-hemoglobinopathies by manipulating the expression of the fetal hemoglobin (α2γ2; HbF). The therapeutic potential of such approaches is indicated by observations of the mild phenotype of individuals with co-inheritance of both homozygous β-thalassemia and hereditary persistence of fetal hemoglobin (HPFH), as well as by those patients with homozygous β-thalassemia who synthesize no adult hemoglobin, but in whom a reduced requirement for transfusions is observed in the presence of increased concentrations of fetal hemoglobin. Furthermore, it has been observed that certain populations of adult patients with β chain abnormalities have higher than normal levels of fetal hemoglobin (HbF), and have been observed to have a milder clinical course of disease than patients with normal adult levels of HbF. For example, a group of Saudi Arabian sickle-cell anemia patients who express 20-30% HbF have only mild clinical manifestations of the disease. It is now accepted that hemoglobin disorders, such as sickle cell anemia and the β-thalassemias, are ameliorated by increased HbF production.

The switch from fetal hemoglobin to adult hemoglobin (α2γ2; HbA) usually proceeds within six months after parturition. However, in the majority of patients with β-hemoglobinopathies, the upstream γ globin genes are intact and fully functional, so that if these genes become reactivated, functional hemoglobin synthesis could be maintained during adulthood, and thus ameliorate disease severity.

There are also experimental evidences supporting the feasibility of reactivation of fetal hemoglobin production. When peripheral blood that contain clonogenic cells were given the appropriate combination of growth factors, it was shown that it produces erythroid burst-forming unit. Individual cells in such colonies can accumulate fetal hemoglobin (HbF), adult hemoglobin (HbA) or a combination of both. In cultures from adult blood, nucleated red cells accumulate either HbA (F-A+) only, or a combination of HbF and HbA (F+A+). Importantly, individual colonies contain both F+ and F- cells, indicating that both types are progeny from the same circulating stem cells. Thus, during the early stages of development in culture, cells execute an option as to whether or not to express HbF. The proportion of adult F+ cells developing in culture does not appear to be preprogrammed *in vivo,* but appears to depend on culture conditions: A shift into the combined HbF and HbA expression pathway can, for example, be achieved *in vitro* by high serum concentrations, due to the activity of an unidentified compound that can be absorbed on activated charcoal.

US 2011/182867 describes modulation of BCL11a for treatment of hemoglobinopathies. Akinsheye et al. Blood, 2011, 7 July; Vol 118(1), pages 19-27, describes fetal hemoglobin in sickle cell anemia. Xu Jian et al., Science, Vol. 334, No. 6058, November 2011, pages 993-996, describes correction of sickle cell disease in adult mice by interference with fetal hemoglobin silencing. Xu Jian et al., Blood, Vol. 116(21), 7 December 2010, pages 282-283, describes reactivation fo silenced human HbF in adult mice by inactivation of BCL11A.

### SUMMARY

Provided herein are combinatorial compositions and methods for increasing fetal hemoglobin expression (HbF) for the treatment and/or amelioration of the symptoms of hemoglobinpathy in a mammal. These compositions comprise a combination of inhibitors of BCL11A expression or activity with epigenetic modifiers, such as inhibitors of DNA methylation and/or inhibitors of histone deactylases. As demonstrated herein, such combinations provide unexpected and synergistic effects on increasing fetal hemoglobin expression, thereby providing novel therapies for disorders associated with decreased/absent fetal hemoglobin, such as hemoglobinopathies.

Accordingly, in some aspects, provided herein are pharmaceutical compositions for increasing fetal hemoglobin levels in a mammal in need thereof. These compositions comprise at least one inhibitor of BCL11A and at least one epigenetic modifier in a pharmaceutically acceptable carrier.

In some embodiments of these aspects and all such aspects described herein, the inhibitor of BCL11A inhibits BCL11A expression and/or inhibits BCL11A activity.

In some embodiments of these aspects and all such aspects described herein, the inhibitor of BCL11A expression is an antibody specific for BCL11A or an antigen-binding fragment thereof, a small molecule, or a BCL11A specific nucleic acid. In some such embodiments, the BCL11A specific nucleic acid is a BCL11A specific RNA interference agent, a vector encoding a BCL11A specific RNA interference agent, or an aptamer that binds BCL11A. In some such embodiments, the RNA interference agent comprises one or more of the nucleotide sequences selected from the group consisting of SEQ ID NO: 3-8.

In some embodiments of these aspects and all such aspects described herein, the at least one epigenetic modifier is a DNA methylation inhibitor. In some such embodiments, the DNA methylation inhibitor is 5-aza-2'-deoxycytidine.

In some embodiments of these aspects and all such aspects described herein the epigenetic modifier is an HDAC inhibitor. In some such embodiments, the HDAC inhibitor is suberoylanilide hydroxamic acid (SAHA or Vorinostat).

Also provided herein are methods for increasing fetal hemoglobin levels in a mammal in need thereof. These methods comprise administering to a mammal a therapeutically effective amount of a composition comprising at least one inhibitor of BCL11A and at least one epigenetic modifier in a pharmaceutically acceptable carrier, as described herein, whereby fetal hemoglobin expression is increased in the mammal, relative to fetal hemoglobin expression prior to the administration.

In some embodiments of these aspects and all such aspects described herein, the mammal has been diagnosed with or is at risk for a hemoglobinopathy. In one embodiment, the hemoglobinopathy is a β-hemoglobinopathy. In one embodiment, the hemoglobinopathy is sickle cell disease. In another embodiment, the hemoglobinopathy is β-thalassemia.

In some embodiments of these aspects and all such aspects described herein, the method further comprises first contacting a hematopoietic progenitor cell with the composition comprising at least one inhibitor of BCL11A and at least one epigenetic modifier in a pharmaceutically acceptable carrier *ex vivo* or *in vitro,* and administering the contacted cell or its progeny to the mammal.

In some embodiments of these aspects and all such aspects described herein, the composition is administered by injection, infusion, instillation, or ingestion.

Also provided herein in other aspects are methods of treating a blood disorder in a mammal in need thereof. These methods comprise administering to the mammal a therapeutically effective amount of a composition comprising at least one inhibitor of BCL11A and at least one epigenetic modifier in a pharmaceutically acceptable carrier, as described herein, whereby fetal hemoglobin expression is increased in the mammal, relative to fetal hemoglobin expression prior to the administration.

In another embodiment, provided herein is a method of treating a blood disorder or increasing fetal hemoglobin level in a mammal in need thereof comprising providing a hematopoietic progenitor cell and contacting the cell with a therapeutically effective amount of a composition comprising at least one inhibitor of BCL11A and at least one epigenetic modifier in a pharmaceutically acceptable carrier, as described herein, whereby fetal hemoglobin expression is increased in the cell, relative to fetal hemoglobin expression prior to the contacting. In some embodiments, the contact in *ex vivo* or *in vitro.*

In one embodiment, the hematopoietic progenitor cell in derived from the mammal, meaning the cell is autologous to the mammal.

In one embodiment, the hematopoietic progenitor cell in derived from another mammal. In one embodiment, the another mammal is at the minimum HLA-match with the mammal needing treatment or needing increased fetal hemoglobin expression.

In one embodiment, the methods further comprise re-introduced back into the mammal.

In some embodiments of these aspects and all such aspects described herein, the blood disorder is a hemoglobinopathy. In one embodiment, the hemoglobinopathy is a β-hemoglobinopathy. In another embodiment, the hemoglobinopathy is sickle cell disease. In another embodiment, the hemoglobinopathy is β-thalassemia.

In some embodiments of these aspects and all such aspects described herein, the method further comprises administering an additional therapeutic agent for the blood disorder.

Also provided herein in some aspects are methods for increasing fetal hemoglobin levels in a cell. These methods comprise the steps of contacting a hematopoietic progenitor cell with an effective amount of a composition comprising at least one inhibitor of BCL11A and at least one epigenetic modifier, as described herein, whereby fetal hemoglobin expression is increased in the cell, or its progeny, relative to fetal hemoglobin expression in the cell prior to the contacting.

In some embodiments of these aspects and all such aspects described herein, the hematopoietic progenitor cell is a cell of the erythroid lineage.

In some embodiments of these aspects and all such aspects described herein, the hematopoietic progenitor cell is contacted *ex vivo* or *in vitro.*

### Definitions

For convenience, certain terms employed herein, in the specification, examples and appended claims are collected here. Unless stated otherwise, or implicit from context, the following terms and phrases include the meanings provided below. Unless explicitly stated otherwise, or apparent from context, the terms and phrases below do not exclude the meaning that the term or phrase has acquired in the art to which it pertains. The definitions are provided to aid in describing particular embodiments, and are not intended to limit the claimed invention, because the scope of the invention is limited only by the claims. Unless otherwise defined, all technical and scientific terms used herein have the same meaning as commonly understood by one of ordinary skill in the art to which this invention belongs.

An "inhibitor of BCL11A" or "BCL11A inhibitor" refers to an agent or compound, that inhibits one or more downstream effector pathways mediated by BCL11A, including fetal hemoglobin expression, as the term is used herein, and can function in a competitive or non-competitive manner, and can function, in some embodiments, by interfering with the expression of the BCL11A protein. A BCL11A inhibitor includes any chemical or biological entity that, upon treatment of a cell, results in inhibition of the biological activity caused by activation of BCL11A in response to cellular signals. BCL11A inhibitors, include, but are not limited to, small molecules, antibodies or antigen-binding antibody fragments, intrabodies, aptamers, antisense constructs, RNA interference agents, and ribozymes. Thus, the term BCL11A inhibitor refers to an agent that inhibits expression of the BCL11A polypeptide or polynucleotide encoding BCL11A, or one that binds to, partially or totally blocks stimulation, decreases, prevents, delays activation, inactivates, desensitizes, or down regulates the activity of the BCL11A polypeptide or polynucleotide encoding BCL11A, such that the agent causes an increase in fetal hemoglobin expression. Such BCL11A inhibitors can *e.g.,* inhibit BCL11A expression, *e.g.,* BCL11A translation, post-translational processing of BCL11A, stability, degradation, or nuclear localization of the BCL11A polypeptide, or transcription, post transcriptional processing, stability or degradation of a polynucleotide encoding BCL11A, or bind to, partially or totally block stimulation, DNA binding, or transcription factor activity of BCL11A.

As used herein, "antibody inhibitors of BCL11A" include complete immunoglobulins, antigen binding fragments of immunoglobulins, as well as antigen-binding fragments that comprise antigen binding domains of immunoglobulins. "Antigen-binding fragments" of immunoglobulins include, for example, Fab, Fab', F(ab')2, scFv and dAbs. Modified antibody formats have been developed which retain binding specificity, but have other characteristics that may be desirable, including for example, bispecificity, multivalence (more than two binding sites), and compact size (e.g., binding domains alone).

A "nucleic acid", as described herein, can be RNA or DNA, and can be single or double stranded, and can be selected, for example, from a group including: nucleic acid encoding a protein of interest, oligonucleotides, nucleic acid analogues, for example peptide- nucleic acid (PNA), pseudo-complementary PNA (pc-PNA), locked nucleic acid (LNA) etc. Such nucleic acid sequences include, for example, but are not limited to, nucleic acid sequence encoding proteins, for example that act as transcriptional repressors, antisense molecules, ribozymes, small inhibitory nucleic acid sequences, for example but are not limited to RNAi, shRNAi, siRNA, micro RNAi (mRNAi), antisense oligonucleotides etc. In one embodiment, the nucleic acid is a single stranded nucleic acid. In another embodiment, the nucleic acid is a double stranded nucleic acid.

The terms "RNA interference agent" and "RNA interference" as they are used herein are intended to encompass those forms of gene silencing mediated by double-stranded RNA, regardless of whether the RNA interfering agent comprises an siRNA, miRNA, shRNA or other double-stranded RNA molecule. "Short interfering RNA" (siRNA), also referred to herein as "small interfering RNA" is defined as an RNA agent which functions to inhibit expression of a target gene, *e.g.,* by RNAi. An siRNA may be chemically synthesized, may be produced by *in vitro* transcription, or may be produced within a host cell. In one embodiment, siRNA is a double stranded RNA (dsRNA) molecule of about 15 to about 40 nucleotides in length, preferably about 15 to about 28 nucleotides, more preferably about 19 to about 25 nucleotides in length, and more preferably about 19, 20, 21, 22, or 23 nucleotides in length, and may contain a 3' and/or 5' overhang on each strand having a length of about 0, 1, 2, 3, 4, or 5 nucleotides. The length of the overhang is independent between the two strands, *i.e.,* the length of the overhang on one strand is not dependent on the length of the overhang on the second strand. Preferably the siRNA is capable of promoting RNA interference through degradation or specific post-transcriptional gene silencing (PTGS) of the target messenger RNA (mRNA). In another embodiment, "RNA interference agent" and "RNA interference" encompass single stranded RNA. In another embodiment, "RNA interference agent" and "RNA interference" encompass double stranded RNA.

As used herein, the term "small molecule" refers to a chemical agent including, but not limited to, peptides, peptidomimetics, amino acids, amino acid analogs, polynucleotides, polynucleotide analogs, aptamers, nucleotides, nucleotide analogs, organic or inorganic compounds (*i.e.,* including heteroorganic and organometallic compounds) having a molecular weight less than about 10,000 grams per mole, organic or inorganic compounds having a molecular weight less than about 5,000 grams per mole, organic or inorganic compounds having a molecular weight less than about 1,000 grams per mole, organic or inorganic compounds having a molecular weight less than about 500 grams per mole, and salts, esters, and other pharmaceutically acceptable forms of such compounds.

As used herein, a "chemical inducer of HbF" or an "HbF chemical inducer" refers to an agent or compound that increases the expression and/or production fetal hemoglobin. Such chemical inducers include, but are not limited to, DNA methylation inhibitors, HDAC inhibitors, amide analogues of trichostatin A, hydroxamic acid analogues of trapoxin or scriptaid and analogues, cytotoxic chemotherapeutic agents, erythropoietin (EPO) preparations, and short chain fatty acids (SCFAs) and derivatives (SCFADs), etc.

As used herein, an "epigenetic modifier" refers to a HbF chemical inducer that acts to increase the expression and/or production of fetal hemoglobin by impacting or modulating one or more epigenetic pathways, such as, for example, histone deacetylation or DNA methylation. Accordingly, as used herein, epigenetic modifiers include agents or compounds that inhibit DNA methylation and histone deacetylation.

As used herein, the terms "DNA methylation inhibitor" or "DNA methyltransferase inhibitor" refer to an agent or compound that directly or indirectly perturbs, disrupts, blocks, modulates or inhibits the methylation of DNA, including the ionic, salt, solvate, isomers, tautomers, N-oxides, ester, prodrugs, isotopes and protected forms thereof, for example, by preventing or antagonizing activity of DNA methyltransferases, or by acting as analogs of the nucleoside deoxycytidine.

As used herein, a "histone deacetylation (HDAC) inhibitor" or "inhibitor of histone deacetylation (HDAC)" refers to a compound which inhibits or modulates the activity of histone deacetylases (HDAC), including the ionic, salt, solvate, isomers, tautomers, N-oxides, ester, prodrugs, isotopes and protected forms thereof. Inhibitors of HDACs for use in the compositions and methods described herein include, but are not limited to, the following structural classes: 1) hydroxamic acids, 2) cyclic peptides, 3) benzamides, and 4) short-chain fatty acids.

In connection with treating a subject or contacting a cell, "increasing the fetal hemoglobin levels" in a subject or cell indicates that fetal hemoglobin is at least 5% higher in the treated subject or cell populations, than in a comparable, control subject or cell population, wherein no combinatorial composition is present. It is preferred that the percentage of fetal hemoglobin expression in a subject or cell population treated with a combinatorial composition is at least 10% higher, at least 20% higher, at least 30% higher, at least 40% higher, at least 50% higher, at least 60% higher, at least 70% higher, at least 80% higher, at least 90% higher, at least 1-fold higher, at least 2-fold higher, at least 5-fold higher, at least 10 fold higher, at least 25 fold higher, at least 50 fold higher ,at least 100 fold higher, at least 1000-fold higher, or more than a control treated cell population of comparable size and culture conditions, or the subject in the absence of the treatment or a control, non-treated subject having the same blood disorder. The term "control treated population" is used herein to describe a population of cells that has been treated with identical media, viral induction, nucleic acid sequences, temperature, confluency, flask size, pH, etc., with the exception of the addition of the combinatorial composition.

The terms "subject" and "individual" are used interchangeably herein, and refer to an animal, for example a human, recipient of the combinatorial composition comprising inhibitors of BCL11A expression or activity and one or more epigenetic modifiers described herein. For treatment of those disease states which are specific for a specific animal, such as a human subject, the term "subject" refers to that specific animal. The terms 'non-human animals' and 'non-human mammals' are used interchangeably herein, and include mammals such as rats, mice, rabbits, sheep, cats, dogs, cows, pigs, and non-human primates. A subject can be an adult subject, an adolescent subject, a child subject, a neonate subject, an infant subject, or an *in utero* subject.

The term "blood disorders" as used herein includes hemoglobinopathies and thalassemias. A "blood disorder" is any disorder of the blood and blood-forming organs, and refers to any disease or malady that can be characterized as a direct or indirect consequence of a defect or disease of hemoglobin producing cells or the production of hemoglobin. The term blood disorder includes nutritional anemias (*e*.*g*., iron deficiency anemia, sideropenic dysphasia, Plummer-Vinson syndrome, vitamin B12 deficiency anemia, vitamin B 12 deficiency anemia due to intrinsic factor, pernicious anemia, folate deficiency anemia, and other nutritional anemias), myelodysplastic syndrome, bone marrow failure or anemia resulting from chemotherapy, radiation or other agents or therapies, hemolytic anemias (*e*.*g*., anemia due to enzyme disorders, anemia due to phosphate dehydrogenase (G6PD) deficiency, favism, anemia due to disorders of glutathione metabolism, anemia due to disorders of glycolytic enzymes, anemias due to disorders of nucleotide metabolism and anemias due to unspecified enzyme disorder), thalassemia, α-thalassemia, β-thalassemia, δβ-thalassemia, thalassemia trait, hereditary persistence of fetal hemoglobin (HPFP), and other thalassemias, sickle cell disorders (sickle cell anemia with crisis, sickle cell anemia without crisis, double heterozygous sickling disorders, sickle cell trait and other sickle cell disorders), hereditary hemolytic anemias (hereditary spherocytosis, hereditary elliptocytosis, other hemoglobinopathies and other specified hereditary hemolytic anemias, such as stomatocyclosis), acquired hemolytic anemia (*e*.*g*., drug-induced autoimmune hemolytic anemia, other autoimmune hemolytic anemias, such as warm autoimmune hemolytic anemia, drug-induced non-autoimmune hemolytic anemia, hemolytic-uremic syndrome, and other non-autoimmune hemolytic anemias, such as microangiopathic hemolytic anemia); aplastic anemias (*e*.*g*., acquired pure red cell aplasia (erythoblastopenia), other aplastic anemias, such as constitutional aplastic anemia and fanconi anemia, acute posthemorrhagic anemic, and anemias in chronic diseases), coagulation defects (*e*.*g*., disseminated intravascular coagulation (difibrination syndrome)), hereditary factor VIII deficiency (hemophilia A), hereditary factor IX deficiency (Christmas disease), and other coagulation defects such as Von Willebrand's disease, hereditary factor Xi deficiency (hemophilia C), purpura (*e*.*g*., qualitative platelet defects and Glanzmann's disease), neutropenia, agranulocytosis, functional disorders of polymorphonuclear neutrophils, other disorders of white blood cells (*e*.*g*., eosinophilia, leukocytosis, lymophocytosis, lymphopenia, monocytosis, and plasmacytosis), diseases of the spleen, methemoglobinemia, other diseases of blood and blood forming organs (*e*.*g*., familial erythrocytosis, secondary polycythemia, essential thrombocytosis and basophilia), thrombocytopenia, infectious anemia, hypoproliferative or hypoplastic anemias, hemoglobin C, D and E disease, hemoglobin lepore disease, and HbH and HbS diseases, anemias due to blood loss, radiation therapy or chemotherapy, or thrombocytopenias and neutropenias due to radiation therapy or chemotherapy, sideroblastic anemias, myelophthisic anemias, antibody-mediated anemias, and certain diseases involving lymphoreticular tissue and reticulohistiocytic system (*e*.*g*., Langerhans' cell hystiocytosis, eosinophilic granuloma, Hand-Schuller-Christian disease, hemophagocytic lymphohistiocytosis, and infection-associated hemophagocytic syndrome).

"Hematopoietic progenitor cell" as the term is used herein, refers to cells of a stem cell lineage that give rise to all the blood cell types including the myeloid (monocytes and macrophages, neutrophils, basophils, eosinophils, erythrocytes, megakaryocytes/platelets, dendritic cells), and the lymphoid lineages (T-cells, B-cells, NK-cells). A "cell of the erythroid lineage" indicates that the cell being contacted is a cell that undergoes erythropoeisis such that upon final differentiation it forms an erythrocyte or red blood cell (RBC). Such cells belong to one of three lineages, erythroid, lymphoid, and myeloid, originating from bone marrow haematopoietic progenitor cells. Upon exposure to specific growth factors and other components of the haematopoietic microenvironment, haematopoietic progenitor cells can mature through a series of intermediate differentiation cellular types, all intermediates of the erythroid lineage, into RBCs. Thus, cells of the "erythroid lineage", as the term is used herein, comprise hematopoietic progenitor cells, rubriblasts, prorubricytes, erythroblasts, metarubricytes, reticulocytes, and erythrocytes.

As used herein, the terms "administering," refers to the placement of a combinatorial compostion comprising one or more BCL11A inhibitors and one or more HbF chemical inducers into a subject by a method or route which results in at least partial localization of the agent at a desired site, *e.g.,* bone marrow. The agent can be administered by any appropriate route which results in an effective treatment in the subject.

As used herein the term "comprising" or "comprises" is used in reference to compositions, methods, and respective component(s) thereof, that are essential to the invention, yet open to the inclusion of unspecified elements, whether essential or not.

As used herein the term "consisting essentially of" refers to those elements required for a given embodiment. The term permits the presence of additional elements that do not materially affect the basic and novel or functional characteristic(s) of that embodiment of the invention.

The term "consisting of" refers to compositions, methods, and respective components thereof as described herein, which are exclusive of any element not recited in that description of the embodiment.

As used in this specification and the appended claims, the singular forms "a," "an," and "the" include plural references unless the context clearly dictates otherwise. Thus for example, references to "the method" includes one or more methods, and/or steps of the type described herein and/or which will become apparent to those persons skilled in the art upon reading this disclosure and so forth.

Other than in the operating examples, or where otherwise indicated, all numbers expressing quantities of ingredients or reaction conditions used herein should be understood as modified in all instances by the term "about." The term "about" when used in connection with percentages can mean ±1%.

Unless otherwise defined herein, scientific and technical terms used in connection with the present application shall have the meanings that are commonly understood by those of ordinary skill in the art to which this disclosure belongs. It should be understood that this invention is not limited to the particular methodology, protocols, and reagents, etc., described herein and as such can vary. The terminology used herein is for the purpose of describing particular embodiments only, and is not intended to limit the scope of the present invention, which is defined solely by the claims. Definitions of common terms in immunology, and molecular biology can be found in The Merck Manual of Diagnosis and Therapy, 18th Edition, published by Merck Research Laboratories, 2006 (ISBN 0-911910-18-2); Robert S. Porter et al. (eds.), The Encyclopedia of Molecular Biology, published by Blackwell Science Ltd., 1994 (ISBN 0-632-02182-9); and Robert A. Meyers (ed.), Molecular Biology and Biotechnology: a Comprehensive Desk Reference, published by VCH Publishers, Inc., 1995 (ISBN 1-56081-569-8); Immunology by Werner Luttmann, published by Elsevier, 2006. Definitions of common terms in molecular biology are found in Benjamin Lewin, Genes IX, published by Jones & Bartlett Publishing, 2007 (ISBN-13: 9780763740634); Kendrew et al. (eds.), The Encyclopedia of Molecular Biology, published by Blackwell Science Ltd., 1994 (ISBN 0-632-02182-9); and Robert A. Meyers (ed.), Maniatis et al., Molecular Cloning: A Laboratory Manual, Cold Spring Harbor Laboratory Press, Cold Spring Harbor, N.Y., USA (1982); Sambrook et al., Molecular Cloning: A Laboratory Manual (2 ed.), Cold Spring Harbor Laboratory Press, Cold Spring Harbor, N.Y., USA (1989); Davis et al., Basic Methods in Molecular Biology, Elsevier Science Publishing, Inc., New York, USA (1986); or Methods in Enzymology: Guide to Molecular Cloning Techniques Vol.152, S. L. Berger and A. R. Kimmerl Eds., Academic Press Inc., San Diego, USA (1987); Current Protocols in Molecular Biology (CPMB) (Fred M. Ausubel, et al. ed., John Wiley and Sons, Inc.), Current Protocols in Protein Science (CPPS) (John E. Coligan, et. al., ed., John Wiley and Sons, Inc.) and Current Protocols in Immunology (CPI) (John E. Coligan, et. al., ed. John Wiley and Sons, Inc.). Unless otherwise stated, exemplary embodiments described herein were performed using standard procedures known to one skilled in the art, for example, in Maniatis et al., Molecular Cloning: A Laboratory Manual, Cold Spring Harbor Laboratory Press, Cold Spring Harbor, N.Y., USA (1982); Sambrook et al., Molecular Cloning: A Laboratory Manual (2 ed.), Cold Spring Harbor Laboratory Press, Cold Spring Harbor, N.Y., USA (1989); Davis et al., Basic Methods in Molecular Biology, Elsevier Science Publishing, Inc., New York, USA (1986); Current Protocols in Molecular Biology (CPMB) (Fred M. Ausubel, et al. ed., John Wiley and Sons, Inc.), Current Protocols in Immunology (CPI) (John E. Coligan, et. al., ed. John Wiley and Sons, Inc.), Current Protocols in Cell Biology (CPCB) (Juan S. Bonifacino et. al. ed., John Wiley and Sons, Inc.), Culture of Animal Cells: A Manual of Basic Technique by R. Ian Freshney, Publisher: Wiley-Liss; 5th edition (2005), Animal Cell Culture Methods (Methods in Cell Biology, Vol. 57, Jennie P. Mather and David Barnes editors, Academic Press, 1st edition, 1998), Methods in Molecular biology, Vol.180, Transgenesis Techniques by Alan R. Clark editor, second edition, 2002, Humana Press, Methods in Meolcular Biology, Vo. 203, 2003, and Transgenic Mouse, editored by Marten H. Hofker and Jan van Deursen.

It should be understood that this technology is not limited to the particular methodology, protocols, and reagents, etc., described herein and as such may vary. The terminology used herein is for the purpose of describing particular embodiments only, and is not intended to limit the scope of the present invention, which is defined solely by the claims.

Patents and patent publications are identified herein for the purpose of describing and disclosing, for example, the methodologies described in such publications that might be used in connection with the present invention. These publications are provided solely for their disclosure prior to the filing date of the present application. Nothing in this regard should be construed as an admission that the inventors are not entitled to antedate such disclosure by virtue of prior invention or for any other reason. All statements as to the date or representation as to the contents of these documents is based on the information available to the applicants and does not constitute any admission as to the correctness of the dates or contents of these documents.

### BRIEF DESCRIPTION OF THE DRAWINGS

**Figures 1A-1E** show BCL11A loss in adult mice reverses γ-silencing.
**Figure 1A** shows the expression of BCL11A protein in CD71⁺Ter119⁺ fetal liver (FL) and bone marrow (BM) cells of control (EpoR-Cre-) and BCL11A knockout (EpoR-Cre+) β-YAC mice. GAPDH was analyzed as a loading control.
**Figure 1B** shows the expression of human fetal (γ) globin genes as monitored by qRT-PCR in FL cells (E12.5 to E18.5) or peripheral blood of postnatal animals (1- to 30-weeks old). Data is plotted as percentage of γ-globin over total β-like human globin gene levels in control (EpoR-Cre-) and BCL11A knockout (EpoR-Cre+) β-YAC mice (n ≥ 4/genotype at each time point). Results are means + SD. All γ-globin levels for the different genotypes are significantly different (P < 1 x 10⁻⁵, two-tailed t-test).
**Figure 1C** shows the immunohistochemical (IHC) stainings for HbF performed on E16.5 FLs from EpoR-Cre- and EpoR-Cre+ animals.
**Figure 1D** shows the transcriptional profiling of control (*Bcl11a*^{+/+}) and *Bcl11a*^{*-*/*-*} (*EpoR-Cre*+) CD71⁺Ter119⁺ erythroid cells (n = 3/genotype). Probes corresponding to mouse α- and β-globin genes were indicated by arrows. Hba-x, ζ-globin; Hba-a1/a2, α-globin; Hbb-y, εy-globin; *Hbb-bhl,* βh1-globin; *Hbb-b1lb2,* β-globin.
**Figure 1E** shows the expression of human γ-globin genes as monitored by qRT-PCR in control (Mx1-Cre-) and BCL11A knockout (Mx1-Cre+) mice before and after pIpC (n ≥ 4/genotype at each time point; *P < 1 × 10⁻⁵).
**Figures 2A-2D** demonstrate that DNA demethylation and HDAC inhibition enhance residual γ-globin expression.

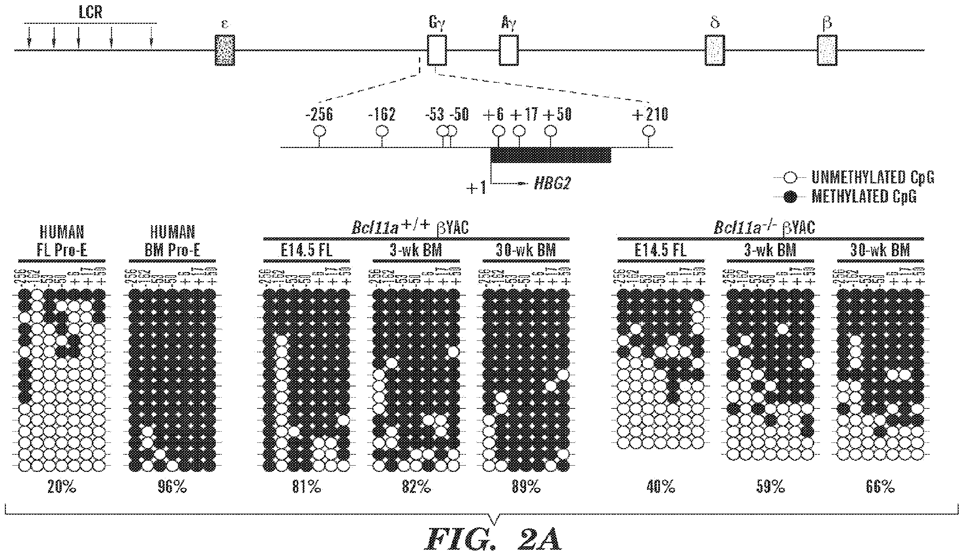

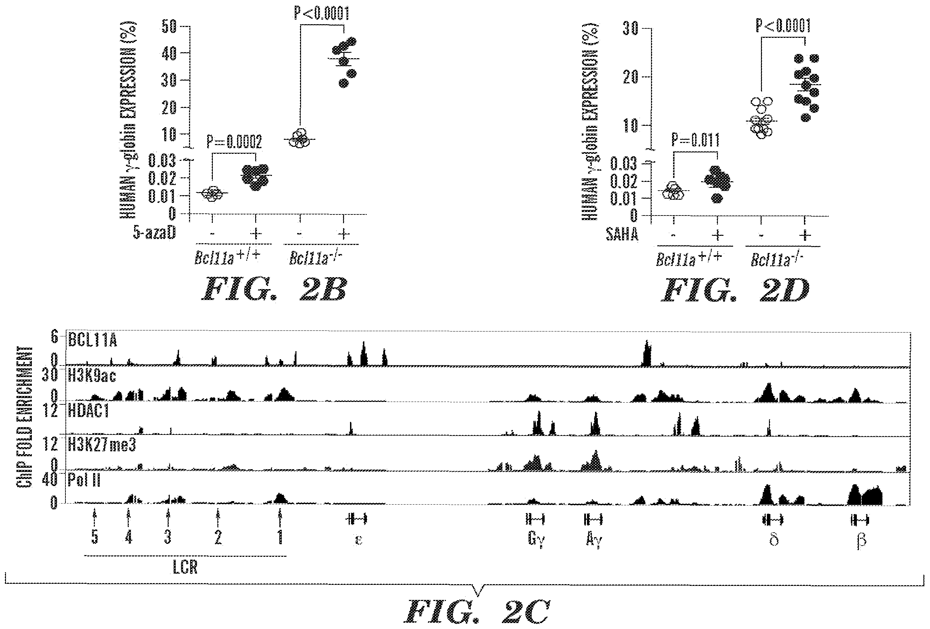

**Figure 2A** shows the CpG methylation within the Gy promoter as measured by bisulfite sequencing analysis in CD71+Ter119+ erythroid progenitors in control (*Bcl11a*^{+/+}) and *Bcl11a*^{*-*/*-*} (EpoR-Cre+) β-YAC mice at various development stages. Human primary FL and BM proerythroblasts (Pro-E) were analyzed as controls. The percentage of methylated CpG dinucleotides is shown for each sample. A diagram of the human β-globin cluster is shown on the top. **Figure 2B** shows the expression of human γ-globin genes as monitored by qRT-PCR in control (*Bcl11a*^{+/+}) and *Bcl11a*^{*-*/*-*} (EpoR-Cre+) β-YAC mice before and after 5-aza-2'-deoxycytidine (5-azaD) treatment (n = 6/genotype/treatment).
**Figure 2C** shows the *in vivo* chromatin occupancy of BCL11A, acetyl-H3 lysine 9 (H3K9ac), HDAC1, trimethyl-H3 lysine 27 (H3K27me3), and RNA Pol II was determined by ChIP-chip in primary human erythroid progenitors. A genome browser representation of binding patterns at the human β-globin cluster is shown.
**Figure 2D** shows the expression of human γ-globin genes as monitored by qRT-PCR in control (*Bcl11a*^{+/+}*,* n=8) and *Bcl11a*^{*-*/*-*} (EpoR-Cre+, n=12) β-YAC mice before and after suberoylanilide hydroxamic acid (SAHA) treatment.

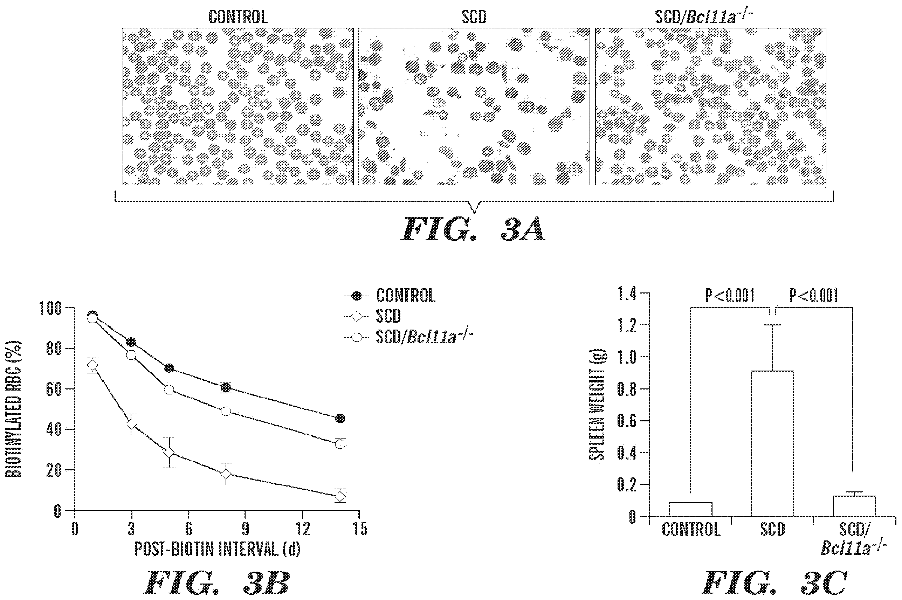

**Figures 3A-3E** demonstrate that the inactivation of BCL11A rescues sickle cell defects in humanized SCD mice.
**Figure 3A** shows the representative blood smears of control, SCD and SCD/*Bcl11a*^{*-*/*-*} mice are shown at 1000x magnification.
**Figure 3B** shows the RBC lifespan is significantly extended in *SCD*/*Bcl11a*^{*-*/*-*} mice at every time point compared to SCD mice (n ≥ 4; P < 0.01). Results are means ± SEM.
**Figure 3C** shows the correction of splenomegaly in SCD/*Bcl11a*^{*-*/*-*} mice (n ≥ 3/genotype). Results are means ± SEM.

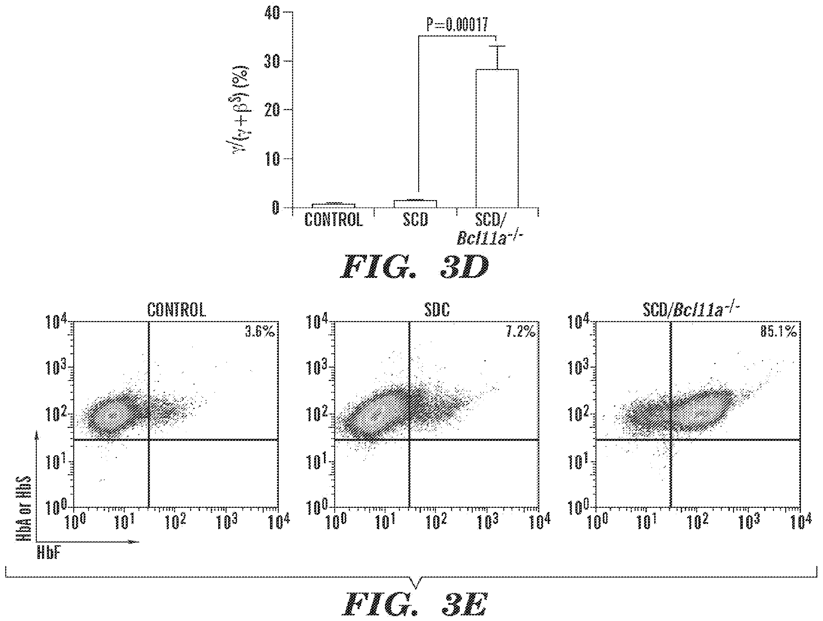

**Figure 3D** shows the expression of fetal (γ) and sickle adult (βs) globin genes as monitored by qRT-PCR in the peripheral blood of control, SCD and SCD/*Bcl11a*^{*-*/*-*} animals (8 to 10-weeks old; n = 5, 6 and 4, respectively). Results are means ± SEM.
**Figure 3E** shows the distribution of HbF in red blood cells of control, SCD and SCD/*Bcl11a*^{*-*/*-*} animals. Representative graphs are shown. The same scale is used in all 3 graphs, and the mean percentage of F-cells (HbF/HbA double-positive) is shown (n = 5,6 and 4, respectively).

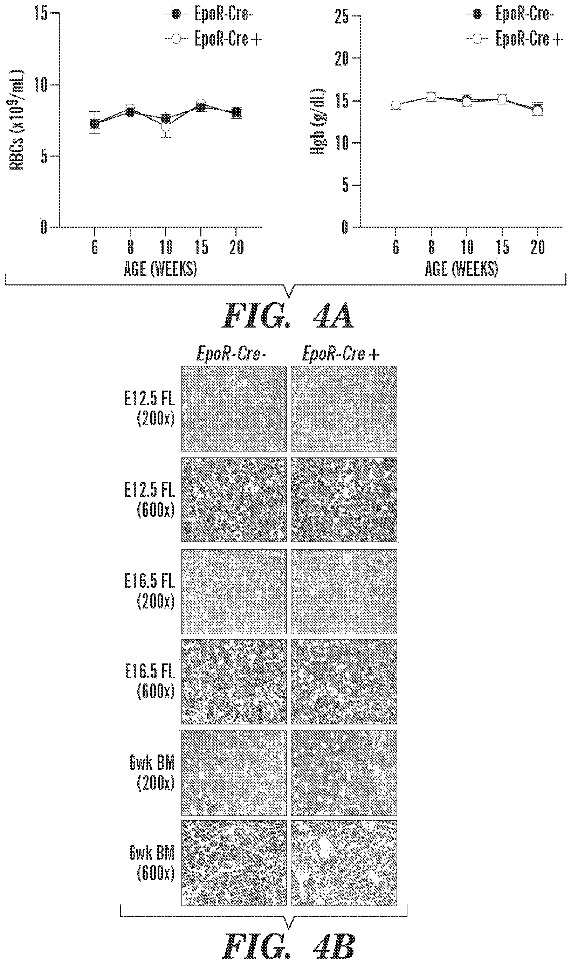

**Figures 4A-4D** demonstrate that mice lacking BCL11A in the erythroid compartment display normal phenotypic and morphological erythropoiesis.
**Figure 4A** shows the RBC counts and hemoglobin (Hgb) levels in control (EpoR-Cre-) and BCL11A knockout (*EpoR-Cre*+) β-YAC mice (n ≥ 7/genotype).
**Figure 4B** shows the hematoxylin-eosin stained histological sections from E12.5, E16.5 fetal livers, and 6-week bone marrows are shown at two magnifications (200x and 600x). These sections reveal clusters of erythroblasts within the FL and BM that appear to be similar in quantity and morphologically normal.

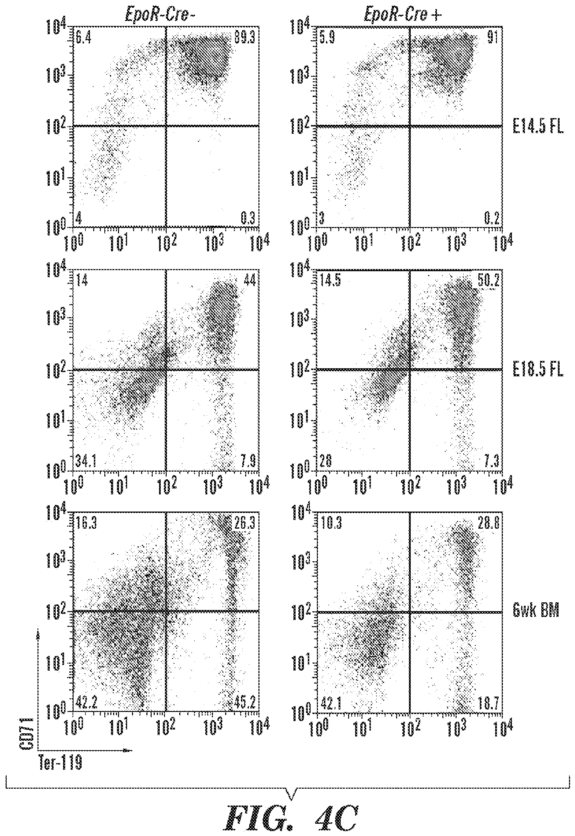

**Figure 4C** shows the erythroid maturation as assessed by using the markers CD71 and Ter119 in E14.5, E18.5 fetal livers and 6-week bone marrows from control (*EpoR-Cre-*) and BCL11A knockout (*EpoR-Cre*+) β-YAC mice. The mean values in each quadrant are shown (n ≥ 6/genotype at each time point).

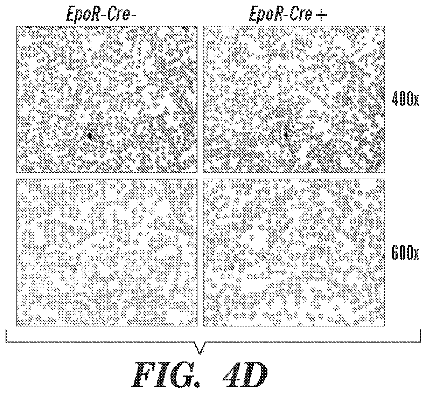

**Figure 4D** shows the blood smears prepared from peripheral blood of 10 to 12-weeks old control (*EpoR-Cre*-) and BCL11A knockout (*EpoR-Cre*+) mice. Smears were stained with May-Grünwald-Giemsa. Images are shown at 400x and 600x magnifications.

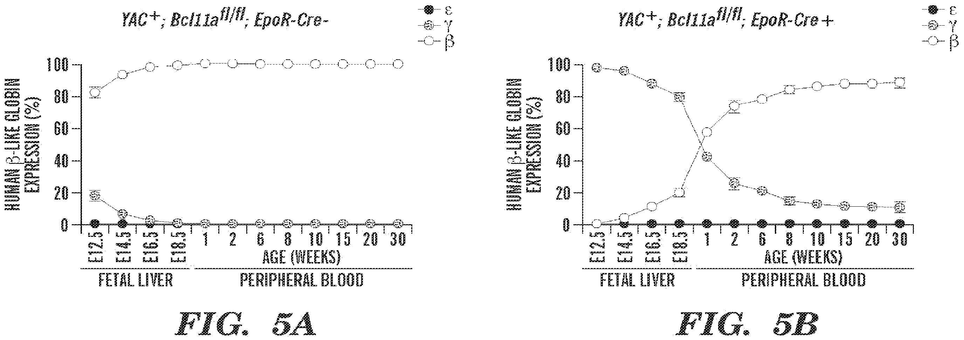

**Figures 5A-5B** demonstrated that mice harboring erythroid-selective deletion of BCL11A failed to silence the expression of human γ-globin genes.
**Figure 5A** shows the expression of human embryonic (ε), fetal (γ), and adult (β) globin genes as monitored by qRT-PCR in fetal liver cells (E12.5 to E18.5) or peripheral blood of postnatal control (YAC+ *Bcl11a*^{fl/fl} *EpoR-Cre*-) animals (1- to 30-weeks old). Data is plotted as percentage of γ-globin over total β-like human globin gene levels calculated based upon qRT-PCR results in the control mice. (n ≥ 4/genotype at each time point). Results are the means ± SD of the measurement. All γ- and β-globin levels for the different genotypes are significantly different (P < 1 × 10⁻⁵, two-tailed t-test).
**Figure 5B** shows the expression of human embryonic (ε), fetal (γ), and adult (β) globin genes as monitored by qRT-PCR in fetal liver cells (E12.5 to E18.5) or peripheral blood of postnatal BCL11A knockout (YAC+ *Bcl11a*^{fl/fl} *EpoR-Cre*+) animals (1- to 30-weeks old). Data is plotted as percentage of γ-globin over total β-like human globin gene levels calculated based upon qRT-PCR results in the BCL11A knockout mice. (n ≥ 4/genotype at each time point). Results are the means ± SD of the measurement. All γ- and β-globin levels for the different genotypes are significantly different (P < 1 × 10⁻⁵, two-tailed t-test).

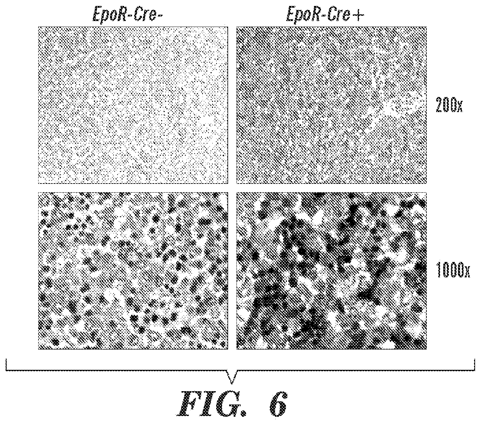

**Figure 6** depicts representative micrographs of IHC for human HbF that was performed on E16.5 fetal livers from control (EpoR-Cre-) and BCL11A knockout (EpoR-Cre+) animals showing the erythroid-specific deletion of BCL11A reactivates HbF expression. Robust expression of HbF is seen in the scattered erythroblasts of the E16.5 fetal liver in BCL11A knockout but not control mice.

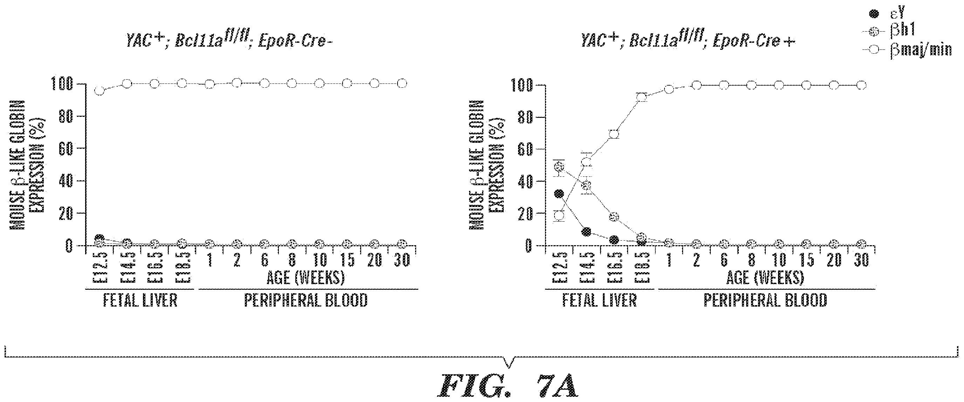

**Figures 7A-7C** show that erythroid-specific deletion of BCL11A causes upregulation of mouse embryonic β-like globin genes.
**Figure 7A** shows that the expression of mouse embryonic (εy and βh1) and adult (βmajor and βminor) globin genes as monitored by qRT-PCR in fetal liver cells (E12.5 to E18.5) or peripheral blood of postnatal control (YAC+ *Bcl11a*^{fl/fl} *EpoR-Cre*-) and BCL11A knockout (YAC+ *Bcl11a*^{fl/fl} *EpoR-Cre*+) animals (1- to 30-weeks old). All data are shown as percentage (%) of total mouse β-like globin expression in control or BCL11A knockout mice respectively (n ≥ 4/genotype at each time point). Results are plotted as the means ± SD of the measurement.

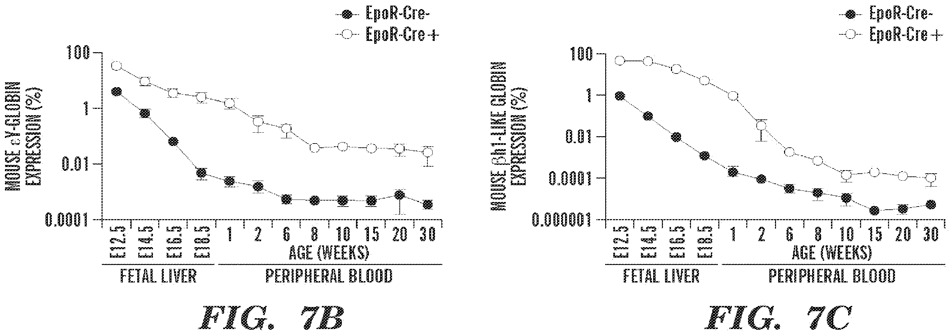

**Figure 7B** shows that the expression of mouse embryonic εy-globin mRNA in *EpoR-Cre-* and *EpoR-Cre*+ mice from Figure 7A is plotted on the same graph. All εy-globin levels for the different genotypes are significantly different (P < 0.001 by a two-tailed *t*-test).
**Figure 7C** shows that the expression of mouse embryonic βh1-globin mRNA in *EpoR-Cre-* and *EpoR-Cre*+ mice from Figure 7A is plotted on the same graph. All βh1-globin levels for the different genotypes are significantly different (P < 0.001 by a two-tailed *t*-test).

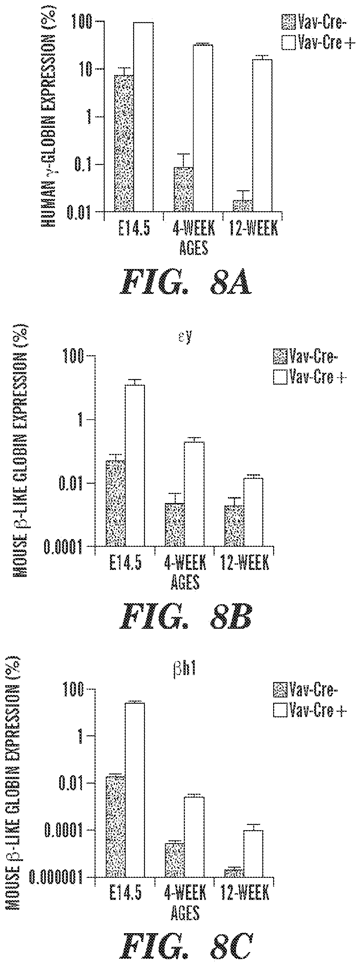

**Figures 8A-8F** demonstrate that BCL11A conditional knockout mice fail to silence the expression of human γ-globins and mouse embryonic β-like globins.
**Figure 8A** shows expression of human γ-globin genes as monitored by qRT-PCR in FACS-sorted CD71+Ter119+ FL (E14.5) and BM (4-week and 12-weeks old) erythroid cells in control (*Bcl11a*^{fl/fl} *Vav-Cre*-) and BCL11A knockout (*Bcl11a*^{fl/fl} *Vav-Cre*+) β-YAC mice. All data are shown as percentage (%) of total human β-like globin expression. Results are plotted as the means ± SD of the measurement (n ≥ 4/genotype at each time point). All γ-globin levels for the different genotypes are significantly different (P < 0.0001 by a two-tailed *t*-test).
**Figure 8B** shows expression of mouse embryonic εy-globin gene as monitored by qRT-PCR in E14.5 FL cells or peripheral blood of postnatal control (*Bcl11a*^{fl/fl} *Vav-Cre*-) and BCL11A knockout (*Bcl11a*^{fl/fl} *Vav-Cre*+) animals (4-week and 12-weeks old). All data are shown as percentage (%) of total mouse β-like globin expression in Vav-Cre- and *Vav*-*Cre*+ animals (n ≥ 4/genotype at each time point). All εy-globin levels for the different genotypes are significantly different (P < 0.0001 by a two-tailed *t*-test).
**Figure 8C** shows expression of mouse embryonic βh1-globin gene as monitored by qRT-PCR in E14.5 FL cells or peripheral blood of postnatal control (*Bcl11a*^{fl/fl} *Vav-Cre*-) and BCL11A knockout (*Bcl11a*^{fl/fl} *Vav-Cre*+) animals (4-week and 12-weeks old). All data are shown as percentage (%) of total mouse β-like globin expression in *Vav-Cre-* and *Vav-Cre*+ animals (n ≥ 4/genotype at each time point). All βh1-globin levels for the different genotypes are significantly different (P < 0.0001 by a two-tailed *t*-test).

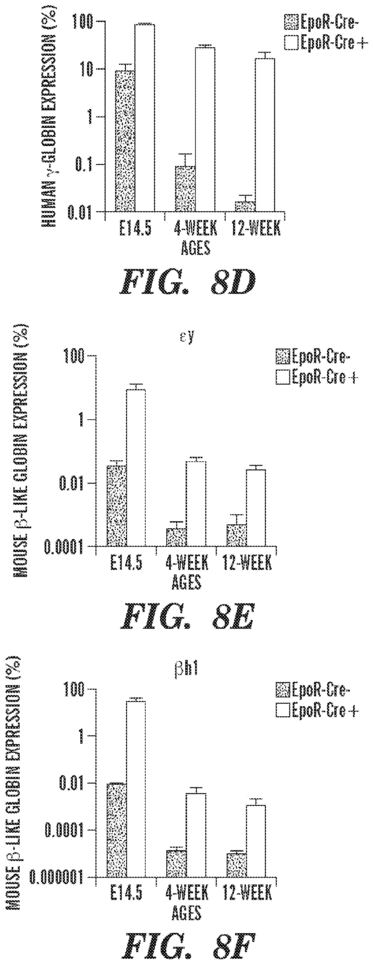

**Figure 8D** shows expression of human γ-globin genes that was monitored by qRT-PCR in *Bcl11a*^{fl/fl} *EpoR-Cre* conditional knockout mice harboring a single copy of human β-globin locus YAC (n ≥ 4/genotype at each time point). All data are shown as percentage (%) of total β-like human globin expression. All γ-globin levels for the different genotypes are significantly different (P < 0.0001 by a two-tailed *t*-test).
**Figure 8E** shows expression of mouse εy-globin as monitored by qRT-PCR in E14.5 FL cells or peripheral blood of postnatal (*Bcl11a*^{fl/fl} *EpoR-Cre*-) and BCL11A knockout (*Bcl11a*^{fl/fl} *EpoR-Cre*+) animals (4-week and 12-weeks old). All data are shown as percentage (%) of total mouse β-like globin expression in the single copy β-YAC mice (control: EpoR-Cre-, BCL11A knockout: EpoR-Cre+) (n ≥ 4/genotype at each time point). Results are plotted as the means ± SD. All εy-globin levels for the different genotypes are significantly different (P < 0.0001 by a two-tailed *t*-test).
**Figure 8F** shows expression of mouse εy- and βh1-globin was monitored by qRT-PCR in E14.5 FL cells or peripheral blood of postnatal (*Bel11a*^{fl/fl} *EpoR-Cre*-) and BCL11A knockout (*Bel11a*^{fl/fl} *EpoR-Cre*+) animals (4-week and 12-weeks old). All data are shown as percentage (%) of total mouse β-like globin expression in the single copy β-YAC mice (control: *EpoR-Cre-,* BCL11A knockout: *EpoR-Cre*+) (n ≥ 4/genotype at each time point). Results are plotted as the means ± SD. All βh1-globin levels for the different genotypes are significantly different (P < 0.0001 by a two-tailed *t*-test).

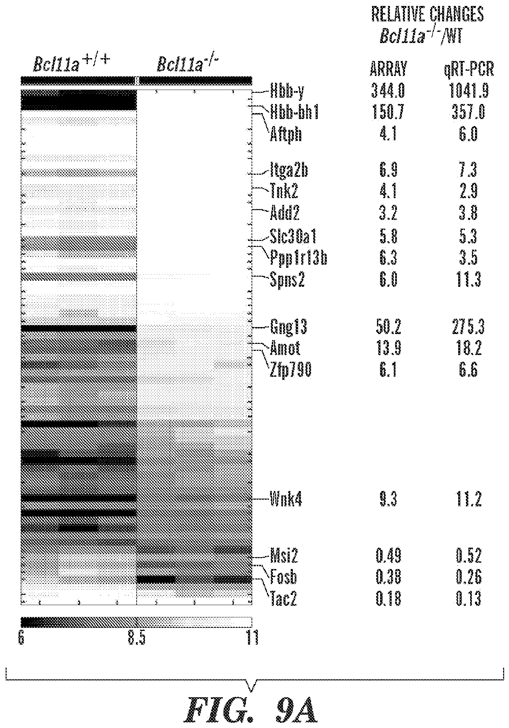

**Figures 9A-9B** show that BCL11A has limited number of non-globin target genes in erythroid cells.
**Figure 9A** shows a heatmap for all genes whose expression differs by more than 2-fold after loss of BCL11A and a selected group of 16 genes with their relative fold changes indicated. Genes are ordered according to difference in normalized expression value.

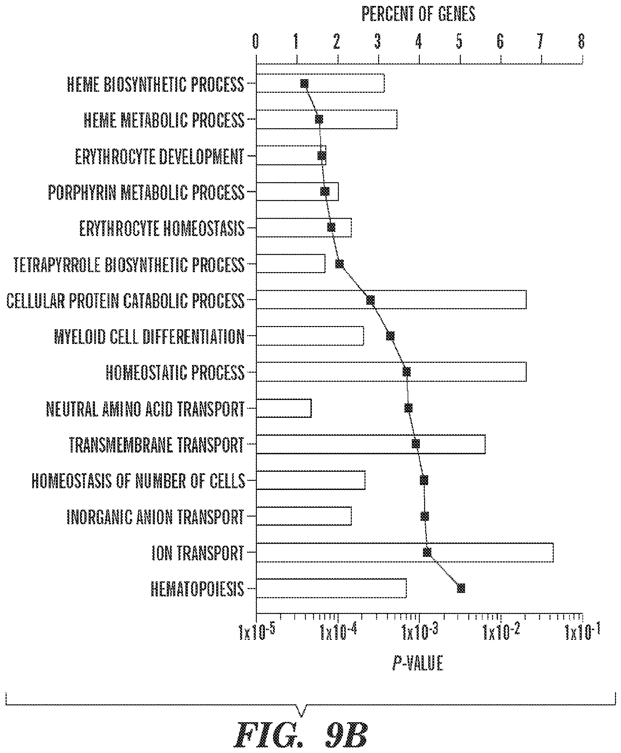

**Figure 9B** shows a functional annotation of the differentially expressed genes as performed using Gene Ontology (GO) Biological Process analysis. Top overrepresented gene categories (P < 0.005) are shown. P-values are indicated by the line. Genes affected by loss of BCL11A are overrepresented in biological process related to heme biosynthesis, iron transport, metabolism, and erythrocyte development.

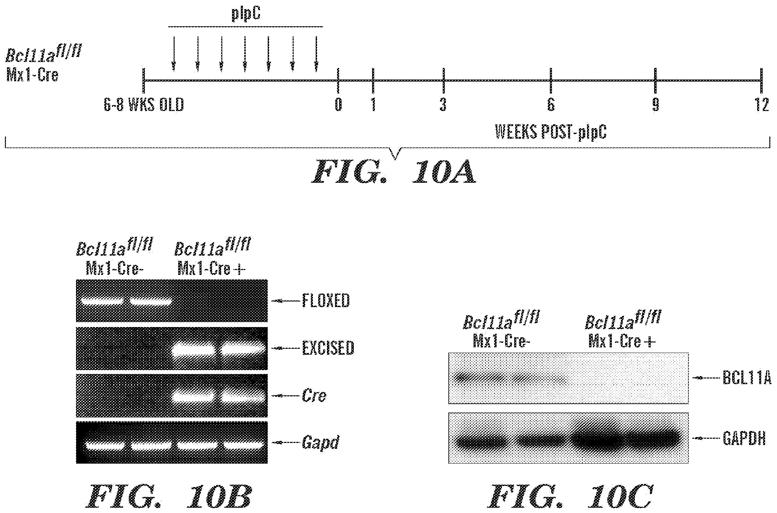

**Figure 10A** is a schematic diagram showing an experimental design for the inactivation of BCL11A in adult *Bcl11a*^{fl/fl} *Mx1-Cre*+ mice via polyI:polyC (pIpC) injection (arrows) to induce interferon-mediated deletion.
**Figure 10B** is a DNA gel showing that there is efficient excision in BCL11A knockout (*Bcl11a*^{fl/fl} *Mxl-Cre*+) β-YAC mice 12 weeks post-injection of pIpC. The DNA for the gel were obtained by genotyping PCR using genomic DNA from FACS-sorted CD71⁺Ter119⁺ BM cells. **Figure 10C** is a Western blot analysis of the BCL11A protein in FACS-sorted CD71Ter⁺119⁺ BM cells in control (*Bcl11a*^{fl/fl} *Mx1-Cre*-) and BCL11A knockout (*Bcl11a*^{fl/fl} *Mx1-Cre*+) mice 12 weeks post-injection of pIpC.

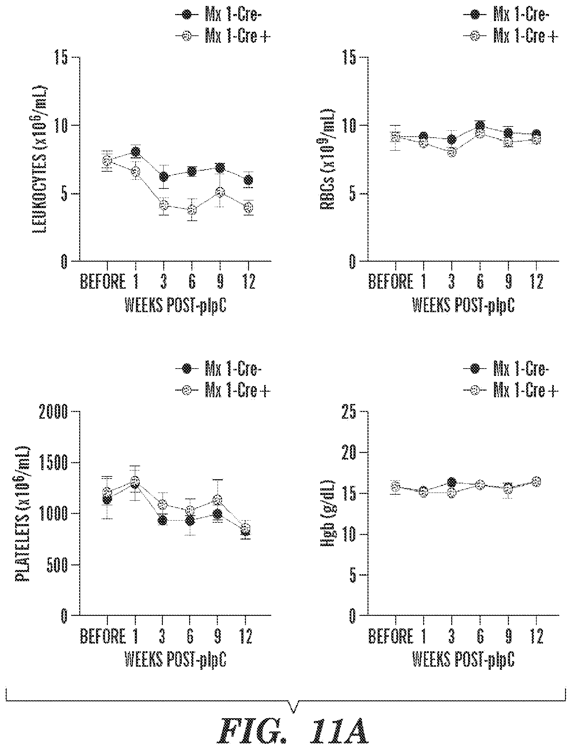

**Figure 11A** shows a hematopoietic analysis of BCL11A conditional knockout mice (*Mx1-Cre*+) compared to control mice (*Mx1-Cre*-)*.* The differential peripheral blood (PB) counts were monitored before and after injection of pIpC (n ≥ 4/genotype at each time point). Data are shown as means ± SEM.

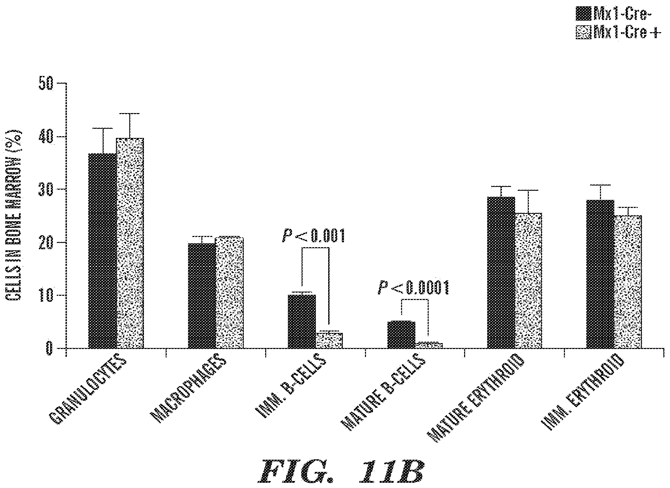

**Figure 11B** shows percentages of major hematopoietic lineages in the bone marrows of control (*Mx1-Cre*-) and BCL11A knockout (*Mx1-Cre*+) mice 12 weeks post-pIpC administration as assessed by FACS. Phenotypic surface markers for the hematopoietic lineages are as follows: granulocytes (Gr-1⁺CD11b⁺), macrophages (CD11b⁺F4/80⁺), immature B lymphocytes (IgM⁻B220⁺), mature B lymphocytes (IgM⁺B220⁺), immature erythroid cells (CD71⁺Ter119⁺), and mature erythroid cells (CD71⁻Ter119⁺) (n ≥ 5/genotype).

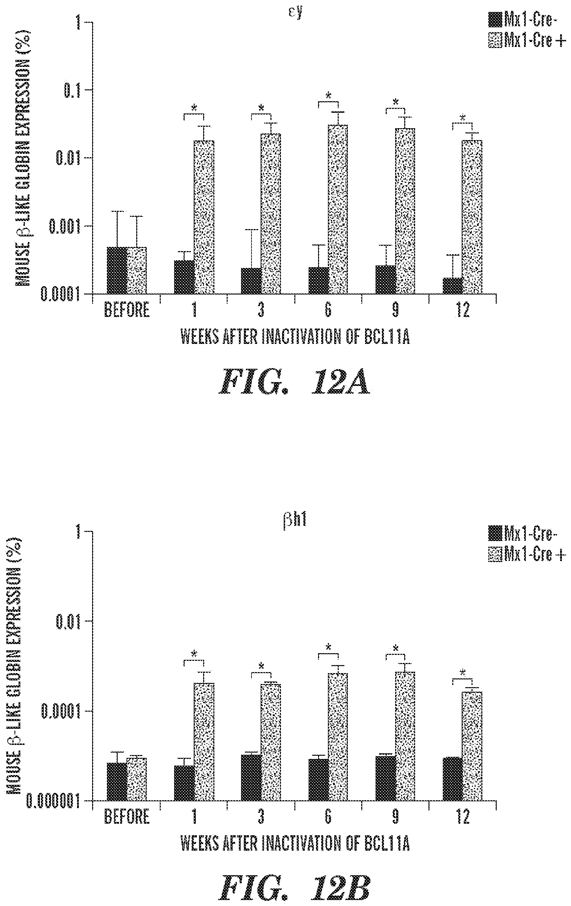

**Figure 12A** shows that inducible deletion of BCL11A in the hematopoietic compartments causes reactivation of mouse embryonic εy-globin gene, the mRNA was monitored by qRT-PCR in control (*Mx1-Cre*-) and BCL11A knockout (Mx1-Cre+) β-YAC mice before and after pIpC injection (n ≥ 4/genotype at each time point; *P < 0.0001 by a two-tailed *t*-test). **Figure 12B** shows that inducible deletion of BCL11A in the hematopoietic compartments causes reactivation of mouse embryonic βh1-globin gene, the mRNA was monitored by qRT-PCR in control (*Mx1-Cre*-) and BCL11A knockout (Mx1-Cre+) β-YAC mice before and after pIpC injection (n ≥ 4/genotype at each time point; *P < 0.0001 by a two-tailed *t*-test).

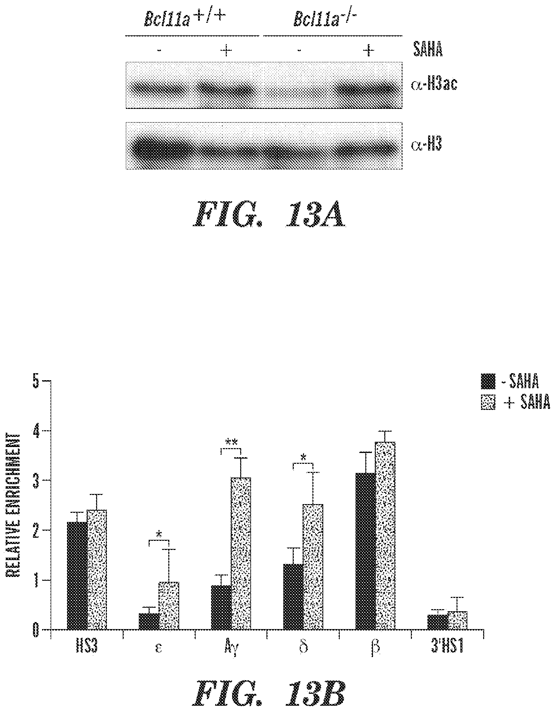

**Figures 13A-13B** demonstrate that suberoylanilide hydroxamic acid (SAHA) treatment inhibits histone deacetylation.
**Figure 13A** shows a Western blot analysis of the level of acetyl-H3 (H3ac) in whole-cell extracts prepared from the bone marrow (BM) cells of control and *Bcl11a*^{*-*/*-*} (*EpoR-Cre*+) mice that have been treated with or without SAHA treatment. Total histone H3 level was analyzed as a control.
**Figure 13B** shows level of *in vivo* chromatin acetyl-H3 within the various human globin loci in control and *Bcl11a*^{*-*/*-*} (*EpoR-Cre*+) β-YAC mice treated with or without SAHA treatment. The data was examined by ChIP using FACS-sorted CD71+Ter119+ BM cells isolated from the mice. Results are means ± SD. *P < 0.01; **P < 0.001.

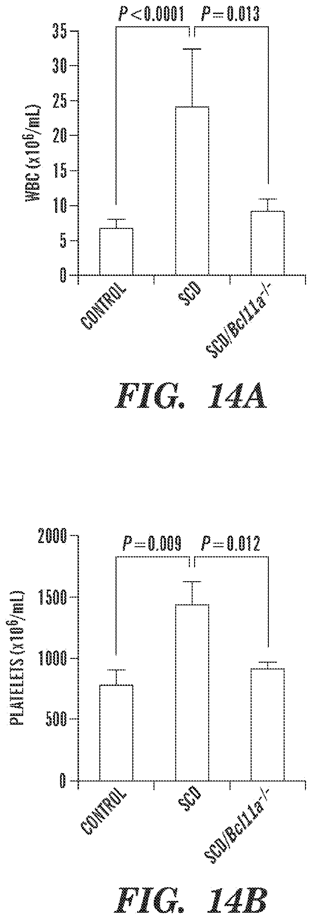

**Figures 14A-14B** demonstrate that a loss of BCL11A corrects the defects of high white blood cell (WBC) and high platelets counts in the sickle cell disease model (SCD) mice.
**Figure 14A** shows a comparison of the WBC counts in the peripheral blood of control, SCD and SCD/*Bcl11a*^{*-*/*-*} animals (n ≥ 5/genotype). Results are shown as means ± SEM of the measurement. P values were calculated by a two-tailed t-test.
**Figure 14B** shows a comparison of the platelet counts in the peripheral blood of control, SCD and SCD/*Bcl11a*^{*-*/*-*} animals (n ≥ 5/genotype). Results are shown as means ± SEM of the measurement. P values were calculated by a two-tailed *t*-test.

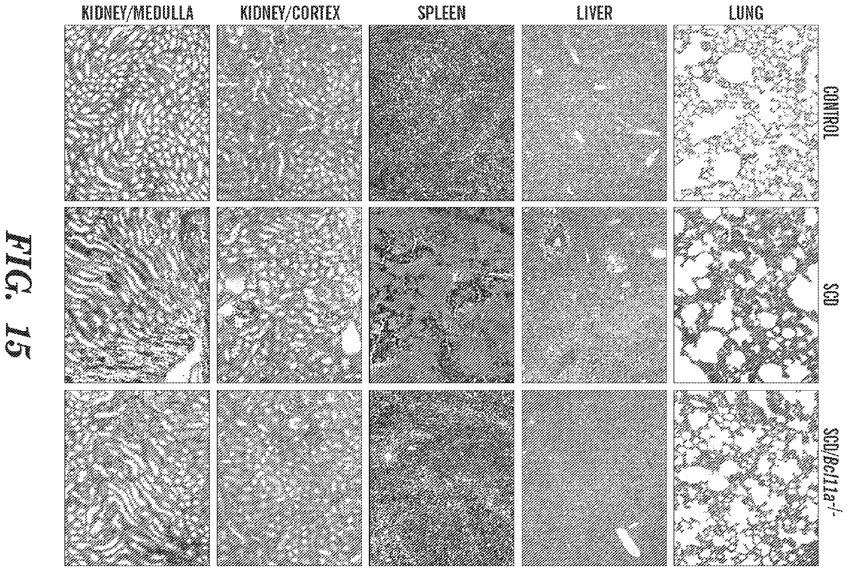

**Figure 15** depicts representative histological sections of the lung, liver, spleen, and kidney in control, SCD and SCD/*Bcl11a*^{*-*/*-*} mice showing that knockdown of BCL11a amelioration certain pathology in these tissues.

### DETAILED DESCRIPTION

As demonstrated herein, the inventors have discovered that known chemical inducers of fetal hemoglobin (HbF) expression, such as epigenetic modifiers, unexpectedly work in a synergistic manner with knockout or knockdown of activity or expression of BCL11a, a transcription factor regulating the fetal switch in mammals, producing a significantly increase in HbF expression compared to when the chemical inducers are used alone. Accordingly, provided herein are combinatorial compositions comprising one or more BCL11A inhibitors and one or more chemical inducers of HbF expression, and methods of use of these compositions in the treatments of diseases and disorders associated with decreased HbF production.

BCL11A is a C2H2-type zinc finger transcription factor that was shown to act as a stage specific regulator of fetal hemoglobin expression such that expression of BCL11A represses γ-globin induction during the "fetal switch." BCL11A was demonstrated to be a potent silencer of fetal hemoglobin in both mouse and humans. Thus, BCL11A can be a therapeutic target for reversing the fetal switch. Therefore, inhibition of BCL11A protein activity or inhibition of the expression of BCL11A is useful in reactivation of fetal hemoglobin production in adults (see, for example, WO2010/030963).

In addition to genetic approaches to reactivating of fetal hemoglobin production, pharmacologic approaches have also been attempted, with limited therapeutic success *in vivo.* These chemicals typically work by manipulation of the kinetics of erythropoiesis by using cytotoxic drugs and modulation of chromatin using histone deacetylase inhibitors. The chemicals that can induce HbF reactivation generally fall into three classes, and the known chemical inducers of HbF reactivation mainly target the two epigenetic pathways, DNA methylation and histone deacetylation, that regulate HbF expression in an adult.

The first group of compounds discovered having HbF reactivation activity were cytotoxic drugs. The ability to cause *de novo* synthesis of HbF by pharmacological manipulation was first shown using 5-azacytidine in experimental animals. Subsequent studies confirmed the ability of 5-azacytidine to increase HbF in patients with β-thalassemia and sickle cell disease. Additional experiments demonstrated that baboons treated with cytotoxic doses of arabinosylcytosine (ara-C) responded with striking elevations of F-reticulocytes, and that treatment with hydroxyurea led to induction of γ-globin in monkeys or baboons. 5-azacytidine and hydroxyurea are inhibitors of DNA methylation. Hydroxyurea is the first and only drug approved by the Food and Drug Administration (FDA) for the treatment of hemoglobinopathies.

The second group of compounds that have HbF reactivation activity are short chain fatty acids. The initial observation in fetal cord blood progenitor cells led to the discovery that γ-aminobutyric acid can act as a fetal hemoglobin inducer. Subsequent studies showed that butyrate stimulated globin production in adult baboons, and it induced γ-globin in erythroid progenitors in adult animals or patients with sickle cell anemia. Derivatives of short chain fatty acids such as phenylbutyrate and valproic acid also have been shown to induce HbF *in vivo.* Presently, however, the use of butyrate or its analogs in sickle cell anemia and β-thalassemia remains experimental and is not recommended for treatment outside of clinical trials.

The third group of compounds that have been shown to reactive HbF expression are histone deacetylase inhibitors, such as analogues of trichostatin A, analogues of trapoxin, and scriptaid and its analogues, and suberoylanilide hydroxamic acid (SAHA or Vorinostat).

Despite the number of chemicals compounds that have been shown to reactive HbF in primary human erythroid cell cultures, these chemicals have limited utility as therapeutic agents *in vivo.* These chemicals have varying drawbacks that contraindicate the long term use of such agents or therapies, including unwanted side effects and variability in patient responses. For example, while hydroxyurea stimulates HbF production and has been shown to clinically reduce sickling crisis, it is potentially limited by myelotoxicity and the risk of carcinogenesis. Risks for long term carcinogenicity also exists in 5-azacytidine-based therapies. Erythropoietin-based therapies have not proved consistent among a range of patient populations. The short half-lives of butyric acid *in vivo* have been viewed as a potential obstacle in adapting these compounds for use in therapeutic interventions. Furthermore, very high dosages of butyric acid are necessary for inducing γ-globin gene expression, requiring catheritization for continuous infusion of the compound. Moreover, these high dosages of butyric acid can be associated with neurotoxicity and multiorgan damage. While even minimal increases in HbF levels are helpful in sickle cell disease, β-thalassemias require a much higher increase that is not reliably, or safely, achieved by any of the currently used agents.

As described herein, the inventors have discovered that DNA methylation of γ-globin promoters progressively increased in BCL11A-null erythroid cells in correlation with the gradual, but partial, silencing of γ-globin expression (Figure 5A). These data demonstrate that, in addition to BCL11a-based transcriptional regulation of the fetal switch, DNA methylation is also involved in turning off HbF expression in an adult mammal. As demonstrated herein, administration of the DNA methylation inhibitor 5-aza-2'-deoxycytidine (5-azaD) was unexpectedly synergistic with BCL11A loss, leading to a 37.9% increase in γ-globin mRNA, in contrast to administration of 5-azaD to normal β-YAC mice, which led to a very small increment in γ-globin mRNA (Figure 5B). The inventors further demonstrated that the administration of an HDAC inhibitor, suberoylanilide hydroxamic acid (SAHA) also synergized with BCL11A in derepression of γ-globin mRNA (Figure 5D).

Accordingly, as demonstrated herein, loss of BCL11A markedly and unexpectedly enhances the effects of DNA demethylation and HDAC inhibitors in reactivating HbF expression, and indicates that BCL11A downregulation can be combined with known HbF chemical inducers for efficient HbF augmentation. Importantly, a combination therapy of BCL11A downregulation with HbF chemical inducers permits lower amounts/doses of HbF chemical inducers to be used for *in vivo* therapies for hemoglobinpathies, while at the same time achieving a sufficient increase of HbF reactivation in a mammal to ameliorate the symptoms of the hemoglobinpathies, *e*.*g*., the need for blood transfusion, a lower frequency of blood transfusion, anemia etc. The use of lower amounts/doses of HbF chemical inducers is greatly desirable because of the various drawbacks of current HbF chemical inducers, such as cell toxicity, as described herein. Accordingly, the problem of HbF chemical inducers having limited utility as a hemoglobinpathy therapeutic agent *in vivo* due to unwanted side effects is solved by using a lower or smaller amount of the HbF chemical inducers in a combination with an inhibitor of BCL11a, as described herein.

Accordingly, provided herein are combinatorial compositions and methods for increasing fetal hemoglobin expression (HbF) for the treatment and/or amelioration of the symptoms of hemoglobinpathy in a mammal. These combinatorial compositions comprise a combination of inhibitors of BCL11A expression or activity and HbF chemical inducers, such as inhibitors of DNA methylation and/or inhibitors of histone deacetylases, for increasing fetal hemoglobin levels in hematopoietic progenitor cell and in a mammal.

### Combinatorial Compositions of Inhibitors of BCL11A and Epigentic Modifiers

As described herein, the inventors have discovered that epigenetic modifiers, such as inhibitors of DNA methylation and/or inhibitors of histone deacetylation (HDAC) have synergistic effects on increasing levels of fetal hemoglobin, thereby providing less toxic and more effective compositions and therapeutic methods for the treatment of various blood disorder.

Accordingly, provided herein, in some aspects, are combinatorial compositions for increasing fetal hemoglobin levels in a mammal in need thereof, the compositions comprising one or more inhibitors of BCL11A and one or more chemical inducers of HbF, such as one or more epigenetic modifiers. These inventive combinations of BCL11A inhibitors and epigenetic modifiers can be administered by a variety of routes, and can be administered or coadministered in any conventional dosage form. In the context of these combinatorial compositions, a "combinatorial composition" is also defined to include coadministration of more than one separate pharmaceutical composition of a therapeutic agent, such as a composition comprising a BCL11A inhibitor and a composition comprising an epigenetic modifier, in the course of a coordinated treatment to achieve an improved clinical outcome. Such coadministration can also be coextensive, that is, occurring during overlapping periods of time, or sequential. For example, the DNA methylation inhibitor can be administered to a patient before, concomitantly, or after the BCL11A inhibitor is administered. The amounts of the combinatorial compositions comprising one or more inhibitors of BCL11A and one or more chemical inducers of HbF can vary, according to determinations made by one of skill in the art, but ideally are in amounts effective to create minimal side-effects of cytotoxicity or cytostatic effects in a subject. Preferably, using these combinatorial compositions the total amount administered of each therapeutic agent, *e.g.,* a BCL11a inhibitor and an epigenetic modifer, is less that the total amount added for individual administration of each of these inhibitors, due to the synergistic effects discovered by the inventors, as described herein.

In those embodiments where a combination therapy regimen is applied or administered, one or more BCL11A inhibitors and one or more epigenetic modifiers as described herein below are administered in a therapeutically effective or synergistic amount. As used in such embodiments encompassing combination therapies, a therapeutically effective amount is such that coadministration of one or more BCL11A inhibitors and one or more epigenetic modifiers, or administration of a therapeutic composition or formulation comprising both a BCL11A inhibitor and one or more epigenetic modifiers, as described herein, results in an increase or upregulation of fetal hemoglobin, or a reduction or inhibition of a blood disorder. A "therapeutically synergistic amount" is that amount of a a BCL11A inhibitor and one or more epigenetic modifiers, such as a DNA methylation inhibitor or histone deactylase inhibitor, necessary to synergistically or significantly reduce or eliminate conditions or symptoms associated with a blood disorder or to increase or upregulate fetal hemoglobin levels.

Accordingly, in some aspects, provided herein are combinatorial compositions for increasing fetal hemoglobin levels in a subject in need thereof, comprising at least one BCL11 inhibitor and at least one HbF chemical inducer.

In one embodiment, provided herein are uses of combinatorial compositions for increasing fetal hemoglobin levels or treating a blood disorder in a subject in need thereof, the compositions comprising at least one BCL11 inhibitor and at least one HbF chemical inducer.

The inhibitor of BCL11A can be one that inhibits BCL11A activity, *e*.*g*., an antibody against BCL11A or an antigen-binding fragment thereof, or a small molecule BCL11A inhibitor. The inhibitor of BCL11A can also be one that inhibits BCL11a expression, *e*.*g*., a BCL11A specific RNA interference agent, a vector encoding a RNA interference agent, or an aptamer that binds BCL11A. In some embodiments of these aspects and all such aspects described herein, the inhibitor of BCL11A expression is a small molecule or a nucleic acid. In some such embodiments, the nucleic acid is a BCL11A specific RNA interference agent, a vector encoding the BCL11A specific RNA interference agent, or an aptamer that binds BCL11A. In some such embodiments, the RNA interference agent comprises one or more of the nucleotide sequences selected from the group consisting of SEQ ID NO: 3-8.

In some embodiments of these aspects, the at least one HbF chemical inducer is at least one epigenetic modifier. In some such embodiments, the at least one epigenetic modifier is at least one histone deacetylase inhibitors and/or at least one DNA methylation inhibitors. Such DNA methylation inhibitors can include 5-aza-2'-deoxycytidine (5-aza). HDAC inhibitors include, but are not limited to, suberoylanilide hydroxamic acid (SAHA or Vorinostat), amide analogues of trichostatin A, hydroxamic acid analogues of trapoxin, and scriptaid and analogues.

In some aspects, provided herein are combinatorial compositions for increasing fetal hemoglobin levels in a subject in need thereof, comprising at least one BCL11A inhibitor and at least one DNA methylation inhibitor. In other aspects, provided herein are combinatorial compositions for increasing fetal hemoglobin levels in a subject in need thereof, comprising at least one BCL11A inhibitor and at least one HDAC inhibitor. In some aspects, provided herein are combinatorial compositions for increasing fetal hemoglobin levels in a subject in need thereof, comprising at least one BCL11A inhibitor, at least one DNA methylation inhibitor, and at least one HDAC inhibitor.

In some embodiments of these aspects and all such aspects described herein, the inhibitor of BCL11A can be one that inhibits BCL11A activity, *e.g.,* an antibody against BCL11A or an antigen-binding fragment thereof, or a small molecule BCL11A inhibitor. The inhibitor of BCL11A can also be one that inhibits BCL11a expression, *e*.*g*., a BCL11A specific RNA interference agent, a vector encoding a RNA interference agent, or an aptamer that binds BCL11A. In some embodiment of these aspects and all such aspects described herein, the inhibitor of BCL11A inhibits BCL11A expression and/or inhibits BCL11A activity.

In some embodiments of these aspects and all such aspects described herein, the at least one HbF chemical inducer is at least one epigenetic modifier. In some such embodiments, the at least one epigenetic modifier is at least one histone deacetylase inhibitors and/or at least one DNA methylation inhibitors. Such DNA methylation inhibitors can include 5-aza-2'-deoxycytidine (5-aza). HDAC inhibitors include, but are not limited to, suberoylanilide hydroxamic acid (SAHA or Vorinostat), amide analogues of trichostatin A, hydroxamic acid analogues of trapoxin, and scriptaid and analogues. In some aspects of these compositions, the HDAC inhibitor is selected from the group consisting of suberoylanilide hydroxamic acid (SAHA), N-Hydroxy-7-(4-dimethylaminobenzoyl)-aminoheptanamide (M344), N-Hydroxy-8-(4-dimethylaminobenzoyl)-aminooctanamide (M360), N-Hydroxy-6-(4-biphenylcarbonyl)-aminocapramide (M355), N-Hydroxy-6-(4-dimethylaminobenzoylamino)-capramide (MD85), (S)-Octanedioic acid hydroxyamide (1-phenethylcarbamoyl-2-phenyl-ethyl)-amide (SW68), (S)-Octanedioic acid hydroxyamide (1-benzylcarbamoyl-2-phenyl-ethyl)-amide (SW70), (S)-3-(4-Methoxy-phenyl)-2-(7-hydroxycarbamoyl-heptanoylamino)-propionic acid methyl ester (SW99), (S)-2-(7-Hydroxycarbamoyl-heptanoylamino)-3-thiophen-2-yl-propionic acid methyl ester (SW86), (S)-3-(4'-Chlorobiphenyl-4-yl)-2-(7-hydroxycarbamoylheptanoylamino)-propionic acid methyl ester (SW183), (S)-3-(3', 4'-Dichlorobiphenyl-4-yl)-2-(7-hydroxycarbamoylheptanoylamino)-propionic acid methyl ester (SW187), (S)-2-(7-Hydroxycarbamoylheptanoylamino)-3-(4-methoxybiphenyl-4-yl)-propionic acid methyl ester (SW188), (S)-2-(7-Hydroxycarbamoylheptanoylamino)-3-(4'-methylbiphenyl-4-yl)-propionic acid methyl ester (SW189), (S)-3-(Phenyl)-2-(7-hydroxycarbamoyl-heptanoylamino)-propionic acid methyl ester (M232), 6-(1,3-Dioxo-1H,3H-benzo[de]isoquinolin-2-yl)-hexanoic acid hydroxyamide (HR13), 6-(4,5,6,7-Tetrachloro-1,3-dioxo-1,3-dihydroisoindol-2-yl) hexanoic acid hydroxyamide (HR10) and 6-(1,3-Dioxo-1,3-dihydroisoindol-2-yl) hexanoic acid hydroxyamide (HR11).

The various types of BCL11A inhibitors, and epigenetic modifiers, such as DNA methylation inhibitors and/or histone deacetylation (HDAC) inhibitors, encompassed for use in the compositions and methods described herein are further described below:

### Inhibitors of BCL11A Expression or Activity

BCL11A is a C2H2-type zinc finger transcription factor that has been shown to act as a stage specific regulator of fetal hemoglobin expression such that expression of BCL11A represses γ-globin induction during the "fetal switch." BCL11A was demonstrated to be a potent silencer of fetal hemoglobin in both mouse and humans. Therefore, inhibition of BCL11A protein activity or inhibition of the expression of BCL11A using the inhibitors of BCL11A is useful in reactivation of fetal hemoglobin production in adults (see, for example, WO2010/030963).

Accordingly, the term "BCL11A" as used herein refers to the 835 amino acid polypeptide having the amino acid sequence of:
MSRRKQGKPQHLSKREFSPEPLEAILTDDEPDHGPLGAPEGDHDLLTCGQCQMNFPLGDILIF IEHKRKQCNGSLCLEKAVDKPPSPSPIEMKKASNPVEVGIQVTPEDDDCLSTSSRGICPKQEHI ADKLLHWRGLSSPRSAHGALIPTPGMSAEYAPQGICKDEPSSYTCTTCKQPFTSAWFLLQHA QNTHGLRIYLESEHGSPLTPRVGIPSGLGAECPSQPPLHGIHIADNNPFNLLRIPGSVSREASGL AEGRFPPTPPLFSPPPRHHLDPHRIERLGAEEMALATHHPSAFDRVLRLNPMAMEPPAMDFS RRLRELAGNTSSPPLSPGRPSPMQRLLQPFQPGSKPPFLATPPLPPLQSAPPPSQPPVKSKSCEF CGKTFKFQSNLVVHRRSHTGEKPYKCNLCDHACTQASKLKRHMKTHMHKSSPMTVKSDD GLSTASSPEPGTSDLVGSASSALKSVVAKFKSENDPNLIPENGDEEEEEDDEEEEEEEEEEEEE LTESERVDYGFGLSLEAARHHENSSRGAVVGVGDESRALPDVMQGMVLSSMQHFSEAFHQ VLGEKHKRGHLAEAEGHRDTCDEDSVAGESDRIDDGTVNGRGCSPGESASGGLSKKLLLGS PSSLSPFSKRIKLEKEFDLPPAAMPNTENVYSQWLAGYAASRQLKDPFLSFGDSRQSPFASSS EHSSENGSLRFSTPPGELDGGISGRSGTGSGGSTPHISGPGPGRPSSKEGRRSDTCEYCGKVFK NCSNLTVHRRSHTGERPYKCELCNYACAQSSKLTRHMKTHGQVGKDVYKCEICKMPFSVY STLEKHMKKWHSDRVLNNDIKTE (**SEQ ID NO:1**), as described by, *e.g.,* NP_075044.2, together with any naturally occurring allelic, splice variants, and processed forms thereof, such as, for example, BCL11A isoform 2 (NP_060484.2) encoded by the mRNA sequence of NM 018014.3, or BCL11A isoform 3 (NP_612569.1) encoded by the mRNA sequence of NM_138559. Typically, BCL11A refers to human BCL11A. The term BCL11A is also used to refer to truncated forms or fragments of the BCL11A polypeptide, comprising, for example, specific BCL11A domains. Reference to any such forms of BCL11A can be identified in the application, *e.g.,* by "BCL11A(11-108). "

The BCL11A polypeptide of SEQ ID NO: 1 is encoded by the mRNA sequence provided herein as **SEQ ID NO: 2**: 
TTTTTTTTTTTTTTTTGCTTAAAAAAAAGCCATGACGGCTCTCCCACAATTCATCTTCCCT
GCGCCATCTTTGTATTATTTCTAATTTATTTGGATGTCAAAAGGCACTGATGAAGATAT
TTTCTCTGGAGTCTCCTTCTTTCTAACCCGGCTCTCCCGATGTGAACCGAGCCGTCGTCC
GCCCGCCGCCGCCGCCGCCGCCGCCGCCGCCCGCCCCGCAGCCCACCATGTCTCGCCGC
AAGCAAGGCAAACCCCAGCACTTAAGCAAACGGGAATTCTCGCCCGAGCCTCTTGAAGC
CATTCTTACAGATGATGAACCAGACCACGGCCCGTTGGGAGCTCCAGAAGGGGATCATG
ACCTCCTCACCTGTGGGCAGTGCCAGATGAACTTCCCATTGGGGGACATTCTTATTTTTA
TCGAGCACAAACGGAAACAATGCAATGGCAGCCTCTGCTTAGAAAAAGCTGTGGATAA
GCCACCTTCCCCTTCACCAATCGAGATGAAAAAAGCATCCAATCCCGTGGAGGTTGGCA
TCCAGGTCACGCCAGAGGATGACGATTGTTATCAACGTCATCTAGAGGAATTTGCCCC
AAACAGGAACACATAGCAGATAAACTTCTGCACTGGAGGGGCCTCTCCTCCCCTCGTTC
TGCACATGGAGCTCTAATCCCCACGCCTGGGATGAGTGCAGAATATGCCCCGCAGGGTA
TTTGTAAAGATGAGCCCAGCAGCTACACATGTACAACTTGCAAACAGCCATTCACCAGT
GCATGGTTTCTCTTGCAACACGCACAGAACACTCATGGATTAAGAATCTACTTAGAAAG
CGAACACGGAAGTCCCCTGACCCCGCGGGTTGGTATCCCTTCAGGACTAGGTGCAGAAT
GTCCTTCCCAGCCACCTCTCCATGGGATTCATATTGCAGACAATAACCCCTTTAACCTGC
TAAGAATACCAGGATCAGTATCGAGAGAGGCTTCCGGCCTGGCAGAAGGGCGCTTTCCA
CCCACTCCCCCCCTGTTTAGTCCACCACCGAGACATCACTTGGACCCCCACCGCATAGAG
CGCCTGGGGGCGGAAGAGATGGCCCTGGCCACCCATCACCCGAGTGCCTTTGACAGGGT
GCTGCGGTTGAATCCAATGGCTATGGAGCCTCCCGCCATGGATTTCTCTAGGAGACTTA
GAGAGCTGGCAGGGAACACGTCTAGCCCACCGCTGTCCCCAGGCCGGCCCAGCCCTATG
CAAAGGTTACTGCAACCATTCCAGCCAGGTAGCAAGCCGCCCTTCCTGGCGACGCCCCC
CCTCCCTCCTCTGCAATCCGCCCCTCCTCCCTCCCAGCCCCCGGTCAAGTCCAAGTCATG
CGAGTTCTGCGGCAAGACGTTCAAATTTCAGAGCAACCTGGTGGTGCACCGGCGCAGCC
ACACGGGCGAGAAGCCCTACAAGTGCAACCTGTGCGACCACGCGTGCACCCAGGCCAG
CAAGCTGAAGCGCCACATGAAGACGCACATGCACAAATCGTCCCCCATGACGGTCAAGT
CCGACGACGGTCTCTCCACCGCCAGCTCCCCGGAACCCGGCACCAGCGACTTGGTGGGC
AGCGCCAGCAGCGCGCTCAAGTCCGTGGTGGCCAAGTTCAAGAGCGAGAACGACCCCA
ACCTGATCCCGGAGAACGGGGACGAGGAGGAAGAGGAGGACGACGAGGAAGAGGAAG
AAGAGGAGGAAGAGGAGGAGGAGGAGCTGACGGAGAGCGAGAGGGTGGACTACGGCT
TCGGGCTGAGCCTGGAGGCGGCGCGCCACCACGAGAACAGCTCGCGGGGCGCGGTCGT
GGGCGTGGGCGACGAGAGCCGCGCCCTGCCCGACGTCATGCAGGGCATGGTGCTCAGCT
CCATGCAGCACTTCAGCGAGGCCTTCCACCAGGTCCTGGGCGAGAAGCATAAGCGCGGC
CACCTGGCCGAGGCCGAGGGCCACAGGGACACTTGCGACGAAGACTCGGTGGCCGGCG
 
AGTCGGACCGCATAGACGATGGCACTGTTAATGGCCGCGGCTGCTCCCCGGGCGAGTCG
GCCTCGGGGGGCCTGTCCAAAAAGCTGCTGCTGGGCAGCCCCAGCTCGCTGAGCCCCTT
CTCTAAGCGCATCAAGCTCGAGAAGGAGTTCGACCTGCCCCCGGCCGCGATGCCCAACA
CGGAGAACGTGTACTCGCAGTGGCTCGCCGGCTACGCGGCCTCCAGGCAGCTCAAAGAT
CCCTTCCTTAGCTTCGGAGACTCCAGACAATCGCCTTTTGCCTCCTCGTCGGAGCACTCC
TCGGAGAACGGGAGTTTGCGCTTCTCCACACCGCCCGGGGAGCTGGACGGAGGGATCTC
GGGGCGCAGCGGCACGGAAGTGGAGGGAGCACGCCCCATATTAGTGGTCCGGGCCCG
GGCAGGCCCAGCTCAAAAGAGGGCAGACGCAGCGACACTTGTGAGTACTGTGGGAAAG
TCTTCAAGAACTGTAGCAATCTCACTGTCCACAGGAGAAGCCACACGGGCGAAAGGCCT
TATAAATGCGAGCTGTGCAACTATGCCTGTGCCCAGAGTAGCAAGCTCACCAGGCACAT
GAAAACGCATGGCCAGGTGGGGAAGGACGTTTACAAATGTGAAATTTGTAAGATGCCTT
TTAGCGTGTACAGTACCCTGGAGAAACACATGAAAAAATGGCACAGTGATCGAGTGTTG
AATAATGATATAAAAACTGAATAGAGGTATATTAATACCCCTCCCTCACTCCCACCTGA
CACCCCCTTTTTCACCACTCCCCTTCCCCATCGCCCTCCAGCCCCACTCCCTGTAGGATTT
TTTTCTAGTCCCATGTGATTTAAACAAACAAACAAACAAACAGAAGTAACGAAGCTAAG
AATATGAGAGTGCTTGTCACCAGCACACCTGTTTTTTTCTTTTTCTTTTTCTTTTTTCTTT
TTCCTTTTTTTTTTTTTTCCTTTATGTTCTCACCGTTTGAATGCATGATCTGTATGGGGCAA
TACTATTGCATTTTACGCAAACTTTGAGCCTTTCTCTTGTGCAATAATTTACATGTTGTGT
ATGTTTTTTTTTAAACTTAGACAGCATGTATGGTATGTTATGGCTATTTTAAATTGTCCCT
AATTCGTTGCTGAGCAAACATGTTGCTGTTTCCAGTTCCGTTCTGAGAGAAAAAGAGAG
AGAGAGAGAAAAAGACCATGCTGCATACATTCTGTAATACATATCATGTACAGTTTTAT
TTTATAACGTGAGGAGGAAAAACAGTCTTTGGATTAACCCTCTATAGACAGAATAGATA
GCACTGAAAAAAAATCTCTATGAGCTAAATGTCTGTCTCTAAAGGGTTAAATGTATCAA
TTGGAAAGGAAGAAAAAAGGCCTTGAATTGACAAATTAACAGAAAAACAGAACAAGTT
TATTCTATCATTTGGTTTTAAAATATGAGTGCCTTGGATCTATTAAAACCACATCGATGG
TTCTTTCTACTTGTTATAAACTTGTAGCTTAATTCAGCATTGGGTGAGGTAATAAACCTT
AGGAACTAGCATATAATTCTATATTGTATTTCTCACAACAATGGCTACCTAAAAAGATG
ACCCATTATGTCCTAGTTAATCATCATTTTTCCTTTAGTTTAATTTTATAAACAAAACTGA
TTATACCAGTATAAAAGCTACTTTGCTCCTGGTGAGAGCTTAAAAGAAATGGGCTGTTTT
GCCCAAAGTTTTATTTTTTTTAAACAATGATTAAATTGAATGTGTAATGTGCAAAAGCCC
TGGAACGCAATTAAATACACTAGTAAGGAGTTCATTTTATGAAGATATTTGCTTTAATAA
TGTCTTTTTAAAAATACTGGCACCAAAAGAAATAGATCCAGATCTACTTGGTTGTCAAGT
GGACAATCAAATGATAAACTTTAAGACCTTGTATACCATATTGAAAGGAAGAGGCTGAC
AATAAGGTTTGACAGAGGGGAACAGAAGAAAATAATATGATTTATTAGCACAACGTGG
TACTATTTGCCATTTAAAACTAGAACAGGTATATAAGCTAATATTGATACAATGATGATT
AACTATGAATTCTTAAGACTTGCATTTAAATGTGACATTCTTAAAAAAAGAAGAGAAAG
AATTTTAAGAGTAGCAGTATATATGTCTGTGCTCCCTAAAAGTTGTACTTCATTTCTTTTC
CATACACTGTGTGCTATTTGTGTTAACATGGAAGAGGATTCATTGTTTTTATTTTTATTTT
 
TTTAATTTTTTCTTTTTTATTAAGCTAGCATCTGCCCCAGTTGGTGTTCAAATAGCACTTG
ACTCTGCCTGTGATATCTGTATCTTTTCTCTAATCAGAGATACAGAGGTTGAGTATAAAA
TAAACCTGCTCAGATAGGACAATTAAGTGCACTGTACAATTTTCCCAGTTTACAGGTCTA
TACTTAAGGGAAAAGTTGCAAGAATGCTGAAAAAAAATTGAACACAATCTCATTGAGG
AGCATTTTTTAAAAACTAAAAAAAAAAAAACTTTGCCAGCCATTTACTTGACTATTGAG
CTTACTTACTTGGACGCAACATTGCAAGCGCTGTGAATGGAAACAGAATACACTTAACA
TAGAAATGAATGATTGCTTTCGCTTCTACAGTGCAAGGATTTTTTGTACAAAACTTTTT
TAAATATAAATGTTAAGAAAAATTTTTTTAAAAAACACTTCATTATGTTTAGGGGGA
ACTGCATTTTAGGGTTCCATTGTCTTGGTGGTGTTACAAGACTTGTTATCCATTTAAAAA
TGGTAGTGGAAATTCTATGCCTTGGATACACACCGCTCTTCAGGTTGTAAAAAAAAAAA
ACATACATTGGGGAAAGGTTTAAGATTATATAGTACTTAAATATAGGAAAATGCACACT
CATGTTGATTCCTATGCTAAAATACATTTATGGTCTTTTTTCTGTATTTCTAGAATGGTAT
TTGAATTAAATGTTCATCTAGTGTTAGGCACTATAGTATTTATATTGAAGCTTGTATTTTT
AACTGTTGCTTGTTCTCTTAAAAGGTATCAATGTACCTTTTTTGGTAGTGGAAAAAAAAA
AGACAGGCTGCCACAGTATATTTTTTTAATTTGGCAGGATAATATAGTGCAAATTATTTG
TATGCTTCAAAAAAAAAAAAAAGAGAGAAACAAAAAAGTGTGACATTACAGATGAGAA
GCCATATAATGGCGGTTTGGGGGAGCCTGCTAGAATGTCACATGGATGGCTGTCATAGG
GGTTGTACATATCCTTTTTTGTTCCTTTTTCCTGCTGCCATACTGTATGCAGTACTGCAAG
CTAATAACGTTGGTTTGTTATGTAGTGTGCTTTTTGTCCCTTTCCTTCTATCACCCTACAT
TCCAGCATCTTACCTTCATATGCAGTAAAAGAAAGAAAGAAAAAAAAAGGAAAAAAAA
AAAAAAACCAATGTTTTGCAGTTTTTTCATTGCCAAAAACTAAATGGTGCTTTATATTT
AGATTGGAAAGAATTTCATATGCAAAGCATATTAAAGAGAAAGCCCGCTTTAGTCAATA
CTTTTTTGTAAATGGCAATGCAGAATATTTTGTTATTGGCCTTTTCTATTCCTGTAATGAA
AGCTGTTTGTCGTAACTTGAAATTTTATCTTTTACTATGGGAGTCACTATTTATTATTGCT
TATGTGCCCTGTTCAAAACAGAGGCACTTAATTTGATCTTTTATTTTTCTTTGTTTTTATT
TTTTTTTTTATTTAGATGACCAAAGGTCATTACAACCTGGCTTTTTATTGTATTTGTTTCT
GGTCTTTGTTAAGTTCTATTGGAAAAACCACTGTCTGTGTTTTTTTGGCAGTTGTCTGCAT
TAACCTGTTCATACACCCATTTTGTCCCTTTATTGAAAAAATAAAAAAAAATTAAAGTACA
 as described by NM_018014.3.

Accordingly, provided herein are inhibitors of BCL11A expression or activity for increasing fetal hemoglobin expression. As used herein, an "inhibitor of BCL11A" or "BCL11A inhibitor" refers to an agent or compound, that inhibits one or more downstream effector pathways mediated by BCL11A, including fetal hemoglobin expression, as the term is used herein. Thus, the term BCL11A inhibitor refers to an agent that inhibits expression of the BCL11A polypeptide or polynucleotide encoding BCL11A, or one that binds to, partially or totally blocks stimulation, decreases, prevents, delays activation, inactivates, desensitizes, or down regulates the activity of the BCL11A polypeptide or polynucleotide encoding BCL11A, such that the agent causes an increase in fetal hemoglobin expression. Such BCL11A inhibitors can *e.g.,* inhibit BCL11A expression, *e.g.,* BCL11A translation, post-translational processing of BCL11A, stability, degradation, or nuclear localization of the BCL11A polypeptide, or transcription, post transcriptional processing, stability or degradation of a polynucleotide encoding BCL11A, or bind to, partially or totally block stimulation, DNA binding, or transcription factor activity of BCL11A.

Different inhibitors of BCL11A, whether an inhibitor of BCL11a activity or an inhibitor of BCL11a expression can have differing types of side effects *in vivo* and varying BCL11a inhibition effects that lead to different increases in HbF expression. Using more than one inhibitor of BCL11A from the same category and/or from a different category can allow a clinician to develop the proper dosage of each inhibitor in the composition in order to adjust the level of undesired side effects or toxicity *in vivo* while achieving sufficient increase in HbF expression to amelioration of the symptoms of a hemoglobinpathy in a mammal. Accordingly, in some embodiments of the aspects described herein, a composition comprising a BCL11A inhibitor can comprise more than one BCL11A inhibitor.

### Antibody Inhibitors of BCL11A

In some embodiments of the compositions and methods described herein, a BCL11A inhibitor is an antibody or antibody fragment thereof that specifically binds to BCL11A and can be used for the inhibition of the factor in vivo. Antibodies to BCL11A are commercially available and can be raised by one of skill in the art using well known methods. The BCL11A inhibitory activity of a given antibody, or, for that matter, any BCL11A inhibitor, can be assessed using methods known in the art or described herein - to avoid doubt, an antibody or antibody fragment thereof that inhibits BCL11A expression or activity useful in the compositions and methods described herein will cause an increase in fetal hemoglobin expression.

Antibody inhibitors of BCL11A for use in the composition and methods described herein include complete immunoglobulins, antigen binding fragments of immunoglobulins, as well as antigen-binding fragments that comprise antigen binding domains of immunoglobulins. "Antigen-binding fragments" of immunoglobulins include, for example, Fab, Fab', F(ab')2, scFv and dAbs. Modified antibody formats have been developed which retain binding specificity, but have other characteristics that may be desirable, including for example, bispecificity, multivalence (more than two binding sites), and compact size (*e*.*g*., binding domains alone).

Antibody inhibitors of BCL11A for use in the composition and methods described herein can be obtained from commercial sources such as AbCam (Cambridge, MA), New England Biolabs (Ipswich, MA), Santa Cruz Biotechnologies (Santa Cruz, CA), Biovision (Mountain View, California), R&D Systems (Minneapolis, MN), and Cell Signaling (Danvers, MA), among others. Antibodies can also be raised against a polypeptide or portion of a polypeptide by methods known to those skilled in the art. Antibodies are readily raised in animals such as rabbits or mice by immunization with the gene product or a fragment thereof. Immunized mice are particularly useful for providing sources of B cells for the manufacture of hybridomas, which in turn are cultured to produce large quantities of monoclonal antibodies. Antibody manufacture methods are described in detail, for example, in Harlow et al., Eds., Antibodies: A Laboratory Manual, Cold Spring Harbor Laboratory, New York (1988). While both polyclonal and monoclonal antibody inhibitors of BCL11A can be used in the methods described herein, it is preferred that a monoclonal antibody inhibitor of BCL11A is used where conditions require increased specificity for a particular protein.

As antibodies can be modified in a number of ways, the term "antibody" should be construed as covering any specific binding member or substance having a binding domain with the required specificity for BCL11A. Thus, this term covers antibody fragments, derivatives, functional equivalents and homologues of antibodies, including any polypeptide comprising an immunoglobulin binding domain, whether natural or wholly or partially synthetic. Chimeric molecules comprising an immunoglobulin binding domain, or equivalent, fused to another polypeptide are therefore included. Cloning and expression of chimeric antibodies are described in EP-A-0120694 and EP-A-0125023 and U.S. Patent Nos. 4,816,397 and 4,816,567.

It has been shown that antigen-binding fragments of a whole antibody can perform the function of binding antigens. Accordingly, in some embodiments of the compositions and methods described herein an inhibitor of BCL11A is an "antigen-binding fragment." Examples of antigen-binding fragments for use with the compositions and methods described herein are (i) the Fab fragment consisting of VL, VH, CL and CH1 domains; (ii) the Fd fragment consisting of the VH and CHI domains; (iii) the Fv fragment consisting of the VL and VH domains of a single antibody; (iv) the dAb fragment (Ward, E.S. et al, Nature 341, 544-546 (1989)) which consists of a VH domain; (v) isolated CDR regions; (vi) F(ab')2 fragments, a bivalent fragment comprising two linked Fab fragments (vii) single chain Fv molecules (scFv), wherein a VH domain and a VL domain are linked by a peptide linker which allows the two domains to associate to form an antigen binding site (Bird et al, Science, 242, 423-426, 1988; Huston et al, PNAS USA, 85, 5879-5883, 1988); (viii) multivalent antibody fragments (scFv dimers, trimers and/or tetramers (Power and Hudson, J Immunol. Methods 242: 193-204 9 (2000))(ix) bispecific single chain Fv dimers (PCT/US92/09965) and (x) "diabodies", multivalent or multispecific fragments constructed by gene fusion (WO94/13804; P. Holliger et al Proc. Natl. Acad. Sci. USA 90 6444-6448, (1993)).

### Nucleic Acid Inhibitors of BCL11A Expression

In some embodiments of the compositions and methods described herein, a BCL11A inhibitor is an RNA interference agent that specifically targets BCL11A and can be used for the inhibition of expression of BCL11A in vivo. RNA interference (RNAi) uses small interfering RNA (siRNA) duplexes that target the messenger RNA encoding a target polypeptide for selective degradation and is a powerful approach for inhibiting the expression of selected target polypeptides. siRNA-dependent post-transcriptional silencing of gene expression involves cleaving the target messenger RNA molecule at a site guided by the siRNA. "RNA interference (RNAi)," as used herein, refers to the evolutionally conserved process whereby the expression or introduction of RNA of a sequence that is identical or highly similar to a target gene results in the sequence specific degradation or specific post-transcriptional gene silencing (PTGS) of messenger RNA (mRNA) transcribed from that targeted gene (see Coburn, G. and Cullen, B. (2002) J. of Virology 76(18):9225), thereby inhibiting expression of the target gene. In some embodiments, the RNA interference agent or siRNA is a double stranded RNA (dsRNA). This process has been described in plants, invertebrates, and mammalian cells. In nature, RNAi is initiated by the dsRNA-specific endonuclease Dicer, which promotes processive cleavage of long dsRNA into double-stranded fragments termed siRNAs. siRNAs are incorporated into a protein complex (termed "RNA induced silencing complex," or "RISC") that recognizes and cleaves target mRNAs. RNAi can also be initiated by introducing nucleic acid molecules, *e*.*g*., synthetic siRNAs or RNA interfering agents, to inhibit or silence the expression of target genes. As used herein, "inhibition of target gene expression" includes any decrease in expression or protein activity or level of the target gene or protein encoded by the target gene as compared to a situation wherein no RNA interference has been induced. The decrease will be of at least 10%, 20%, 30%, 40%, 50%, 60%, 70%, 80%, 90%, 95% or 99% or more as compared to the expression of a target gene or the activity or level of the protein encoded by a target gene which has not been targeted by an RNA interfering agent.

As used herein, siRNAs also include small hairpin (also called stem loop) RNAs (shRNAs). In some embodiments, these shRNAs are composed of a short (*e*.*g*., about 19 to about 25 nucleotide) antisense strand, followed by a nucleotide loop of about 5 to about 9 nucleotides, and the analogous sense strand. Alternatively, in other embodiments, the sense strand can precede the nucleotide loop structure and the antisense strand can follow. These shRNAs can be contained in plasmids, retroviruses, and lentiviruses and expressed from, for example, the pol III U6 promoter, or another promoter (see, *e*.*g*., Stewart, et al. (2003) RNA Apr;9(4):493-501). The target gene or sequence of the RNA interfering agent can be a cellular gene or genomic sequence, *e.g.,* the human BCL11A genomic sequence. An siRNA can be substantially homologous to the target gene or genomic sequence, or a fragment thereof, *i.e.,* the BCL11A gene or mRNA. As used in this context, the term "homologous" is defined as being substantially identical, sufficiently complementary, or similar to the target BCL11A mRNA, or a fragment thereof, to effect RNA interference of the target BCL11A. In addition to native RNA molecules, RNA suitable for inhibiting or interfering with the expression of a target sequence include RNA derivatives and analogs. Preferably, the siRNA is identical to its target. The siRNA preferably targets only one sequence.

Each of the RNA interfering agents, such as siRNAs, can be screened for potential off-target effects by, for example, expression profiling. Such methods are known to one skilled in the art and are described, for example, in Jackson et al. Nature Biotechnology 6:635-637, 2003. In addition to expression profiling, one can also screen the potential target sequences for similar sequences in the sequence databases to identify potential sequences which may have off-target effects. For example, according to Jackson et al. (Id.), 15, or perhaps as few as 11 contiguous nucleotides, of sequence identity are sufficient to direct silencing of non-targeted transcripts. Therefore, one can initially screen the proposed siRNAs to avoid potential off-target silencing using the sequence identity analysis by any known sequence comparison methods, such as BLAST. siRNA sequences are chosen to maximize the uptake of the antisense (guide) strand of the siRNA into RISC and thereby maximize the ability of RISC to target human GGT mRNA for degradation. This can be accomplished by scanning for sequences that have the lowest free energy of binding at the 5'-terminus of the antisense strand. The lower free energy leads to an enhancement of the unwinding of the 5'- end of the antisense strand of the siRNA duplex, thereby ensuring that the antisense strand will be taken up by RISC and direct the sequence-specific cleavage of the human BCL11A mRNA.

siRNA molecules need not be limited to those molecules containing only RNA, but, for example, further encompasses chemically modified nucleotides and non-nucleotides, and also include molecules wherein a ribose sugar molecule is substituted for another sugar molecule or a molecule which performs a similar function. Moreover, a non-natural linkage between nucleotide residues can be used, such as a phosphorothioate linkage. The RNA strand can be derivatized with a reactive functional group of a reporter group, such as a fluorophore. Particularly useful derivatives are modified at a terminus or termini of an RNA strand, typically the 3' terminus of the sense strand. For example, the 2'-hydroxyl at the 3' terminus can be readily and selectively derivatized with a variety of groups. Other useful RNA derivatives incorporate nucleotides having modified carbohydrate moieties, such as 2'O-alkylated residues or 2'-O-methyl ribosyl derivatives and 2'-O-fluoro ribosyl derivatives. The RNA bases can also be modified. Any modified base useful for inhibiting or interfering with the expression of a target sequence may be used. For example, halogenated bases, such as 5-bromouracil and 5-iodouracil can be incorporated. The bases can also be alkylated, for example, 7-methylguanosine can be incorporated in place of a guanosine residue. Non-natural bases that yield successful inhibition can also be incorporated. The most preferred siRNA modifications include 2'-deoxy-2'-fluorouridine or locked nucleic acid (LAN) nucleotides and RNA duplexes containing either phosphodiester or varying numbers of phosphorothioate linkages. Such modifications are known to one skilled in the art and are described, for example, in Braasch et al., Biochemistry, 42: 7967-7975, 2003. Most of the useful modifications to the siRNA molecules can be introduced using chemistries established for antisense oligonucleotide technology. Preferably, the modifications involve minimal 2'-O-methyl modification, preferably excluding such modification. Modifications also preferably exclude modifications of the free 5'-hydroxyl groups of the siRNA. The Examples herein provide specific examples of RNA interfering agents, such as shRNA molecules that effectively target BCL11A mRNA.

This included a non-targeting pool (D-001810-10) and a BCL11A targeting pool (L-006996-00). The BCL11A siRNA target sequences used are presented in Table 1.

**Table 1: BCL11A siRNA Targeting Sequences**

| | |
|---|---|
| **SEQ ID NO: 3** | GAGCACAAACGGAAACAAU |
| **SEQ ID NO: 4** | GCCACAGGAUGACGAUUGU |
| **SEQ ID NO: 5** | GCACUUAAGCAAACGGGAA |
| **SEQ ID NO: 6** | ACAGAACACUCAUGGAUUA |

These siRNAs were prepared as 100 µM stocks, as recommended by the manufacturer. Aliquots were stored at -80oC until use. siRNAs were introduced into expanded and differentiating CD34 cells using the Microporator-Mini (Digital Bio Technology). Manufacturer's protocols were followed and after screening a number of conditions, it was found that with a single pulse of 1800 V (using a pulse width of 20 ms) the best transduction efficiency was obtained, as assessed using a GFP reporter plasmid. Transduction efficiency was estimated to be ∼50-60% of viable cells. Typically, ∼250,000 cells were transduced with 4 µl of siRNAs in a volume of ∼15 µl. Cells were then seeded into fresh differentiation medium. Accordingly, in some embodiments of the aspects described herein, an inhibitor of BCL11A is an shRNA selected from the group consisting of SEQ ID NO: 3 to SEQ ID NO:6.

shRNA clones in the pLKO vector were obtained from a large collection of shRNAs that has been previously described (Moffat et al., Cell 124:1283 (2006)). Two shRNAs targeting BCL11A were obtained with the sequences presented Table 2:

**Table 2**

| | |
|---|---|
| **SEQ ID NO**: **7** | CCGGCGCACAGAACACTCATGGATTCTCGAGAATCCATGAGTGTTCTGT GCGTTTTTG |
| **SEQ ID NO: 8** | CCGGCCAGAGGATGACGATTGTTTACTCGAGTAAACAATCGTCATCCTC TGGTTTTTG |

These shRNAs were chosen, since they target both of the major isoforms of BCL11A found in erythroid cells. Accordingly, in some embodiments of the aspects described herein, an inhibitor of BCL11A is an shRNA selected from the group consisting of SEQ ID NO: 7 and SEQ ID NO:8.

In some embodiments, the RNA interference agent is delivered or administered in a pharmaceutically acceptable carrier. Additional carrier agents, such as liposomes, can be added to the pharmaceutically acceptable carrier. In another embodiment, the RNA interference agent is delivered by a vector encoding the small hairpin RNA (shRNA) in a pharmaceutically acceptable carrier to the cells in an organ of an individual. The shRNA is converted by the cells after transcription into siRNA capable of targeting, for example, BCL11A.

In some embodiments, the vector is a regulatable vector, such as tetracycline inducible vector. Methods described, for example, in Wang et al. Proc. Natl. Acad. Sci. 100: 5103-5106, using pTet-On vectors (BD Biosciences Clontech, Palo Alto, CA) can be used. In some embodiments, the RNA interference agents used in the methods described herein are taken up actively by cells in vivo following intravenous injection, *e*.*g*., hydrodynamic injection, without the use of a vector, illustrating efficient in vivo delivery of the RNA interfering agents. One method to deliver the siRNAs is catheterization of the blood supply vessel of the target organ. Other strategies for delivery of the RNA interference agents, *e.g.,* the siRNAs or shRNAs used in the methods of the invention, can also be employed, such as, for example, delivery by a vector, *e.g.,* a plasmid or viral vector, *e.g.,* a lentiviral vector. Such vectors can be used as described, for example, in Xiao-Feng Qin et al. Proc. Natl. Acad. Sci. U.S.A., 100: 183-188. Other delivery methods include delivery of the RNA interfering agents, *e.g.,* the siRNAs or shRNAs targeting BCL11A described herein, using a basic peptide by conjugating or mixing the RNA interfering agent with a basic peptide, *e.g.,* a fragment of a TAT peptide, mixing with cationic lipids or formulating into particles. The RNA interference agents, *e.g.,* the siRNAs targeting BCL11A mRNA, can be delivered singly, or in combination with other RNA interference agents, *e*.*g*., siRNAs, such as, for example siRNAs directed to other cellular genes.

Synthetic siRNA molecules, including shRNA molecules, can be generated using a number of techniques known to those of skill in the art. For example, the siRNA molecule can be chemically synthesized or recombinantly produced using methods known in the art, such as using appropriately protected ribonucleoside phosphoramidites and a conventional DNA/RNA synthesizer (see, *e*.*g*., Elbashir, S.M. et al. (2001) Nature 411:494-498; Elbashir, S.M., W. Lendeckel and T. Tuschl (2001) Genes & Development 15:188-200; Harborth, J. et al. (2001) J. Cell Science 114:4557-4565; Masters, J.R. et al. (2001) Proc. Natl. Acad. Sci., USA 98:8012-8017; and Tuschl, T. et al. (1999) Genes & Development 13:3191-3197). Alternatively, several commercial RNA synthesis suppliers are available including, but not limited to, Proligo (Hamburg, Germany), Dharmacon Research (Lafayette, CO, USA), Pierce Chemical (part of Perbio Science , Rockford, IL , USA), Glen Research (Sterling, VA, USA), ChemGenes (Ashland, MA, USA), and Cruachem (Glasgow, UK). As such, siRNA molecules are not overly difficult to synthesize and are readily provided in a quality suitable for RNAi. In addition, dsRNAs can be expressed as stem loop structures encoded by plasmid vectors, retroviruses and lentiviruses (Paddison, P.J. et al. (2002) Genes Dev. 16:948-958; McManus, M.T. et al. (2002) RNA 8:842-850; Paul, C.P. et al. (2002) Nat. Biotechnol. 20:505-508; Miyagishi, M. et al. (2002) Nat. Biotechnol. 20:497-500; Sui, G. et al. (2002) Proc. Natl. Acad. Sci., USA 99:5515-5520; Brummelkamp, T. et al. (2002) Cancer Cell 2:243; Lee, N.S., et al. (2002) Nat. Biotechnol. 20:500-505; Yu, J.Y., et al. (2002) Proc. Natl. Acad. Sci., USA 99:6047-6052; Zeng, Y., et al. (2002) Mol. Cell 9:1327-1333; Rubinson, D.A., et al. (2003) Nat. Genet. 33:401-406; Stewart, S.A., et al. (2003) RNA 9:493-501). These vectors generally have a polIII promoter upstream of the dsRNA and can express sense and antisense RNA strands separately and/or as a hairpin structures. Within cells, Dicer processes the short hairpin RNA (shRNA) into effective siRNA.

The targeted region of the siRNA molecule for use in the compositions and methods described herein can be selected from a given target gene sequence, *e.g..,* a BCL11A coding sequence, beginning from about 25 to 50 nucleotides, from about 50 to 75 nucleotides, or from about 75 to 100 nucleotides downstream of the start codon. Nucleotide sequences can contain 5' or 3' UTRs and regions nearby the start codon. One method of designing a siRNA molecule for use in the compositions and methods described herein involves identifying the 23 nucleotide sequence motif AA(N19)TT (SEQ. ID. NO: 21) (where N can be any nucleotide) and selecting hits with at least 25%, 30%, 35%, 40%, 45%, 50%, 55%, 60%, 65%, 70% or 75% G/C content. The "TT" portion of the sequence is optional. Alternatively, if no such sequence is found, the search can be extended using the motif NA(N21), where N can be any nucleotide. In this situation, the 3' end of the sense siRNA may be converted to TT to allow for the generation of a symmetric duplex with respect to the sequence composition of the sense and antisense 3' overhangs. The antisense siRNA molecule can then be synthesized as the complement to nucleotide positions 1 to 21 of the 23 nucleotide sequence motif. The use of symmetric 3' TT overhangs can be advantageous to ensure that the small interfering ribonucleoprotein particles (siRNPs) are formed with approximately equal ratios of sense and antisense target RNA-cleaving siRNPs (Elbashir et al., (2001) supra and Elbashir et al., 2001 supra). Analysis of sequence databases, including but not limited to the NCBI, BLAST, Derwent and GenSeq as well as commercially available oligosynthesis companies such as OLIGOENGINE®, can also be used to select siRNA sequences against EST libraries to ensure that only one gene is targeted.

### Delivery of RNA Interfering Agents

Methods of delivering RNA interference agents, *e.g.,* an siRNA, or vectors containing an RNA interference agent, to the target cells, *e*.*g*., lymphocytes or other desired target cells, for uptake include injection of a composition containing the RNA interference agent, *e.g.,* an siRNA targeting BCL11A, or directly contacting the cell, *e.g.,* a lymphocyte, with a composition comprising an RNA interference agent, *e.g.,* an siRNA targeting BCL11A. In other embodiments, an RNA interference agent, *e.g.,* an siRNA targeting BCL11A, can be injected directly into any blood vessel, such as vein, artery, venule or arteriole, via, *e*.*g*., hydrodynamic injection or catheterization. Administration can be by a single injection or by two or more injections. The RNA interference agent is delivered in a pharmaceutically acceptable carrier. One or more RNA interference agents can be used simultaneously. In some preferred embodiments, only one siRNA that targets human BCL11A is used.

In some embodiments, specific cells are targeted with RNA interference, limiting potential side effects of RNA interference caused by non-specific targeting of RNA interference. The method can use, for example, a complex or a fusion molecule comprising a cell targeting moiety and an RNA interference binding moiety that is used to deliver RNA interference effectively into cells. For example, an antibody-protamine fusion protein when mixed with siRNA, binds siRNA and selectively delivers the siRNA into cells expressing an antigen recognized by the antibody, resulting in silencing of gene expression only in those cells that express the antigen. The siRNA or RNA interference-inducing molecule binding moiety is a protein or a nucleic acid binding domain or fragment of a protein, and the binding moiety is fused to a portion of the targeting moiety. The location of the targeting moiety can be either in the carboxyl-terminal or amino-terminal end of the construct or in the middle of the fusion protein. A viral-mediated delivery mechanism can also be employed to deliver siRNAs to cells *in vitro* and in vivo as described in Xia, H. et al. (2002) Nat Biotechnol 20(10): 1006). Plasmid- or viral-mediated delivery mechanisms of shRNA can also be employed to deliver shRNAs to cells *in vitro* and in vivo as described in Rubinson, D.A., et al. ((2003) Nat. Genet. 33:401-406) and Stewart, S.A., et al. ((2003) RNA 9:493-501). The RNA interference agents targeting BCL11A, *e.g.,* the siRNAs or shRNAs, can be introduced along with components that perform one or more of the following activities: enhance uptake of the RNA interfering agents, *e.g.,* siRNA, by the cell, *e.g.,* lymphocytes or other cells, inhibit annealing of single strands, stabilize single strands, or otherwise facilitate delivery to the target cell and increase inhibition of the target BCL11A. The dose of the particular RNA interfering agent will be in an amount necessary to effect RNA interference, *e*.*g*., post translational gene silencing (PTGS), of the particular target gene, thereby leading to inhibition of target gene expression or inhibition of activity or level of the protein encoded by the target gene.

### Small Molecule Inhibitors of BCL11A

In some embodiments of the compositions and methods described herein, a BCL11A inhibitor is a small molecule inhibitor or agent that specifically targets BCL11A and can be used for the inhibition of expression of BCL11A *in vivo* for increasing fetal hemoglobin expression.

The term "agent" as used herein in reference to an inhibitor of BCL11A means any compound or substance such as, but not limited to, a small molecule, nucleic acid, polypeptide, peptide, drug, ion, etc. An "agent" can be any chemical, entity, or moiety, including, without limitation, synthetic and naturally-occurring proteinaceous and non-proteinaceous entities. In certain embodiments, as described herein, agents are small molecules having a chemical moiety. For example, chemical moieties include unsubstituted or substituted alkyl, aromatic, or heterocyclyl moieties. Compounds can be known to have a desired activity and/or property, *e*.*g*., inhibit BCL11A expression or activity activity, or can be selected from a library of diverse compounds, using, for example, the screening methods described herein.

As used herein, the term "small molecule" refers to a chemical agent which can include, but is not limited to, a peptide, a peptidomimetic, an amino acid, an amino acid analog, a polynucleotide, a polynucleotide analog, an aptamer, a nucleotide, a nucleotide analog, an organic or inorganic compound (*e*.*g*., including heterorganic and organometallic compounds) having a molecular weight less than about 10,000 grams per mole, organic or inorganic compounds having a molecular weight less than about 5,000 grams per mole, organic or inorganic compounds having a molecular weight less than about 1,000 grams per mole, organic or inorganic compounds having a molecular weight less than about 500 grams per mole, and salts, esters, and other pharmaceutically acceptable forms of such compounds.

In some embodiments, a small molecule inhibitor of BCL11A selectively binds to BCL11A. As used herein, "selectively binds" or "specifically binds" refers to the ability of a BCL11A inhibitor described herein to bind to the BCL11A polypeptide, with a K_{D} 10⁻⁵ M (10000 nM) or less, *e.g.,* 10⁻⁶ M or less, 10⁻⁷ M or less, 10⁻⁸ M or less, 10⁻⁹ M or less, 10⁻¹⁰ M or less, 10⁻¹¹ M or less, or 10⁻¹² M or less. For example, if an inhibitor described herein binds to the BCL11A polypeptide with a K_{D} of 10⁻⁵ M or lower, but not to other molecules, or a related homologue, then the agent is said to specifically bind the BCL11A polypeptide. Specific binding can be influenced by, for example, the affinity and avidity of the agonist or inhibitor and the concentration of the agonist or inhibitor used. A person of ordinary skill in the art can determine appropriate conditions under which the inhibitors described herein selectively bind using any suitable methods, such as titration of a BCL11A inhibitor in a suitable cell binding assay.

### Chemical Inducers of HbF

The compositions described herein comprise, in addition to one or more BCL11A inhibitors, one or more HbF chemical inducers, as the term is used herein. Such chemical inducers include, but are not limited to, DNA methylation inhibitors, HDAC inhibitors, amide analogues of trichostatin A, hydroxamic acid analogues of trapoxin or scriptaid and analogues, cytotoxic chemotherapeutic agents, erythropoietin (EPO) preparations, and short chain fatty acids (SCFAs) and derivatives (SCFADs), etc. Such combinatorial compositions are used herein, in part, because the different HbF chemical inducer members of the same category can have varying degrees and types of side effects *in vivo* and varying HbF induction activity. Combinations of HbF chemical inducers from the same category and/or from a different category can allow the clinician to develop the proper dosage of each inducer in the composition in order to adjust the level of undesired side effects or toxicity *in vivo* while achieving sufficient increase in HbF expression to amelioration of the symptoms of a hemoglobinpathy in a mammal. In addition, different combinations of HbF chemical inducers can have synergistic effects with other inducers or BCL11A inhibitors, as demonstrated herein.

Accordingly, provided herein, in some aspects, are compositions for increasing fetal hemoglobin levels in a mammal in need thereof, the compositions comprising one or more inhibitors of BCL11A and one or more HbF chemical inducers. The HbF chemical inducer has been shown, at the minimum, to induce HbF expression in erythroid cells *in vitro.* In some embodiments of these aspects, the HbF chemical inducer is an "epigenetic modifier." The term "epigenetic modifier" refers to a HbF chemical inducer that acts to increase the expression and/or production of fetal hemoglobin by impacting or modulating one or more epigenetic pathways, such as, for example, histone deacetylation or DNA methylation. Accordingly, as used herein, epigenetic modifiers include agents or compounds that inhibit DNA methylation and histone deacetylation, such as an inhibitor of DNA methylation or an inhibitor of HDAC, as further described herein.

### DNA Methylation Inhibitors

DNA methylation is an epigenetic process with several effects, including chromatin structure modulation, transcriptional repression and the suppression of transposable elements. DNA methylation has been shown to play an important role in cancer initiation and progression by suppression of genes essential for the control of normal cell growth and differentiation. "DNA methylation," as used herein, refers to the addition of a methyl group to the 5' position of cytosine. In mammals, DNA methylation is catalyzed primarily by three DNA methyltransferases, DNMT1, DNMT3a, and DNMT3b, and occurs largely at the cytosine residues contained within the dinucleotide sequence cytosine-phosphate diesterguanine (CpG). Fully methylated CpG islands (areas of increased density of CpGs) are found exclusively in the promoter regions of selected imprinted autosomal genes and various silenced genes on the inactivated X chromosome of females.

As demonstrated herein, DNA methylation inhibitors have a synergistic effect with BCL11A inhibition or loss-of-function leading to much greater HbF production than when either a DNA methylation inhibitor or BCL11A inhibition is used alone. Accordingly, in some embodiments, a DNA methylation inhibitor is used as the HbF chemical inducer in the compositions and methods described herein. As used herein, the terms "DNA methylation inhibitor" or "DNA methyltransferase inhibitor" refer to an agent or compound that directly or indirectly perturbs, disrupts, blocks, modulates or inhibits the methylation of DNA, including the ionic, salt, solvate, isomers, tautomers, N-oxides, ester, prodrugs, isotopes and protected forms thereof, for example, by preventing or antagonizing activity of DNA methyltransferases, or by acting as analogs of the nucleoside deoxycytidine. In some embodiments of the compositions and methods described herein, a DNA methylation inhibitor is selected from the group consisting of 5-azacytidine (azacitidine), 5-aza-2'-deoxycytidine (decitabine), 1-β-d-arabinofuranosyl-5-azacytosine (fazarabine) and dihydro-5-azacytidine (DHAC).

For example, SuperGen Inc and MGI Pharma Inc have developed decitabine (Dacogen), an inhibitor of DNA methyltransferase, that prevents methylation of cytosine residues on DNA and leadsto hypomethylation of gene promoters, thereby reactivating silenced genes. Decitabine or 5-aza-2'-deoxycytidine, is an antagonist of its related natural nucleoside, deoxycytidine. The only structural difference between these two compounds is the presence of a nitrogen at position 5 of the cytosine ring in decitabine as compared to a carbon at this position for deoxycytidine. Decitabine possesses multiple pharmacological characteristics. At a molecular level, it is capable of specifically inhibiting cell growth at S phase and DNA methylation. At a cellular level, decitabine can induce cell differentiation and exert hematological toxicity. Despite having a short half life in vivo, decitabine has excellent tissue distribution.

The most prominent function of decitabine is its ability to specifically and potently inhibit DNA methylation. Inside a cell, decitabine is first converted into its active form, the phosphorylated 5-aza-deoxycytidine, by deoxycytidine kinase which is primarily synthesized during the S phase of the cell cycle. The affinity of decitabine for the catalytical site of deoxycytidine kinase is similar to the natural substrate, deoxycytidine. Momparler et al. (1985) 30:287-299. After conversion to its triphosphate form by deoxycytidine kinase, decitabine is incorporated into replicating DNA at a rate similar to that of the natural substrate, dCTP. Bouchard and Momparler (1983) Mol. Pharmacol. 24:109-114. Incorporation of decitabine into the DNA strand has a hypomethylation effect. Each class of differentiated cells has its own distinct methylation pattern. After chromosomal duplication, in order to conserve this pattern of methylation, the 5-methylcytosine on the parental strand serves to direct methylation on the complementary daughter DNA strand. Substituting the carbon at the 5 position of the cytosine for a nitrogen interferes with this normal process of DNA methylation. The replacement of 5-methylcytosine with decitabine at a specific site of methylation produces an irreversible inactivation of DNA methyltransferase, presumably due to formation of a covalent bond between the enzyme and decitabine. Juttermann et al. (1994) Proc. Natl. Acad. Sci. USA 91:11797-11801. Decitabine/Dacogen and other analogues thereof can be made as outlined in U.S. Pat. No. 3,432,549 and further discussed on WO 006/017278 and WO 2006/037024 to SuperGen Inc.

Another DNA methylase inhibitor for use with the compositions and methods for increasing HbF production is temozolomide (3,4-dihydro-3-methyl-4-oxoimidazo[5,1-d]-as-tetrazine-8-carboxamide). Temozolomide is commercially available for example from Schering Corporation under the trade name Temodar, or can be prepared for example as described in German patent specification No. 3231255, or by processes analogous thereto, and is used for the treatment of glioblastoma multiforme, and first-line treatment of patients with advanced metastatic malignant melanoma. This compound undergoes rapid chemical conversion at physiological pH to the active compound, monomethyl triazeno imidazole carboxamide (MTIC) which is responsible for the methylation of DNA at the O⁶ position of guanine residues, which leads to a suppression in expression of DNA methyltransferase and thus results in hypomethylation).

Another DNA methylation inhibitor for use in some embodiments of the compositions and methods described herein for increasing HbF production is azacytidine (5-azacitidine, 5-azacytidine or Vidaza). Other DNA methylation inhibitors include pseudoisocytidine and 5-fluoro-2'-deoxycytidine, which are further analogs with modifications of the 5 position of the pyrimidine ring.

Accordingly, in some embodiments, the DNA methylation inhibitor is a cytidine analog or derivative. Examples of cytidine analogs or derivatives include, but are not limited to, 5-azacytidine and 5-aza-2'-deoxycytidine. In some embodiments, the DNA methylation inhibitor is 5-aza-2'-deoxycytidine (5-aza-CdR or decitabine).

### Histone Deacetylation Inhibitors

The amount of acetylation on the histones is controlled by the opposing activities of two types of enzymes, histone acetyl transferase (HATs) and histone deacetylases (HDACs). Substrates for these enzymes include e-amino groups of lysine residues located in the amino-terminal tails of the histones H3, H4, H2A, and H2B. These amino acid residues are acetylated by HATs and deacetylated by HDACs. With the removal of the acetyl groups from the histone lysine by HDACs, a positive charge is restored to the lysine residue, thereby condensing the structure of nucleosome and silencing the genes contained within. Thus, to activate these genes silenced by deacetylase of histones, the activity of HDACs is inhibited. With the inhibition of HDAC, histones are acetylated and the DNA that is tightly wrapped around a deacetylated histone core relaxes. The opening of DNA conformation leads to expression of specific genes.

In addition to deacelation of histones, HDACs can also regulated gene expression by deacetylating transcription factors, such as p53 (a tumor suppressor gene), GATA-1, TFIIE, and TFIIF. Gu and Roeder (1997) Cell 90:595-606 (p53); and Boyes et al. (1998) Nature 396:594-598 (GATA-1). HDACs also participate in cell cycle regulation, for example, by transcription repression which is mediated by RB tumor suppressor proteins recruiting HDACs. Brehm et al. (1998) Nature 391:597-601. Thus, inhibition of HDACs can activate expression of tumor suppressor genes such as p53 and RB and as a result promote cell growth arrest, differentiation and apoptosis induced by these genes.

As demonstrated herein, histone deacetylation inhibitors have a unexpected synergistic effect with BCL11A inhibition or loss-of-function on increasing HbF production. Accordingly, in some embodiments, a histone deacetylation inhibitor is used as the HbF chemical inducer in the compositions and methods described herein. As used herein, a "histone deacetylation (HDAC) inhibitor" or "inhibitor of histone deacetylation (HDAC)" refers to a compound which inhibits or modulates the activity of histone deacetylases (HDAC), including the ionic, salt, solvate, isomers, tautomers, N-oxides, ester, prodrugs, isotopes and protected forms thereof. Inhibitors of HDACs for use in the compositions and methods described herein include, but are not limited to, the following structural classes: 1) hydroxamic acids, 2) cyclic peptides, 3) benzamides, and 4) short-chain fatty acids.

Examples of hydroxamic acids and hydroxamic acid derivatives, but are not limited to, trichostatin A (TSA), suberoylanilide hydroxamic acid (SAHA), oxamflatin, suberic bishydroxamic acid (SBHA), m-carboxy-cinnamic acid bishydroxamic acid (CBHA), and pyroxamide. TSA was isolated as an antifungi antibiotic (Tsuji et al (1976) J. Antibiot (Tokyo) 29:1-6) and found to be a potent inhibitor of mammalian HDAC (Yoshida et al. (1990) J. Biol. Chem. 265:17174-17179). Other hydroxamic acid-based HDAC inhibitors, such as SAHA, SBHA, and CBHA, are synthetic compounds that are able to inhibit HDAC at micromolar concentration or lower *in vitro* or in vivo. Glick et al. (1999) Cancer Res. 59:4392-4399. These hydroxamic acid-based HDAC inhibitors all possess an essential structural feature: a polar hydroxamic terminal linked through a hydrophobic methylene spacer (*e.g.* 6 carbon at length) to another polar site which is attached to a terminal hydrophobic moiety (*e*.*g*., benzene ring). Compounds developed having such essential features also fall within the scope of the hydroxamic acids that can be used as HDAC inhibitors for use with the combinatorial compositions and methods of increasing HbF levels described herein .

Cyclic peptides used as HDAC inhibitors are mainly cyclic tetrapeptides. Examples of cyclic peptides include, but are not limited to, trapoxin A, apicidin and FR901228. Trapoxin A is a cyclic tetrapeptide that contains a 2-amino-8-oxo-9,10-epoxy-decanoyl (AOE) moiety. Kijima et al. (1993) J. Biol. Chem. 268:22429-22435. Apicidin is a fungal metabolite that exhibits potent, broad-spectrum antiprotozoal activitity and inhibits HDAC activity at nanomolar concentrations. Darkin-Rattray et al. (1996) Proc. Natl. Acad. Sci. USA. 93; 13143-13147. FR901228 is a depsipeptide that is isolated from Chromobacterium violaceum, and has been shown to inhibit HDAC activity at micromolar concentrations.

Examples of benzamides include, but are not limited to, MS-27-275 or N-(2-aminophenyl)-4-[N-(pyridine-3-yl-methoxycarbonyl)aminomethyl]benzamide (Saito et al. (1990) Proc. Natl. Acad. Sci. USA. 96:4592-4597). Examples of short-chain fatty acids include but are not limited to butyrates (e.g., butyric acid, arginine butyrate and phenylbutyrate (PB)). Newmark et al. (1994) Cancer Lett. 78:1-5; and Carducci et al. (1997) Anticancer Res. 17:3972-3973. In addition, depudecin which has been shown to inhibit HDAC at micromolar concentrations (Kwon et al. (1998) Proc. Natl. Acad. Sci. USA. 95:3356-3361) also falls within the scope of histone deacetylase inhibitor as described herein.

Other HDAC inhibitors for use in the combinatorial compositions and methods of treatments described herein, include, but are not limited to, JNJ-16241199 from Johnson and Johnson Inc, LAQ-824 from Novartis, MGCD-0103 from MethylGene, and PXD-101 from Prolifix, peptide chlamydocin, and A-173, also from Abbott Laboratories.

Accordingly, in some embodiments of the combinatorial compositions and methods of treatments described herein, the HDAC inhibitor is suberoylanilide hydroxamic acid (SAHA or Vorinostat). In some embodiments of the combinatorial compositions and methods of treatments described herein, the HDAC inhibitor is selected from the categories of HDAC: amide analogues of trichostatin A, hydroxamic acid analogues of trapoxin, and scriptaid and analogues thereof. In some embodiments of the combinatorial compositions and methods of treatments described herein, the HDAC inhibitor is selected from the group consisting of suberoylanilide hydroxamic acid (SAHA), N-Hydroxy-7-(4-dimethylaminobenzoyl)-aminoheptanamide (M344), N-Hydroxy-8-(4-dimethylaminobenzoyl)-aminooctanamide (M360), N-Hydroxy-6-(4-biphenylcarbonyl)-aminocapramide (M355), N-Hydroxy-6-(4-dimethylaminobenzoylamino)-capramide (MD85), (S)-Octanedioic acid hydroxyamide (1-phenethylcarbamoyl-2-phenyl-ethyl)-amide (SW68), (S)-Octanedioic acid hydroxyamide (1-benzylcarbamoyl-2-phenyl-ethyl)-amide (SW70), (S)-3-(4-Methoxy-phenyl)-2-(7-hydroxycarbamoyl-heptanoylamino)-propionic acid methyl ester (SW99), (S)-2-(7-Hydroxycarbamoyl-heptanoylamino)-3-thiophen-2-yl-propionic acid methyl ester (SW86), (S)-3-(4'-Chlorobiphenyl-4-yl)-2-(7-hydroxycarbamoylheptanoylamino)-propionic acid methyl ester (SW183), (S)-3-(3',4'-Dichlorobiphenyl-4-yl)-2-(7-hydroxycarbamoylheptanoylamino)-propionic acid methyl ester (SW187), (S)-2-(7-Hydroxycarbamoylheptanoylamino)-3-(4-methoxybiphenyl-4-yl)-propionic acid methyl ester (SW188), (S)-2-(7-Hydroxycarbamoylheptanoylamino)-3-(4'-methylbiphenyl-4-yl)-propionic acid methyl ester (SW189), (S)-3-(Phenyl)-2-(7-hydroxycarbamoyl-heptanoylamino)-propionic acid methyl ester (M232), 6-(1,3-Dioxo-1H,3H-benzo[de]isoquinolin-2-yl)-hexanoic acid hydroxyamide (HR13), 6-(4,5,6,7-Tetrachloro-1,3-dioxo-1,3-dihydroisoindol-2-yl) hexanoic acid hydroxyamide (HR10) and 6-(1,3-Dioxo-1,3-dihydroisoindol-2-yl) hexanoic acid hydroxyamide (HR11).

### Pharmaceutical Combinatorial Compositions & Therapeutic Methods of Treatment for Increasing HbF

Provided herein are combinatorial compositions comprising inhibitors of BCL11A expression or activity with epigenetic modifiers, such as inhibitors of DNA methylation and/or inhibitors of histone deactylases that provide unexpected and synergistic effects on increasing fetal hemoglobin expression. Accordingly, the combinatorial compositions described herein can be used in methods of treating a subject in need of increasing fetal hemoglobin levels or a subject having or at risk for developing a blood disorder, such as a hemoglobinopathy. The combinatorial compositions comprising inhibitors of BCL11A expression or activity, such as an antibody or antibody fragment specific for BCL11A or a BCL11 specific RNA interference agent, with epigenetic modifiers, such as inhibitors of DNA methylation (*e*.*g*., 5-aza-2'-deoxycytidine) and/or inhibitors of histone deactylases (*e.g.,* SAHA) can be administered to a subject in need thereof by any appropriate route which results in an effective treatment in the subject.

Accordingly, in some aspects, provided herein are methods for increasing fetal hemoglobin levels in a subject, such as a mammal, in need thereof. Such methods comprise administering to a subject in need of increased fetal hemoglobin a therapeutically effective amount of a combinatorial composition comprising inhibitors of BCL11A expression or activity and epigenetic modifiers, such as inhibitors of DNA methylation and/or inhibitors of histone deactylases described herein, such that fetal hemoglobin is increased in the subject, relative to expression prior to the treatment.

In some aspects, provided herein are methods for increasing fetal hemoglobin levels in a subject, such as a mammal, in need thereof, comprising administering to a subject in need of increased fetal hemoglobin an effective amount of a composition comprising at least one inhibitor of BCL11A and at least one HbF chemical inducer, such that fetal hemoglobin is increased in the subject, relative to expression prior to the treatment.

In some aspects, provided herein are methods for increasing fetal hemoglobin levels in a subject, such as a mammal, in need thereof, comprising administering to a subject in need of increased fetal hemoglobin an effective amount of a composition comprising at least one inhibitor of BCL11A and at least one epigenetic modifier, such that fetal hemoglobin is increased in the subject, relative to expression prior to the treatment.

In some aspects, provided herein are methods for increasing fetal hemoglobin levels in a subject, such as a mammal, in need thereof, comprising administering to a subject in need of increased fetal hemoglobin an effective amount of a composition comprising at least one inhibitor of BCL11A and at least one DNA methylation inhibitor, such that fetal hemoglobin is increased in the subject, relative to expression prior to the treatment.

In some aspects, provided herein are methods for increasing fetal hemoglobin levels in a subject, such as a mammal, in need thereof, comprising administering to a subject in need of increased fetal hemoglobin an effective amount of a composition comprising at least one inhibitor of BCL11A and at least one histone deacetylation inhibitor, such that fetal hemoglobin is increased in the subject, relative to expression prior to the treatment.

In some aspects, provided herein are methods for increasing fetal hemoglobin levels in a subject, such as a mammal, in need thereof, comprising administering to a subject in need of increased fetal hemoglobin an effective amount of a composition comprising at least one inhibitor of BCL11A, at least one DNA methylation inhibitor, and at least one histone deacetylation inhibitor, such that fetal hemoglobin is increased in the subject, relative to expression prior to the treatment.

In some embodiments of these methods, the subject in need of treatment has been diagnosed with or is at risk for a hemoglobinopathy. In some such embodiments, the hemoglobinopathy is a β-hemoglobinopathy. In some such embodiments, the hemoglobinopathy is sickle cell disease. In some such embodiments, the hemoglobinopathy is β-thalassemia.

In some aspects, provided herein are methods of treatment of a blood disorder in a subject, such as a mammal, in need thereof. Such methods comprise administering to a subject having or at risk for a blood disorder, such as a mammal, with a therapeutically effective amount of a combinatorial composition comprising inhibitors of BCL11A expression or activity and epigenetic modifiers, such as inhibitors of DNA methylation and/or inhibitors of histone deactylases described herein, whereby fetal hemoglobin expression is increased in said subject, relative to expression prior to said administration.

In some aspects, provided herein are methods of treatment of a blood disorder in a subject, such as a mammal, in need thereof, comprising administering to a subject in having or at risk for a blood disorder an effective amount of a composition comprising at least one inhibitor of BCL11A and at least one HbF chemical inducer, such that fetal hemoglobin is increased in the subject, relative to expression prior to the treatment.

In some aspects, provided herein are methods of treatment of a blood disorder in a subject, such as a mammal, in need thereof, comprising administering to a subject in having or at risk for a blood disorder an effective amount of a composition comprising at least one inhibitor of BCL11A and at least one epigenetic modifier, such that fetal hemoglobin is increased in the subject, relative to expression prior to the treatment.

In some aspects, provided herein are methods of treatment of a blood disorder in a subject, such as a mammal, in need thereof, comprising administering to a subject in having or at risk for a blood disorder an effective amount of a composition comprising at least one inhibitor of BCL11A and at least one DNA methylation inhibitor, such that fetal hemoglobin is increased in the subject, relative to expression prior to the treatment.

In some aspects, provided herein are methods of treatment of a blood disorder in a subject, such as a mammal, in need thereof, comprising administering to a subject in having or at risk for a blood disorder an effective amount of a composition comprising at least one inhibitor of BCL11A and at least one histone deacetylation inhibitor, such that fetal hemoglobin is increased in the subject, relative to expression prior to the treatment.

In some aspects, provided herein are methods of treatment of a blood disorder in a subject, such as a mammal, in need thereof, comprising administering to a subject in having or at risk for a blood disorder an effective amount of a composition comprising at least one inhibitor of BCL11A, at least one DNA methylation inhibitor, and at least one histone deacetylation inhibitor, such that fetal hemoglobin is increased in the subject, relative to expression prior to the treatment.

In some embodiments of these methods, the subject having a blood disorder has a hemoglobinopathy. In some such embodiments, the hemoglobinopathy is a β-hemoglobinopathy. In some such embodiments, the hemoglobinopathy is sickle cell disease. In some such embodiments, the hemoglobinopathy is β-thalassemia.In some embodiments of these methods, the method further comprises selecting a subject diagnosed with or at risk for a blood disorder. In some such embodiments, the selecting step involves a genetic test.

The term "blood disorders" as used herein includes hemoglobinopathies and thalassemias. Blood disorders include disorders that can be treated, prevented, or otherwise ameliorated by the administration of the combinatorial composition comprising inhibitors of BCL11A expression or activity and epigenetic modifiers described herein. A blood disorder is any disorder of the blood and blood-forming organs. The term blood disorder includes nutritional anemias (*e*.*g*., iron deficiency anemia, sideropenic dysphasia, Plummer-Vinson syndrome, vitamin B 12 deficiency anemia, vitamin B 12 deficiency anemia due to intrinsic factor, pernicious anemia, folate deficiency anemia, and other nutritional anemias), myelodysplastic syndrome, bone marrow failure or anemia resulting from chemotherapy, radiation or other agents or therapies, hemolytic anemias (*e*.*g*., anemia due to enzyme disorders, anemia due to phosphate dehydrogenase (G6PD) deficiency, favism, anemia due to disorders of glutathione metabolism, anemia due to disorders of glycolytic enzymes, anemias due to disorders of nucleotide metabolism and anemias due to unspecified enzyme disorder), thalassemia, α-thalassemia, β-thalassemia, δβ-thalassemia, thalassemia trait, hereditary persistence of fetal hemoglobin (HPFP), and other thalassemias, sickle cell disorders (sickle cell anemia with crisis, sickle cell anemia without crisis, double heterozygous sickling disorders, sickle cell trait and other sickle cell disorders), hereditary hemolytic anemias (hereditary spherocytosis, hereditary elliptocytosis, other hemoglobinopathies and other specified hereditary hemolytic anemias, such as stomatocyclosis), acquired hemolytic anemia (*e*.*g*., drug-induced autoimmune hemolytic anemia, other autoimmune hemolytic anemias, such as warm autoimmune hemolytic anemia, drug-induced non-autoimmune hemolytic anemia, hemolytic-uremic syndrome, and other non-autoimmune hemolytic anemias, such as microangiopathic hemolytic anemia); aplastic anemias (*e*.*g*., acquired pure red cell aplasia (erythoblastopenia), other aplastic anemias, such as constitutional aplastic anemia and fanconi anemia, acute posthemorrhagic anemic, and anemias in chronic diseases), coagulation defects (*e*.*g*., disseminated intravascular coagulation (difibrination syndrome)), hereditary factor VIII deficiency (hemophilia A), hereditary factor IX deficiency (Christmas disease), and other coagulation defects such as Von Willebrand's disease, hereditary factor Xi deficiency (hemophilia C), purpura (*e*.*g*., qualitative platelet defects and Glanzmann's disease), neutropenia, agranulocytosis, functional disorders of polymorphonuclear neutrophils, other disorders of white blood cells (*e*.*g*., eosinophilia, leukocytosis, lymophocytosis, lymphopenia, monocytosis, and plasmacytosis), diseases of the spleen, methemoglobinemia, other diseases of blood and blood forming organs (*e*.*g*., familial erythrocytosis, secondary polycythemia, essential thrombocytosis and basophilia), thrombocytopenia, infectious anemia, hypoproliferative or hypoplastic anemias, hemoglobin C, D and E disease, hemoglobin lepore disease, and HbH and HbS diseases, anemias due to blood loss, radiation therapy or chemotherapy, or thrombocytopenias and neutropenias due to radiation therapy or chemotherapy, sideroblastic anemias, myelophthisic anemias, antibody-mediated anemias, and certain diseases involving lymphoreticular tissue and reticulohistiocytic system (*e*.*g*., Langerhans' cell hystiocytosis, eosinophilic granuloma, Hand-Schuller-Christian disease, hemophagocytic lymphohistiocytosis, and infection-associated hemophagocytic syndrome).

Accordingly, as disclosed herein, the combinatorial composition comprising inhibitors of BCL11A expression or activity and epigenetic modifiers described herein can be administered to a subject to treat a blood disorder, where a blood disorder is any disease or malady that can be characterized as a direct or indirect consequence of a defect or disease of hemoglobin producing cells or the production of hemoglobin. The blood disorder can be associated with an anemia such as sickle cell anemia, hemolytic anemia, infectious anemia, aplastic anemias, hypoproliferative or hypoplastic anemias, sideroblastic anemias, myelophthisic anemias, antibody-mediated anemias, anemias due to enzyme-deficiencies or chronic diseases, anemias due to blood loss, radiation therapy or chemotherapy, thalassemias including α-like and β-like thalassemias, or globin disorders due to infections of viral, bacterial or parasitic origin such as malaria, trypanosomiasis, human immunodeficiency virus and other retroviruses, a polyoma virus such as JC virus, or a hepatitis virus such as human hepatitis viruses types A-G. Treatable blood disorders also include syndromes such as hemoglobin C, D and E disease, hemoglobin lepore disease, and HbH and HbS diseases. Treatment ameliorates one or more symptoms associated with the disorder. Symptoms typically associated with blood disorders include, for example, anemia, tissue hypoxia, organ dysfunction, abnormal hematocrit values, ineffective erythropoiesis, abnormal reticulocyte (erythrocyte) count, abnormal iron load, the presence of ring sideroblasts, splenomegaly, hepatomegaly, impaired peripheral blood flow, dyspnea, increased hemolysis, jaundice, anemic crises and pain such as angina pectoris.

In some embodiments, the blood deficiencies are acquired or genetic deficiencies. Genetic blood disorders are well known by persons of ordinary skill in the art, and include, without limitation, Thalassemias, Sickle cell disease, hereditary spherocytosis, G6PD Deficiency hemolytic anemia, Kostman's syndrome, Swachman-Diamond Syndrome, Cyclic neutropenia, Hereditary neutropenia, Dyskeratosis Congenita, Hereditary thrombocytopenia syndromes, Wiskott-Aldrich Syndrome, May-Hegglin anomaly, Thrombocytopenia with Absent Radii Syndrome, Fanconi's anemia and other hereditary blood disorders.

The terms "subject" and "individual" are used interchangeably herein, and refer to an animal, for example a human, recipient of the combinatorial composition comprising inhibitors of BCL11A expression or activity and one or more epigenetic modifiers described herein. For treatment of those disease states which are specific for a specific animal, such as a human subject, the term "subject" refers to that specific animal. The terms 'non-human animals' and 'non-human mammals' are used interchangeably herein, and include mammals such as rats, mice, rabbits, sheep, cats, dogs, cows, pigs, and non-human primates. A subject can be an adult subject, an adolescent subject, a child subject, a neonate subject, an infant subject, or an *in utero* subject. Administration of the combinatorial composition comprising inhibitors of BCL11A expression or activity and epigenetic modifiers described herein can be short term, continuous or sporadic as necessary. Patients with a suspected or diagnosed with a blood disorder may only require composition treatment for short periods of time or until symptoms have abated or have been effectively eliminated.

In other aspects, provided herein are methods for increasing fetal hemoglobin levels in a cell. Such methods comprise the steps of contacting a hematopoietic progenitor cell with an effective amount of a combinatorial composition comprising inhibitors of BCL11A expression or activity and epigenetic modifiers described herein, whereby fetal hemoglobin expression is increased in the cell, or its progeny, relative to the cell prior to the contacting.

Accordingly, provided here are methods for increasing HbF levels in a cell, the method comprising the steps of contacting a hematopoietic progenitor cell with an effective amount of a composition comprising at least one inhibitor of BCL11A and at least one HbF chemical inducer, whereby HbF expression is increased in the cell, or its progeny, relative to the cell prior to the contacting.

In some aspects, provided here are methods for increasing HbF levels in a cell, the method comprising the steps of contacting a hematopoietic progenitor cell with an effective amount of a composition comprising at least one inhibitor of BCL11A and at least one epigenetic modifier, whereby HbF expression is increased in the cell, or its progeny, relative to the cell prior to the contacting.

In some aspects, provided here are methods for increasing HbF levels in a cell, the method comprising the steps of contacting a hematopoietic progenitor cell with an effective amount of a composition comprising at least one inhibitor of BCL11A and at least one DNA methylation inhibitor, whereby HbF expression is increased in the cell, or its progeny, relative to the cell prior to the contacting.

In some aspects, provided here are methods for increasing HbF levels in a cell, the method comprising the steps of contacting a hematopoietic progenitor cell with an effective amount of a composition comprising at least one inhibitor of BCL11A and at least one histone deacetylation inhibitor, whereby HbF expression is increased in the cell, or its progeny, relative to the cell prior to the contacting.

In some aspects, provided here are methods for increasing HbF levels in a cell, the method comprising the steps of contacting a hematopoietic progenitor cell with an effective amount of a composition comprising at least one inhibitor of BCL11A, at least one DNA methylation inhibitor, and at least one histone deacetylation inhibitor, whereby HbF expression is increased in the cell, or its progeny, relative to the cell prior to the contacting.

In some embodiments of these methods, the haematopoietic progenitor cell has at least one of the cell surface markers characteristic of haematopoietic progenitor cells, such as, for example, CD34+, CD59+, Thy1/CD90+,CD38lo/-, and C-kit/CD117+. Preferably, the haematopoietic progenitor cells being contacted have at least two of these markers.

In some embodiments of these methods, the hematopoietic progenitor cell is a cell of the erythroid lineage.

In some embodiment of these methods, the haematopoietic progenitor cells of the erythroid lineage have the cell surface marker characteristic of the erythroid lineage, CD71 and Ter119.

In some embodiments of these methods, the hematopoietic progenitor cell is contacted *ex vivo* or *in vitro.* In some embodiments of these methods, the methods further comprising administering the contacted hematopoietic progenitor cell and/or its progeny cells thereof to a subject, such as a mammalian subject.

In connection with treating a subject or contacting a cell with a combinatorial composition comprising inhibitors of BCL11A expression or activity and one or more epigenetic modifiers described herein, "increasing the fetal hemoglobin levels" in the subject or cell indicates that fetal hemoglobin is at least 5% higher in the subject or cell populations treated with a BCL11A inhibitor, than in a comparable, control subject or cell population, wherein no combinatorial composition is present. It is preferred that the percentage of fetal hemoglobin expression in subject or cell population treated with a combinatorial composition is at least 10% higher, at least 20% higher, at least 30% higher, at least 40% higher, at least 50% higher, at least 60% higher, at least 70% higher, at least 80% higher, at least 90% higher, at least 1-fold higher, at least 2-fold higher, at least 5-fold higher, at least 10 fold higher, at least 25 fold higher, at least 50 fold higher ,at least 100 fold higher, at least 1000-fold higher, or more than a control treated cell population of comparable size and culture conditions, or the subject in the absence of the treatment. The term "control treated population" is used herein to describe a population of cells that has been treated with identical media, viral induction, nucleic acid sequences, temperature, confluency, flask size, pH, etc., with the exception of the addition of the combinatorial composition.

"Hematopoietic progenitor cell" as the term is used herein, refers to cells of a stem cell lineage that give rise to all the blood cell types including the myeloid (monocytes and macrophages, neutrophils, basophils, eosinophils, erythrocytes, megakaryocytes/platelets, dendritic cells), and the lymphoid lineages (T-cells, B-cells, NK-cells). A "cell of the erythroid lineage" indicates that the cell being contacted is a cell that undergoes erythropoeisis such that upon final differentiation it forms an erythrocyte or red blood cell (RBC). Such cells belong to one of three lineages, erythroid, lymphoid, and myeloid, originating from bone marrow haematopoietic progenitor cells. Upon exposure to specific growth factors and other components of the haematopoietic microenvironment, haematopoietic progenitor cells can mature through a series of intermediate differentiation cellular types, all intermediates of the erythroid lineage, into RBCs. Thus, cells of the "erythroid lineage," as the term is used herein, comprise hematopoietic progenitor cells, rubriblasts, prorubricytes, erythroblasts, metarubricytes, reticulocytes, and erythrocytes.

Stem cells, such as hematopoietic progenitor cells, are capable of proliferation and giving rise to more progenitor cells having the ability to generate a large number of mother cells that can in turn give rise to differentiated, or differentiable daughter cells. The daughter cells themselves can be induced to proliferate and produce progeny that subsequently differentiate into one or more mature cell types, while also retaining one or more cells with parental developmental potential. The term "stem cell" refers then, to a cell with the capacity or potential, under particular circumstances, to differentiate to a more specialized or differentiated phenotype, and which retains the capacity, under certain circumstances, to proliferate without substantially differentiating.

The term progenitor or stem cell refers to a generalized mother cell whose descendants (progeny) specialize, often in different directions, by differentiation, *e*.*g*., by acquiring completely individual characters, as occurs in progressive diversification of embryonic cells and tissues. Cellular differentiation is a complex process typically occurring through many cell divisions. A differentiated cell may derive from a multipotent cell which itself is derived from a multipotent cell, and so on. While each of these multipotent cells may be considered stem cells, the range of cell types each can give rise to may vary considerably. Some differentiated cells also have the capacity to give rise to cells of greater developmental potential. Such capacity can be natural or can be induced artificially upon treatment with various factors. In many biological instances, stem cells are also "multipotent" because they can produce progeny of more than one distinct cell type, but this is not required for "stem-ness." Self-renewal is the other classical part of the stem cell definition, and it is essential as used in this document. In theory, self-renewal can occur by either of two major mechanisms. Stem cells may divide asymmetrically, with one daughter retaining the stem state and the other daughter expressing some distinct other specific function and phenotype. Alternatively, some of the stem cells in a population can divide symmetrically into two stems, thus maintaining some stem cells in the population as a whole, while other cells in the population give rise to differentiated progeny only. Generally, "progenitor cells" have a cellular phenotype that is more primitive (*i.e.,* is at an earlier step along a developmental pathway or progression than is a fully differentiated cell). Often, progenitor cells also have significant or very high proliferative potential. Progenitor cells can give rise to multiple distinct differentiated cell types or to a single differentiated cell type, depending on the developmental pathway and on the environment in which the cells develop and differentiate.

In the context of cell ontogeny, the adjective "differentiated", or "differentiating" is a relative term. A "differentiated cell" is a cell that has progressed further down the developmental pathway than the cell it is being compared with. Thus, stem cells can differentiate to lineage-restricted precursor cells (such as a hematopoietic progenitor cell), which in turn can differentiate into other types of precursor cells further down the pathway (such as an erthyrocyte precursor), and then to an end-stage differentiated cell, such as an erthyrocyte, which plays a characteristic role in a certain tissue type, and may or may not retain the capacity to proliferate further.

### Administration, Dosages, and Durations

A combinatorial composition comprising inhibitors of BCL11A expression or activity and one or more epigenetic modifiers, such as DNA methylation inhibitors and/or HDAC inhibitors, can be formulated, dosed, and administered in a fashion consistent with good medical practice for use in the treatment of the blood disorders described herein, such as β-hemoglobinopathies. Factors for consideration in this context include the particular blood disorder or type of blood disorder being treated, the particular subject being treated, the clinical condition of the individual subject, the cause of the disorder, the site of delivery of the agent, the method of administration, the scheduling of administration, and other factors known to medical practitioners.

Accordingly, the "therapeutically effective amount" of a combinatorial composition comprising inhibitors of BCL11A expression or activity and one or more epigenetic modifiers, such as DNA methylation inhibitors and/or HDAC inhibitors, to be administered to a subject or contacted with a cell is governed by such considerations, and, as used herein, refers to the minimum amount necessary to prevent, ameliorate, or treat, or stabilize, a disorder or condition, such as a β-hemoglobinopathy .

In those aspects and embodiments relating to blood disorders such as β-hemoglobinopathies, the therapeutically effective amount of a combinatorial composition comprising inhibitors of BCL11A expression or activity and one or more epigenetic modifiers, such as DNA methylation inhibitors and/or HDAC inhibitors described herein is the minimum amount necessary to, for example, increase the amount of fetal hemoglobin in the blood, increase the number of F-cells in the blood, increase the number of F-reticulocytes in the blood of the subject, increase total hemoglobin in the blood, increase the percentage of reticulocytes in the blood, increase the number of reticulocytes in the blood, increase hematocrit, or increase red blood cell production.

An effective amount as used herein also includes an amount sufficient to delay the development of a symptom of the blood disorder, alter the course of a blood disorder, or reverse a symptom of the blood disorder. Symptoms of a blood disorder, such as a β-hemoglobinopathy, can include, for example, anemia, marrow expansion, facial deformities, splenomegaly, growth retardation, endocrinopathies, and osteopenia. Thus, it is not possible to specify the exact "effective amount". However, for any given case, an appropriate "effective amount" can be determined by one of ordinary skill in the art using only routine experimentation.

Effective amounts, toxicity, and therapeutic efficacy of the combinatorial composition comprising inhibitors of BCL11A expression or activity and one or more epigenetic modifiers, such as DNA methylation inhibitors and/or HDAC inhibitors described herein can be determined by standard pharmaceutical procedures in cell cultures or experimental animals, *e*.*g*., for determining the LD₅₀ (the dose lethal to 50% of the population) and the ED₅₀ (the dose therapeutically effective in 50% of the population). The dosage can vary depending upon the dosage form employed and the route of administration utilized. The dose ratio between toxic and therapeutic effects is the therapeutic index and can be expressed as the ratio LD₅₀/ED₅₀. Compositions and methods that exhibit large therapeutic indices are preferred. A therapeutically effective dose can be estimated initially from cell culture assays. Also, a dose can be formulated in animal models to achieve a circulating plasma concentration range that includes the IC₅₀ (*i*.*e*., the concentration of the combinatorial composition), which achieves a half-maximal inhibition of symptoms) as determined in cell culture, or in an appropriate animal model. Levels in plasma can be measured, for example, by high performance liquid chromatography. The effects of any particular dosage can be monitored by a suitable bioassay. The dosage can be determined by a physician and adjusted, as necessary, to suit observed effects of the treatment.

Depending on the type and severity of the disease, about 1 µg/kg to 100 mg/kg (*e.g.,* 0.1-20 mg/kg) of a combinatorial composition comprising inhibitors of BCL11A expression or activity and one or more epigenetic modifiers, such as DNA methylation inhibitors and/or HDAC inhibitors, described herein is an initial candidate dosage range for administration to the subject, whether, for example, by one or more separate administrations, or by continuous infusion. A typical daily dosage might range from about 1 µg/kg to about 100 mg/kg or more, depending on the factors mentioned above. For repeated administrations over several days or longer, depending on the condition, the treatment is sustained until the disorder is treated, as measured by the methods described above or known in the art. However, other dosage regimens can be useful. The progress of the therapeutic methods described herein is easily monitored by conventional techniques and assays, such as those described herein, or known to one of skill in the art. r.

The duration of the therapeutic methods described herein can continue for as long as medically indicated or until a desired therapeutic effect (*e.g.,* those described herein) is achieved. In certain embodiments, administration of a combinatorial composition comprising inhibitors of BCL11A expression or activity and one or more epigenetic modifiers, such as DNA methylation inhibitors and/or HDAC inhibitors is continued for at least 1 month, at least 2 months, at least 4 months, at least 6 months, at least 8 months, at least 10 months, at least 1 year, at least 2 years, at least 3 years, at least 4 years, at least 5 years, at least 10 years, at least 20 years, or for at least a period of years up to the lifetime of the subject.

The combinatorial compositions comprising inhibitors of BCL11A expression or activity and one or more epigenetic modifiers, such as DNA methylation inhibitors and/or HDAC inhibitors, can be administered to a subject, *e.g.,* a human subject, in accordance with known methods, such as intravenous administration as a bolus or by continuous infusion over a period of time, by intramuscular, intraperitoneal, intracerobrospinal, subcutaneous, intra-articular, intrasynovial, intrathecal, oral, topical, or inhalation routes. Local administration can be used if, for example, extensive side effects or toxicity is associated with the combinatorial composition comprising inhibitors of BCL11A expression or activity and one or more epigenetic modifiers, such as DNA methylation inhibitors and/or HDAC inhibitors. An *ex vivo* strategy can also be used for therapeutic applications, for example, contacting cells *ex vivo* and administering the contacted cells to the patient.

Exemplary modes of administration of the combinatorial compositions comprising inhibitors of BCL11A expression or activity and one or more epigenetic modifiers, such as DNA methylation inhibitors and/or HDAC inhibitors, include, but are not limited to, injection, infusion, inhalation (*e*.*g*., intranasal or intratracheal), ingestion, rectal, and topical (including buccal and sublingual) administration. The phrases "parenteral administration" and "administered parenterally" as used herein, refer to modes of administration other than enteral and topical administration, usually by injection. As used herein, "injection" includes, without limitation, intravenous, intramuscular, intraarterial, intrathecal, intraventricular, intracapsular, intraorbital, intracardiac, intradermal, intraperitoneal, transtracheal, subcutaneous, subcuticular, intraarticular, sub capsular, subarachnoid, intraspinal, intracerebro spinal, and intrasternal injection and infusion. The phrases "systemic administration," "administered systemically", "peripheral administration" and "administered peripherally" as used herein refer to the administration of a combinatorial composition comprising inhibitors of BCL11A expression or activity and one or more epigenetic modifiers, such as DNA methylation inhibitors and/or HDAC inhibitors, other than directly into a target site, tissue, or organ, such as the lung, such that it enters the subject's circulatory system and, thus, is subject to metabolism and other like processes.

In some embodiments, the combinatorial compositions comprising inhibitors of BCL11A expression or activity and one or more epigenetic modifiers, such as DNA methylation inhibitors and/or HDAC inhibitors are administered by intravenous infusion or injection. Additionally, in some embodiments, the combinatorial compositions comprising inhibitors of BCL11A expression or activity and one or more epigenetic modifiers, such as DNA methylation inhibitors and/or HDAC inhibitors described herein can be administered by pulse infusion, particularly with declining doses of the compositions. Preferably the dosing is given by injections, most preferably intravenous or subcutaneous injections, depending in part on whether the administration is brief or chronic.

In some embodiments, the combinatorial composition comprising inhibitors of BCL11A expression or activity and one or more epigenetic modifiers, such as DNA methylation inhibitors and/or HDAC inhibitors is administered locally, *e.g.,* by direct injections, and the injections can be repeated periodically. The combinatorial compositions comprising inhibitors of BCL11A expression or activity and one or more epigenetic modifiers, such as DNA methylation inhibitors and/or HDAC inhibitors described herein can also be delivered systemically to the subject.

### Pharmaceutical Formulations

Therapeutic formulations can be prepared, in some aspects, by mixing the combinatorial composition comprising one or more inhibitors of BCL11A expression or activity and one or more epigenetic modifiers, such as DNA methylation inhibitors and/or HDAC inhibitors having the desired degree of purity with one or more pharmaceutically acceptable carriers, excipients or stabilizers (Remington's Pharmaceutical Sciences 16th edition, Osol, A. Ed. (1980)), in the form of lyophilized formulations or aqueous solutions. Such therapeutic formulations of the combinatorial compositions described herein include formulation into pharmaceutical compositions or pharmaceutical formulations for parenteral administration, *e.g.,* intravenous; mucosal, *e.g.,* intranasal; enteral, *e.g.,* oral; topical, *e*.*g*., transdermal; ocular, or other mode of administration.

As used herein, the phrase "pharmaceutically acceptable" refers to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio. The phrase "pharmaceutically acceptable carrier" as used herein means a pharmaceutically acceptable material, composition or vehicle, such as a liquid or solid filler, diluent, excipient, solvent, media, encapsulating material, manufacturing aid (*e*.*g*., lubricant, talc magnesium, calcium or zinc stearate, or steric acid), or solvent encapsulating material, involved in maintaining the activity of, carrying, or transporting the combinatorial composition comprising inhibitors of BCL11A expression or activity and one or more epigenetic modifiers, such as DNA methylation inhibitors and/or HDAC inhibitors from one organ, or portion of the body, to another organ, or portion of the body.

Some non-limiting examples of acceptable carriers, excipients, or stabilizers that are nontoxic to recipients at the dosages and concentrations employed, include pH buffered solutions such as phosphate, citrate, and other organic acids; antioxidants, including ascorbic acid and methionine; lubricating agents, such as magnesium stearate, sodium lauryl sulfate and talc; excipients, such as cocoa butter and suppository waxes; oils, such as peanut oil, cottonseed oil, safflower oil, sesame oil, olive oil, corn oil and soybean oil; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, HDL, LDL, or immunoglobulins; hydrophilic polymers, such as polyvinylpyrrolidone; amino acids such as glycine, glutamine, asparagine, histidine, arginine, or lysine; monosaccharides, disaccharides, and other carbohydrates including mannose, starches (corn starch or potato starch), or dextrins; cellulose, and its derivatives, such as sodium carboxymethyl cellulose, methylcellulose, ethyl cellulose, microcrystalline cellulose and cellulose acetate; chelating agents such as EDTA; sugars such as sucrose, glucose, lactose, mannitol, trehalose or sorbitol; salt-forming counter-ions such as sodium; metal complexes (*e*.*g*. Zn-protein complexes); glycols, such as propylene glycol; polyols, such as glycerin; esters, such as ethyl oleate and ethyl laurate; agar; buffering agents, such as magnesium hydroxide and aluminum hydroxide; alginic acid; pyrogen-free water; isotonic saline; Ringer's solution; polyesters, polycarbonates and/or polyanhydrides; C2-C12 alcohols, such as ethanol; powdered tragacanth; malt; and/or non-ionic surfactants such as TWEEN™, PLURONICS™ or polyethylene glycol (PEG); and/or other non-toxic compatible substances employed in pharmaceutical formulations. Wetting agents, coloring agents, release agents, coating agents, sweetening agents, flavoring agents, perfuming agents, preservative and antioxidants can also be present in the formulation.

In some embodiments, the therapeutic formulation comprising the combinatorial composition comprising inhibitors of BCL11A expression or activity and one or more epigenetic modifiers, such as DNA methylation inhibitors and/or HDAC inhibitors comprises a pharmaceutically acceptable salt, typically, *e*.*g*., sodium chloride, and preferably at about physiological concentrations. Optionally, the formulations described herein can contain a pharmaceutically acceptable preservative. In some embodiments, the preservative concentration ranges from 0.1 to 2.0%, typically v/v. Suitable preservatives include those known in the pharmaceutical arts. Benzyl alcohol, phenol, m-cresol, methylparaben, and propylparaben are examples of preservatives. Optionally, the formulations of the invention can include a pharmaceutically acceptable surfactant at a concentration of 0.005 to 0.02%.

In some embodiments of the aspects described herein, the combinatorial compositions comprising inhibitors of BCL11A expression or activity and one or more epigenetic modifiers, such as DNA methylation inhibitors and/or HDAC inhibitors, can be specially formulated for administration of the compositions to a subject in solid, liquid or gel form, including those adapted for the following: (1) oral administration, for example, drenches (aqueous or non-aqueous solutions or suspensions), lozenges, dragees, capsules, pills, tablets (*e*.*g*., those targeted for buccal, sublingual, and systemic absorption), boluses, powders, granules, pastes for application to the tongue; (2) parenteral administration, for example, by subcutaneous, intramuscular, intravenous or epidural injection as, for example, a sterile solution or suspension, or sustained-release formulation; (3) topical application, for example, as a cream, ointment, or a controlled-release patch or spray applied to the skin; (4) intravaginally or intrarectally, for example, as a pessary, cream or foam; (5) sublingually; (6) ocularly; (7) transdermally; (8) transmucosally; or (9) nasally. Additionally, the combinatorial compositions comprising inhibitors of BCL11A expression or activity and one or more epigenetic modifiers, such as DNA methylation inhibitors and/or HDAC inhibitors can be implanted into a patient or injected using a drug delivery system. See, for example, Urquhart, et al., Ann. Rev. Pharmacol. Toxicol. 24: 199-236 (1984); Lewis, ed. "Controlled Release of Pesticides and Pharmaceuticals" (Plenum Press, New York, 1981); U.S. Pat. No. 3,773,919; and U.S. Pat. No. 35 3,270,960. Examples of dosage forms include, but are not limited to: tablets; caplets; capsules, such as hard gelatin capsules and soft elastic gelatin capsules; cachets; troches; lozenges; dispersions; suppositories; ointments; cataplasms (poultices); pastes; powders; dressings; creams; plasters; solutions; patches; aerosols (*e.g.,* nasal sprays or inhalers); gels; liquids such as suspensions (*e.g.,* aqueous or non-aqueous liquid suspensions, oil-in-water emulsions, or water-in-oil liquid emulsions), solutions, and elixirs; and sterile solids (*e*.*g*., crystalline or amorphous solids) that can be reconstituted to provide liquid dosage forms.

In some embodiments, parenteral dosage forms combinatorial compositions comprising inhibitors of BCL11A expression or activity and one or more epigenetic modifiers, such as DNA methylation inhibitors and/or HDAC inhibitors, can be administered to a subject with a blood disorder or in need of increased fetal hemoglobin by various routes, including, but not limited to, subcutaneous, intravenous (including bolus injection), intramuscular, and intraarterial. Since administration of parenteral dosage forms typically bypasses the patient's natural defenses against contaminants, parenteral dosage forms are preferably sterile or capable of being sterilized prior to administration to a patient. Examples of parenteral dosage forms include, but are not limited to, solutions ready for injection, dry products ready to be dissolved or suspended in a pharmaceutically acceptable vehicle for injection, suspensions ready for injection, controlled-release parenteral dosage forms, and emulsions.

Suitable vehicles that can be used to provide parenteral dosage forms described herein are well known to those skilled in the art. Examples include, without limitation: sterile water; water for injection USP; saline solution; glucose solution; aqueous vehicles such as but not limited to, sodium chloride injection, Ringer's injection, dextrose Injection, dextrose and sodium chloride injection, and lactated Ringer's injection; water-miscible vehicles such as, but not limited to, ethyl alcohol, polyethylene glycol, and propylene glycol; and non-aqueous vehicles such as, but not limited to, corn oil, cottonseed oil, peanut oil, sesame oil, ethyl oleate, isopropyl myristate, and benzyl benzoate.

In some embodiments, the combinatorial compositions comprising inhibitors of BCL11A expression or activity and one or more epigenetic modifiers, such as DNA methylation inhibitors and/or HDAC inhibitors, are formulated to be suitable for oral administration, for example as discrete dosage forms, such as, but not limited to, tablets (including without limitation scored or coated tablets), pills, caplets, capsules, chewable tablets, powder packets, cachets, troches, wafers, aerosol sprays, or liquids, such as but not limited to, syrups, elixirs, solutions or suspensions in an aqueous liquid, a non-aqueous liquid, an oil-in-water emulsion, or a water-in-oil emulsion. Such compositions contain a predetermined amount of the pharmaceutically acceptable salt of the disclosed compounds, and may be prepared by methods of pharmacy well known to those skilled in the art. See generally, Remington's Pharmaceutical Sciences, 18th ed., Mack Publishing, Easton, Pa. (1990).

Due to their ease of administration, tablets and capsules represent the most advantageous solid oral dosage unit forms, in which case solid pharmaceutical excipients are used. If desired, tablets can be coated by standard aqueous or nonaqueous techniques. These dosage forms can be prepared by any of the methods of pharmacy. In general, pharmaceutical compositions and dosage forms are prepared by uniformly and intimately admixing the active ingredient(s) with liquid carriers, finely divided solid carriers, or both, and then shaping the product into the desired presentation if necessary.

Typical oral dosage forms of the compositions are prepared by combining the pharmaceutically acceptable salt of an inhibitor of BCL11A expression or activity and one or more epigenetic modifiers, such as DNA methylation inhibitors and/or HDAC inhibitors, in an intimate admixture with at least one excipient according to conventional pharmaceutical compounding techniques. Excipients can take a wide variety of forms depending on the form of the composition desired for administration. For example, excipients suitable for use in oral liquid or aerosol dosage forms include, but are not limited to, water, glycols, oils, alcohols, flavoring agents, preservatives, and coloring agents. Examples of excipients suitable for use in solid oral dosage forms (*e.g.,* powders, tablets, capsules, and caplets) include, but are not limited to, starches, sugars, microcrystalline cellulose, kaolin, diluents, granulating agents, lubricants, binders, and disintegrating agents.

Binders suitable for use in the pharmaceutical formulations described herein include, but are not limited to, corn starch, potato starch, or other starches, gelatin, natural and synthetic gums such as acacia, sodium alginate, alginic acid, other alginates, powdered tragacanth, guar gum, cellulose and its derivatives (*e*.*g*., ethyl cellulose, cellulose acetate, carboxymethyl cellulose calcium, sodium carboxymethyl cellulose), polyvinyl pyrrolidone, methyl cellulose, pre-gelatinized starch, hydroxypropyl methyl cellulose, (*e.g.,* Nos. 2208, 2906, 2910), microcrystalline cellulose, and mixtures thereof.

Examples of fillers suitable for use in the pharmaceutical formulations described herein include, but are not limited to, talc, calcium carbonate (*e*.*g*., granules or powder), microcrystalline cellulose, powdered cellulose, dextrates, kaolin, mannitol, silicic acid, sorbitol, starch, pre-gelatinized starch, and mixtures thereof. The binder or filler in pharmaceutical compositions described herein is typically present in from about 50 to about 99 weight percent of the pharmaceutical composition.

Disintegrants are used in the oral pharmaceutical formulations described herein to provide tablets that disintegrate when exposed to an aqueous environment. A sufficient amount of disintegrant that is neither too little nor too much to detrimentally alter the release of the active ingredient(s) should be used to form combinatorial compositions comprising inhibitors of BCL11A expression or activity and one or more epigenetic modifiers, such as DNA methylation inhibitors and/or HDAC inhibitors. The amount of disintegrant used varies based upon the type of formulation, and is readily discernible to those of ordinary skill in the art. Disintegrants that can be used to form oral pharmaceutical formulations include, but are not limited to, agar, alginic acid, calcium carbonate, microcrystalline cellulose, croscarmellose sodium, crospovidone, polacrilin potassium, sodium starch glycolate, potato or tapioca starch, other starches, pre-gelatinized starch, clays, other algins, other celluloses, gums, and mixtures thereof.

Lubricants that can be used to form oral pharmaceutical formulations of the AhR inhibitors described herein, include, but are not limited to, calcium stearate, magnesium stearate, mineral oil, light mineral oil, glycerin, sorbitol, mannitol, polyethylene glycol, other glycols, stearic acid, sodium lauryl sulfate, talc, hydrogenated vegetable oil (*e.g.,* peanut oil, cottonseed oil, sunflower oil, sesame oil, olive oil, corn oil, and soybean oil), zinc stearate, ethyl oleate, ethyl laureate, agar, and mixtures thereof. Additional lubricants include, for example, a syloid silica gel (AEROSIL® 200, manufactured by W. R. Grace Co. of Baltimore, Md.), a coagulated aerosol of synthetic silica (marketed by Degussa Co. of Piano, Tex.), CAB-O-SIL® (a pyrogenic silicon dioxide product sold by Cabot Co. of Boston, Mass.), and mixtures thereof. If used at all, lubricants are typically used in an amount of less than about 1 weight percent of the pharmaceutical compositions or dosage forms into which they are incorporated.

In other embodiments, lactose-free pharmaceutical formulations and dosage forms are provided , wherein such compositions preferably contain little, if any, lactose or other mono- or disaccharides. As used herein, the term "lactose- free" means that the amount of lactose present, if any, is insufficient to substantially increase the degradation rate of an active ingredient. Lactose-free compositions of the disclosure can comprise excipients which are well known in the art and are listed in the USP (XXI)/NF (XVI).

The oral formulations of the combinatorial compositions comprising inhibitors of BCL11A expression or activity and one or more epigenetic modifiers, such as DNA methylation inhibitors and/or HDAC inhibitors, further encompass, in some embodiments, anhydrous pharmaceutical compositions and dosage forms comprising the inhibitors of BCL11A and one or more epigenetic modifiers described herein as active ingredients, since water can facilitate the degradation of some compounds. For example, the addition of water (*e*.*g*., 5%) is widely accepted in the pharmaceutical arts as a means of simulating long-term storage in order to determine characteristics such as shelf life or the stability of formulations over time. See, *e*.*g*., Jens T. Carstensen, Drug Stability: Principles & Practice, 379-80 (2nd ed., Marcel Dekker, NY, N.Y.: 1995). Anhydrous pharmaceutical compositions and dosage forms described herein can be prepared using anhydrous or low moisture containing ingredients and low moisture or low humidity conditions. Pharmaceutical compositions and dosage forms that comprise lactose and at least one active ingredient that comprises a primary or secondary amine are preferably anhydrous if substantial contact with moisture and/or humidity during manufacturing, packaging, and/or storage is expected. Anhydrous compositions are preferably packaged using materials known to prevent exposure to water such that they can be included in suitable formulary kits. Examples of suitable packaging include, but are not limited to, hermetically sealed foils, plastics, unit dose containers (e.g., vials) with or without desiccants, blister packs, and strip packs.

In some embodiments, the active ingredients of the formulations comprising combinatorial compositions comprising inhibitors of BCL11A expression or activity and one or more epigenetic modifiers, such as DNA methylation inhibitors and/or HDAC inhibitors described herein can also be entrapped in microcapsules prepared, for example, by coacervation techniques or by interfacial polymerization, for example, hydroxymethylcellulose or gelatin-microcapsules and poly-(methylmethacylate) microcapsules, respectively, in colloidal drug delivery systems (for example, liposomes, albumin microspheres, microemulsions, nano-particles and nanocapsules) or in macroemulsions. Such techniques are disclosed in Remington's Pharmaceutical Sciences 16th edition, Osol, A. Ed. (1980).

In some embodiments of these aspects, the combinatorial compositions comprising inhibitors of BCL11A expression or activity and one or more epigenetic modifiers, such as DNA methylation inhibitors and/or HDAC inhibitors can be administered to a subject by controlled- or delayed-release means. Ideally, the use of an optimally designed controlled-release preparation in medical treatment is characterized by a minimum of drug substance being employed to cure or control the condition in a minimum amount of time. Advantages of controlled-release formulations include: 1) extended activity of the drug; 2) reduced dosage frequency; 3) increased patient compliance; 4) usage of less total drug; 5) reduction in local or systemic side effects; 6) minimization of drug accumulation; 7) reduction in blood level fluctuations; 8) improvement in efficacy of treatment; 9) reduction of potentiation or loss of drug activity; and 10) improvement in speed of control of diseases or conditions. (Kim, Cherng-ju, Controlled Release Dosage Form Design, 2 (Technomic Publishing, Lancaster, Pa.: 2000)). Controlled-release formulations can be used to control, for example, a BCL11A inhibitor's onset of action, duration of action, plasma levels within the therapeutic window, and peak blood levels. In particular, controlled- or extended-release dosage forms or formulations can be used to ensure that the maximum effectiveness of the combinatorial compositions comprising inhibitors of BCL11A expression or activity and one or more epigenetic modifiers, such as DNA methylation inhibitors and/or HDAC inhibitors, described herein is achieved while minimizing potential adverse effects and safety concerns, which can occur both from under-dosing a drug (*i*.*e*., going below the minimum therapeutic levels) as well as exceeding the toxicity level for the drug.

A variety of known controlled- or extended-release dosage forms, formulations, and devices can be adapted for use with the combinatorial compositions comprising inhibitors of BCL11A expression or activity and one or more epigenetic modifiers, such as DNA methylation inhibitors and/or HDAC inhibitors. Examples include, but are not limited to, those described in U.S. Pat. Nos.: 3,845,770; 3,916,899; 3,536,809; 3,598,123; 4,008,719; 5674,533; 5,059,595; 5,591 ,767; 5,120,548; 5,073,543; 5,639,476; 5,354,556; 5,733,566; and 6,365,185 B1. These dosage forms can be used to provide slow or controlled-release of one or more active ingredients using, for example, hydroxypropylmethyl cellulose, other polymer matrices, gels, permeable membranes, osmotic systems (such as OROS® (Alza Corporation, Mountain View, Calif. USA)), multilayer coatings, microparticles, liposomes, or microspheres or a combination thereof to provide the desired release profile in varying proportions. Additionally, ion exchange materials can be used to prepare immobilized, adsorbed salt forms of the disclosed compounds and thus effect controlled delivery of the drug. Examples of specific anion exchangers include, but are not limited to, Duolite® A568 and Duolite® AP143 (Rohm&Haas, Spring House, Pa. USA).

In some embodiments of the aspects, the combinatorial compositions comprising inhibitors of BCL11A expression or activity and one or more epigenetic modifiers, such as DNA methylation inhibitors and/or HDAC inhibitors, for use in the various therapeutic formulations and compositions, and methods thereof, described herein, is administered to a subject by sustained release or in pulses. Pulse therapy is not a form of discontinuous administration of the same amount of a composition over time, but comprises administration of the same dose of the composition at a reduced frequency or administration of reduced doses. Sustained release or pulse administrations are particularly preferred in chronic conditions, or when an agent or compound is known to have toxic or adverse side-effesct, as each pulse dose can be reduced and the total amount of a compound, *e*.*g*., a DNA methylation inhibitor, administered over the course of treatment to the patient is minimized.

The interval between pulses, when necessary, can be determined by one of ordinary skill in the art. Often, the interval between pulses can be calculated by administering another dose of the composition when the composition or the active component of the composition is no longer detectable in the subject prior to delivery of the next pulse. Intervals can also be calculated from the *in vivo* half-life of the composition. Intervals may be calculated as greater than the *in vivo* half-life, or 2, 3, 4, 5 and even 10 times greater the composition half-life. Various methods and apparatus for pulsing compositions by infusion or other forms of delivery to the patient are disclosed in U.S. Pat. Nos. 4,747,825; 4,723,958; 4,948,592; 4,965,251 and 5,403,590.

Individual pulses of a combinatorial composition comprising inhibitors of BCL11A expression or activity and one or more epigenetic modifiers, such as DNA methylation inhibitors and/or HDAC inhibitors, described herein can be delivered to the subject continuously over a period of several hours, such as about 2, 4, 6, 8, 10, 12, 14 or 16 hours, or several days, such as 2, 3, 4, 5, 6, or 7 days, preferably from about 1 hour to about 24 hours and more preferably from about 3 hours to about 9 hours. Alternatively, periodic doses can be administered in a single bolus or a small number of injections of the composition over a short period of time, typically less than 1 or 2 hours. For example, arginine butyrate has been administered over a period of 4 days with infusions for about 8 hours per day or overnight, followed by a period of 7 days of no treatment. This has been shown to be an effective regimen for thalassemic disorders, as fetal hemoglobin levels rise substantially and there is a significant rise in the number of both adult and fetal hemoglobin expressing cells. In certain instances, a substantial rise in HbF further indicates that there are positive consequences that raise the subject's quality of living such as, for example, increased activity or mobility, fewer side-effects, fewer hospital stays or visits to the physician, or fewer transfusions. For instance, HbF levels above 20% are generally considered to be sufficient to eliminate symptoms associated with a sickle cell disease.

The interval between pulses or the interval of no delivery is greater than 24 hours and preferably greater than 48 hours, and can be for even longer such as for 3, 4, 5, 6, 7, 8, 9 or 10 days, two, three or four weeks or even longer. Often, the interval between pulses can be calculated by administering another dose of the composition when the composition or the active component of the composition is no longer detectable in the patient prior to delivery of the next pulse. Intervals can also be calculated from the *in vivo* half-life of the composition. Intervals may be calculated as greater than the in vivo half-life, or 2, 3, 4, 5 and even 10 times greater the composition half-life. For compositions with fairly rapid half lives, intervals may be 25, 50, 100, 150, 200, 250 300 and even 500 times the half life of the chemical composition. The number of pulses in a single therapeutic regimen can be as little as two, but is typically from about 5 to 10, 10 to 20, 15 to 30 or more.

In some embodiments, sustained-release preparations comprising the small molecule combinatorial composition comprising inhibitors of BCL11A expression or activity and one or more epigenetic modifiers, such as DNA methylation inhibitors and/or HDAC inhibitors, described herein can be prepared. Suitable examples of sustained-release preparations include semipermeable matrices of solid hydrophobic polymers containing the inhibitor, in which matrices are in the form of shaped articles, *e*.*g*., films, or microcapsule. Examples of sustained-release matrices include polyesters, hydrogels (for example, poly(2-hydroxyethyl-methacrylate), or poly(vinylalcohol)), polylactides (U.S. Pat. No. 3,773,919), copolymers of L-glutamic acid and y ethyl-L-glutamate, non-degradable ethylene-vinyl acetate, degradable lactic acid-glycolic acid copolymers such as the LUPRON DEPOT.TM. (injectable microspheres composed of lactic acid-glycolic acid copolymer and leuprolide acetate), and poly-D-(-)-3-hydroxybutyric acid.

The formulations comprising the combinatorial composition comprising inhibitors of BCL11A expression or activity and one or more epigenetic modifiers, such as DNA methylation inhibitors and/or HDAC inhibitors, described herein to be used for *in vivo* administration are preferably sterile. This is readily accomplished by filtration through, for example, sterile filtration membranes, and other methods known to one of skill in the art.

### Efficacy of the Treatment

One key advantage of the methods, uses and compositions comprising inhibitors of BCL11A expression or activity and one or more epigenetic modifiers, such as DNA methylation inhibitors and/or HDAC inhibitors, described herein is the ability of producing marked increases in fetal hemoglobin in a human subject without causing significant toxicities or adverse effects, as occurs when an epigenetic modifier is used alone, due to the synergistic effects on fetal hemoglobin production achieved using both a BCL11A inhibitor and an epigenetic modifier, as discovered by the inventors. he efficacy of the treatments described herein can be measured by various parameters and symptoms commonly used in evaluating treatments of blood disorders, including, but not limited to, reduction or improvement of symptoms such as anemia, tissue hypoxia, organ dysfunction, abnormal hematocrit values, ineffective erythropoiesis, abnormal reticulocyte count, abnormal iron load, splenomegaly, hepatomegaly, impaired peripheral blood flow, dyspnea, increased hemolysis, jaundice, anemic crises and pain, such as angina pectoris, overall response rate, duration of response, and quality of life.Relief and even partial relief from one or more of these symptoms corresponds to an increased life span or simply an increased quality of life. Further, treatments that alleviate a pathological symptom can allow for other treatments to be administered., For example, fetal hemoglobin levels above 20% are generally considered to be sufficient to eliminate symptoms associated with sickle cell disease.

As used herein, the terms "treat," "treatment," "treating," or "amelioration" refer to therapeutic treatments, wherein the object is to reverse, alleviate, ameliorate, inhibit, slow down or stop the progression or severity of a condition associated with, a disease or disorder. The term "treating" includes reducing or alleviating at least one adverse effect or symptom of a condition, disease or disorder associated with a chronic immune condition, such as, but not limited to, a chronic infection or a cancer. Treatment is generally "effective" if one or more symptoms or clinical markers are reduced. Alternatively, treatment is "effective" if the progression of a disease is reduced or halted. That is, "treatment" includes not just the improvement of symptoms or markers, but also a cessation of at least slowing of progress or worsening of symptoms that would be expected in absence of treatment. Beneficial or desired clinical results include, but are not limited to, alleviation of one or more symptom(s), diminishment of extent of disease, stabilized (*i*.*e*., not worsening) state of disease, delay or slowing of disease progression, amelioration or palliation of the disease state, and remission (whether partial or total), whether detectable or undetectable. The term "treatment" of a disease also includes providing relief from the symptoms or side-effects of the disease (including palliative treatment).

For example, in some embodiments, the methods described herein comprise administering an effective amount of a combinatorial composition comprising inhibitors of BCL11A expression or activity and one or more epigenetic modifiers, such as DNA methylation inhibitors and/or HDAC inhibitors, to a subject in order to alleviate a symptom of a blood disorder. As used herein, "alleviating a symptom of a blood disorder" is ameliorating or reducing any condition or symptom associated with the blood disorder. As compared with an equivalent untreated control subject, or the subject prior to such administration, such reduction or degree of prevention is at least 5%, 10%, 20%, 40%, 50%, 60%, 80%, 90%, 95%, or 100% as measured by any standard technique. A patient who is being treated for a blood disorder is one who a medical practitioner has diagnosed as having such a condition. Diagnosis can be by any suitable means. Diagnosis and monitoring can involve, for example, detecting the level of fetal hemoglobin or total hemoglobin in a biological sample (for example, a blood sample), detecting the level of red blood cells in a biological sample, or detecting symptoms associated with the specific blood sample.

Prophylactic treatments can involve administration of a combinatorial composition comprising inhibitors of BCL11A expression or activity and one or more epigenetic modifiers, such as DNA methylation inhibitors and/or HDAC inhibitors, to a subject having a confirmed or suspected blood disorder without having any overt symptoms. For example, otherwise healthy patients who have been genetically screened and determined to be at high risk for the future development of a blood disorder can be administered compositions of the invention prophylactically. Administration can begin at birth and continue, if necessary, for life.

The present invention is defined in the claims. Embodiments of the disclosure are defined in any of the following numbered paragraphs:
[1] A pharmaceutical composition for increasing fetal hemoglobin levels in a mammal in need thereof, the composition comprising at least one inhibitor of BCL11A and at least one epigenetic modifier in a pharmaceutically acceptable carrier.
[2] The composition of paragraph 1, wherein the inhibitor of BCL11A inhibits BCL11A expression and/or inhibits BCL11A activity.
[3] The composition of paragraphs 1 or 2, wherein the inhibitor of BCL11A expression is an antibody specific for BCL11A or an antigen-binding fragment thereof, a small molecule, or a BCL11A specific nucleic acid.
[4] The composition of paragraph 3, wherein the nucleic acid is a BCL11A specific RNA interference agent, a vector encoding a BCL11A specific RNA interference agent, or an aptamer that binds BCL11A.
[5] The composition of paragraph 4, wherein said RNA interference agent comprises one or more of the nucleotide sequences selected from the group consisting of SEQ ID NO: 3-8.
[6] The composition of any one of paragraphs 1-5, wherein the at least one epigenetic modifier is a DNA methylation inhibitor.
[7] The composition of paragraph 6, wherein the DNA methylation inhibitor is 5-aza-2'-deoxycytidine.
[8] The composition of any one of paragraphs 1-7, wherein the epigenetic modifier is an HDAC inhibitor.
[9] The composition of paragraph 8, wherein the HDAC inhibitor is suberoylanilide hydroxamic acid (SAHA or Vorinostat).
[10] A method for increasing fetal hemoglobin levels in a mammal in need thereof, the method comprising administering to the mammal a therapeutically effective amount of a composition of any one of paragraphs 1-9, whereby fetal hemoglobin expression is increased in said mammal, relative to fetal hemoglobin expression prior to said administration.
[11] The method of paragraph 10, wherein said mammal has been diagnosed with or is at risk for a hemoglobinopathy.
[12] The method of paragraph 11, wherein said hemoglobinopathy is a β-hemoglobinopathy.
[13] The method of paragraph 11, wherein the hemoglobinopathy is sickle cell disease.
[14] The method of paragraph 11, wherein the hemoglobinopathy is β-thalassemia.
[15] The method of any one of paragraphs 10-14, wherein a hematopoietic progenitor cell is contacted with a composition of any one of paragraphs 1-9 ex vivo or in vitro, and said cell or its progeny is administered to said mammal.
[16] The method of any one of paragraphs 10-15, wherein the composition is administered by injection, infusion, instillation, or ingestion.
[17] A method for increasing fetal hemoglobin levels in a cell, the method comprising the steps of contacting a hematopoietic progenitor cell with an effective amount of a composition of any one of paragraphs 1-9, whereby fetal hemoglobin expression is increased in said cell, or its progeny, relative to fetal hemoglobin expression in said cell prior to said contacting.
[18] The method of paragraph 17, wherein the hematopoietic progenitor cell is a cell of the erythroid lineage.
**[19]** The method of paragraphs 16 or 17, wherein the hematopoietic progenitor cell is contacted ex vivo or in vitro.
[20] A method of treatment of a blood disorder in a mammal in need thereof, the method comprising administering to the mammal a therapeutically effective amount of a composition of any one of paragraphs 1-9, whereby fetal hemoglobin expression is increased in said mammal, relative to fetal hemoglobin expression prior to said administration.
[21] The method of paragraph 20, wherein the blood disorder is a hemoglobinopathy.
[22] The method of paragraph 21, wherein said hemoglobinopathy is a β-hemoglobinopathy.
[23] The method of paragraph 21, wherein the hemoglobinopathy is sickle cell disease.
[24] The method of paragraph 21, wherein the hemoglobinopathy is β-thalassemia.
[25] The method of any one of paragraphs 20-24, further comprising administering an additional therapeutic agent for the blood disorder.

This invention is further illustrated by the following example which should not be construed as limiting.

### EXAMPLES

### Introduction

The switch from fetal (HbF, α2γ2) to adult hemoglobin (HbA, α2β2), a paradigm for transcriptional control in development, is critical to the pathogenesis of sickle cell disease (SCD) and the β-thalassemias. As increased HbF lessens the severity of these conditions (1, 2), elucidation of mechanisms to relieve HbF silencing in adult erythroid cells has been a long-sought goal. As demonstrated herein, inactivation of one component involved in HbF regulation, the transcription factor BCL11A, provided phenotypic correction of SCD in a mouse model. These findings provide a crucial proof for therapeutic and targeted reactivation of HbF.

Sickle cell disease (SCD), the first "molecular disease", is caused by substitution of valine for β-6 glutamic acid in the β-globin chain of adult hemoglobin (3). The mutated, assembled hemoglobin, HbS (α2βS2), undergoes polymerization upon deoxygenation, resulting in erythrocyte deformation, hemolysis, and morbid complications secondary to microvascular occlusion. HbS polymerization is highly sensitive to inhibition by HbF (4).

The level of HbF varies among adult individuals and is inherited as a quantitative trait. Genome-wide association studies (GWAS) recently provided critical insight into loci controlling HbF. Three loci harboring genetic variants with large effects were identified, including the β-globin cluster itself, a HBS1L-MYB gene interval, and the gene encoding BCL11A (5-7). Functional studies demonstrate that BCL11A serves as transcriptional repressor of HbF expression (8-10). BCL11A controls the developmental switch from embryonic to adult β-globin in the mouse and the silencing of HbF expressed from human β-globin locus transgenes in mouse fetal liver. Moreover, BCL11A contributes to HbF silencing in cultured, primary human erythroid cells (8, 11). While providing compelling support for BCL11A as a regulator of globin switching and HbF silencing in development, these findings do not address its *in vivo* role at the adult stage and its use as a therapeutic target for reactivation of HbF. Besides the GWAS-identified regions, numerous nuclear factors have been implicated in globin switching and/or HbF silencing (12). Apart from their proposed roles in globin regulation, many of these are essential for proper maturation of erythroid cells, thereby complicating their consideration as targets for therapeutic manipulation.

### Materials and Methods

### Experimental Animals

The wild-type β-globin locus transgenic (β-YAC) mouse strain was kindly provided by K. Peterson and was created with the insertion of a 213 kb YAC containing the entire human β-globin locus (30). This β-YAC line contains three intact copies of the human β-globin locus integrated at a single genomic locus. The A20 β-YAC line harboring a single copy of a 150 kb β-globin locus YAC was kindly provided by K. Gaensler and M. Groudine (31). These transgenes were maintained in the hemizygous state. C57BL/6 mice containing a *Bcl11a* floxed allele (with loxP sites flanking exon 1) were created through gene targeting approaches (G.C.I. et al, in preparation). To obtain the *Bcl11a* conditional knockout mice, the *Bcl11a*^{fl/fl} mice were crossed with various Cre strains, including EpoR-GFP Cre knock-in mice (13), Vav-Cre (32) and Mx1- Cre (33) transgenic mice. The humanized mouse model of sickle cell anemia that expresses exclusively human sickle hemoglobin (HbS) was obtained from The Jackson Laboratory and has been described and characterized previously (19). These mice contain the human sickle transgene [Tg(Hu-miniLCRα1^{G}γ^{A}γδβ^{S})Hba⁰//Hba⁰Hbb⁰//Hbb⁰] and knockout of both the murine α- and β-globin genes (hereafter called SCD mice). The human sickle transgene were created by three DNA fragments consisting of a 6.5-kb mini-LCR, a 1.5-kb *Pst* I fragment containing the human α1-globin gene, and a 39-kb *Kpn* I fragment containing the human ^{G}γ, ^{A}γ, δ, and β^{S} globin genes in their normal genomic context (19). No significant difference in globin gene expression and SCD phenotypes were observed between mice that are knockout of one copy or both copies of murine α-globin genes. To generate BCL11A knockout SCD mice, the inventors introduced the EpoR-Cre and *Bcl11a*^{fl/fl} alleles into the SCD transgenic knockout mice (hereafter called *SCD*/*Bcl11a*^{*-*/*-*} mice). Mice containing both the human sickle transgene and at least one copy of murine β-globin gene were used as controls. PolyI:ployC (pIpC, SIGMA®) was prepared at 2 mg/ml in PBS and administrated via intraperitoneal (i.p.) injection daily at a dose of 25 µg/kg for 7 days. 5-aza-2'-deoxycytidine (5-azaD, SIGMA®) was prepared at 1.5 mg/ml in PBS and administrated daily at a dose of 5 mg/kg for 5 days via i.p. injection as previously described (34). Suberoylanilide hydroxamic acid (SAHA or Vorinostat, Cayman Chemical Company) was prepared in DMSO and administrated by i.p. injection daily at the dose of 25 mg/kg in 0.1 ml volume (diluted in 45% PEG400 in saline solution) for up to three weeks as previously described (35-37). Genotyping primers are listed in Table 1. All experiments were performed with the approval of the Children's Hospital Boston Animal Ethics Committee.

### Hematopoietic Analysis

Single cell suspensions of fetal liver (FL) and bone marrow (BM) were prepared from embryos and adult mice, respectively. Cells were labeled with CD71 and Ter119, as well as 7-AAD. The CD71⁺Ter119⁺ populations were FACS-sorted as described (9). Immunohistochemistry (IHC) using an anti-HbF polyclonal antibody was performed on paraffin-embedded E16.5 fetal liver sections as described previously (38). Blood smears were prepared and stained with May-Grunwald-Giemsa. For assessment of bone marrow cellularity, whole bone marrow cells were isolated from adult animals 12-weeks after pIpC treatment and analyzed with markers for various cell lineages. All antibody conjugates and matched isotype controls were obtained from PharMingen or eBioscience. Flow cytometry was performed on a FACSCalibur™ (BD™ Biosciences), and sorting was performed on a FACSAria™ (BD™ Biosciences).

### Hematological Indices and Histopathology

Peripheral blood (PB) was isolated via the retro-orbital plexus and analyzed on a HEMAVET® HV950 multispecies hematology system (Drew Scientific, Inc.). Reticulocytes were labeled by Reti-COUNT reagent (Becton Diskinson) and quantified by flow cytometry. Urine osmolality was measured using a Fiske Model 210 Osmometer after the mice were deprived of food and water for 10-16 hours. F cells were analyzed by flow cytometry using the fetal hemoglobin test kit with FITC-conjugated monoclonal antibody directed to HbF (INVITROGEN™) and PE-conjugated monoclonal antibody directed to HbA (sc-21757, Santa Cruz Biotechnology Inc.). Tissue preparations from lung, liver, spleen, and kidney were fixed in Bouin's solution, embedded in paraffin, sectioned, and stained with hematoxylin-eosin by standard methods.

### Measurement of Red blood cells (RBC) lifespan

Red blood cells (RBCs) were labeled *in vivo* using Biotin N-hydroxysuccinimide (NHS), and the lifespan of circulating RBCs was measured as previously described (39). Briefly, a single dose of 50 mg/kg of NHS-Biotin was injected into 8-10 week old mice intravenously. Blood was collected from the tail at regular intervals and incubated with a fluorochrome-conjugated streptavidin. The percentage of biotinylated RBCs was calculated by determining the fraction of peripheral blood cells labeled with Ter119 that were also labeled with streptavidin by flow cytometry. FACS analyses were performed for up to 2 weeks after injection to measure the progressive clearance of the biotin-labeled RBCs from circulation.

### Cell Culture

Primary human CD34⁺ cells were obtained from Yale Center of Excellence in Molecular Hematology (YCEMH). *Ex vivo* erythroid maturation of human CD34⁺ cells was performed using a two-phase liquid culture system as previously described (10). Cells were initially cultured for expansion in StemSpan SFEM Medium (StemCell Technologies Inc.) supplied with 1 x CC100 cytokine mix (STEMCELL™ Technologies Inc.) and 2% penicillin-streptomycin (P/S) for 6 days. Cells were maintained at a density of 0.1∼1 x 10⁶ cells/ml with media changes every other day. On day 6, cells were pelleted and transferred into StemSpan SFEM Medium with 2% P/S, erythropoietin (1 U/ml), IL-3 (5 mg/ml), SCF (20 ng/ml), dexamethasone (2 µM), and β-estradiol (1 µM). Cells were incubated at 37°C with 5% CO² and maintained at a density of 0.1∼1 x 10⁶ cells/ml by supplementing cultures every other day with fresh media.

### Western Blot Analysis

Expression of BCL11A was assessed using antibody 14B5 and 15E3AC11 (Abcam Inc.), as described previously (9). Antibody against acetyl-Histone H3 (H3ac, 06-599) was obtained from MILLIPORE®, and antibody against histone H3 (ab1791) was obtained from Abcam. GAPDH (sc-25778, Santa Cruz Biotechnology Inc.) was analyzed as a loading control.

### Bisulfite sequencing

Bisulfite sequencing was performed as previously described (40). Sequences of PCR primers are listed in Table 1.

### Gene expression analysis

FACS-sorted CD71⁺Ter1 19⁺ erythroid cells was isolated from bone marrows of 6-week old mice. Total RNA was isolated by Trizol (INVITROGEN™), reverse-transcribed, labeled, and hybridized onto AFFYMETRIX® mouse genome 430 2.0 arrays. The microarray data were deposited in the Gene Expression Omnibus (see the World Wide Website of National Institute of Health) under accession number GSE28333. Model-based expression value of probe sets were calculated by dChip from raw AFFYMETRIX® CEL files, and QQ-normalization was then applied to normalize the expression value distribution among multiple arrays (41). Genes whose expression differed by more than 1.5-fold (or 2-fold) between two groups with a Student's *t*-test *P*-value less than 0.05 were considered differentially expressed. Gene ontology (GO) Biological Process analysis was performed by DAVID (42). For quantitative RT-PCR analysis, RNA was extracted using a QIAamp® RNA Blood Mini Kit or an RNeasy® Plus Mini Kit (QIAGEN®). Quantified RNA was reverse-transcribed using an iScript cDNA Synthesis Kit (BIO-RAD®). cDNA samples were analyzed in triplicate with the iQ™ SYBR® Green Supermix using the iCycler Real-Time PCR Detection System (BIO-RAD®). PCR amplification parameters were 95°C (3 min) and 45 cycles of 95°C (15 sec), 60°C (30 sec), and 72°C (30 sec).

### ChIP and ChIP-chip

ChIP was performed as previously described (10). ChIP-chip experiments were performed using AFFYMETRIX® human ENCODE 2.0R arrays as previously described (10). The ChIP signals are shown as percentages of the input DNA signals. Precipitated DNA samples were quantified using primers designed to amplify several control regions within the human β-globin locus as indicated below the graph. Results are means ± SD. *P < 0.01; **P < 0.001.

### Statistics

Statistical analysis was performed using two-tailed *t*-tests. P-values are indicated in the relevant figures.

### Results

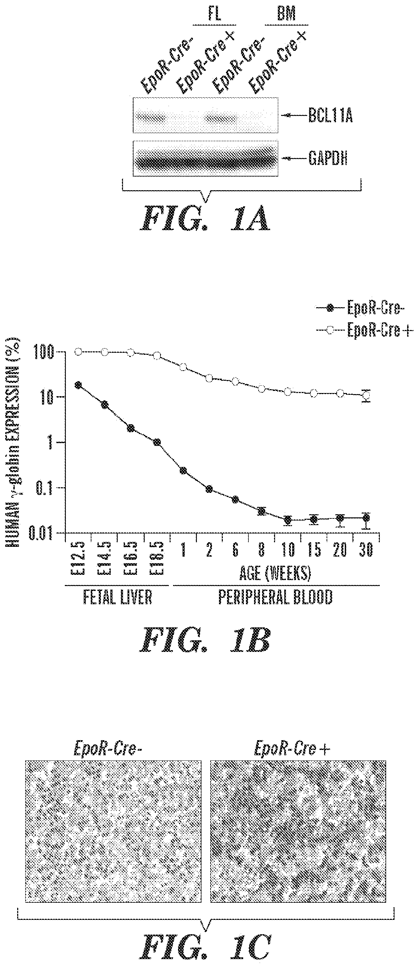

To address *in vivo* roles of BCL11A, stringent genetic tests were employed in mice carrying the human β-globin gene cluster as a yeast artificial chromosome transgene (β-YAC mice). Knockout of BCL11A interrupts silencing of endogenous β-like embryonic genes and human γ-globin genes in mouse fetal liver (9). As BCL11A-null mice are postnatally lethal, the inventors examined the contribution of BCL11A to γ-silencing in adults through conditional inactivation of BCL11A with erythroid-selective EpoR-GFP Cre alleles (13) (Figure 1A). Mice harboring erythroid-specific inactivation of BCL11A developed normally. Erythropoiesis in fetal liver and adult bone marrow appeared normal in the absence of BCL11A (Figure 4). As in the conventional knockout, hemoglobin switching failed to occur in fetal liver, such that γ constituted >80% of the β-like human globins (Figure 1B and Figure 5). HbF was robustly expressed in definitive erythroid cells of E16.5 fetal liver (Figure 1C and Figure 6). After birth, the level of γ-globin declined progressively to a residual level of ∼11% in 30-week and older adults (Figure 1B and Figure 5). Mouse embryonic β-like globin genes (εy and βh1) were also upregulated in BCL11A-null erythroid cells throughout development (Figure 7). The inventors further examined several experimental features (YAC copy number and time of Cre-mediated gene inactivation) that can contribute to the observed silencing and showed that they were non-contributory (Figure 8).

The inventors observed gradual silencing of'y-globin expression in BCL 1 1A-null erythroid cells. Upon erythroid-specific inactivation of BCL11A, expression of γ-globin was maintained at very high level in fetal livers, gradually declined after birth, and reached a steady level of ∼10-15% in adult mice (Figure 1B and Figure 5). To determine whether the gradual silencing of γ-globin expression in the BCL11A conditional knockout mice reflects the use of EpoR-Cre, which excises around the CFU-E stage of erythroid lineage commitment, the inventors inactivated BCL11A from the onset of hematopoiesis by use of Vav-Cre. Mice lacking BCL11A in all hematopoietic compartments were similarly viable. Again, human γ-globin genes and mouse embryonic β-like globin genes (εy and βh1) failed to silence properly in erythroid cells of both fetal liver and adult bone marrow. The levels of derepressed γ-globins were similar to that in the EpoR-Cre BCL11A conditional knockout mice at all ages (Figurea 8A-8C), indicating that inactivation during erythroid commitment is as (or nearly as) effective as excision much earlier in hematopoiesis. Moreover, these results demonstrate that BCL11A is functionally required for γ-silencing only at the CFU-E or later stages. The inventors next asked whether the gradual silencing is related to use of a β-locus mouse line that contains 3 copies of 13-locus YAC (30). The inventors conditionally inactivated BCL11A in an independently derived mouse line carrying a single copy of human β-YAC (A20 strain) (43). Erythroid-specific deletion of BCL11A in the single-copy β-YAC mice reactivated the expression of human γ-globin and mouse embryonic globin genes to the same level as observed in the 3- copy β-YAC mice (Figure 8D-8F) indicated that the observed partial silencing is not mouse strain-specific.

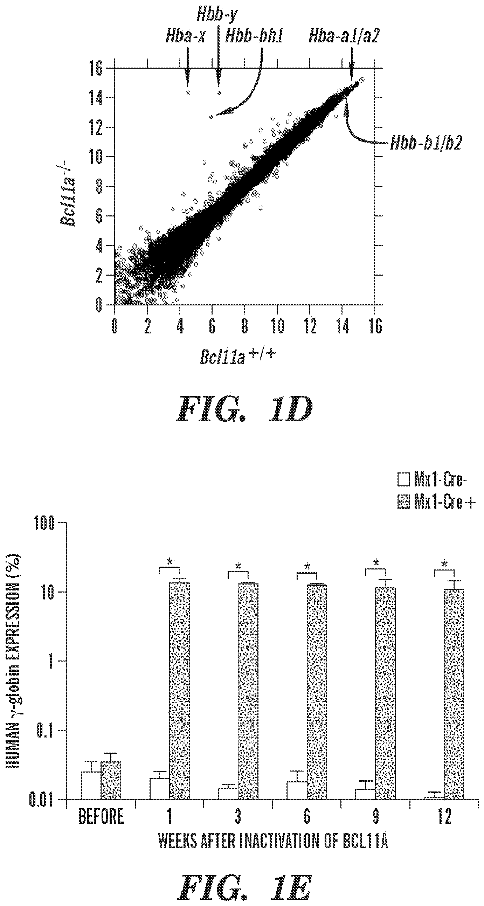

Transcriptional profiling of BCL11A-null erythroid cells purified from adult bone marrow was used to assess the quality of erythroid maturation. BCL11A-null and wild-type erythroid cells exhibited highly similar patterns of gene expression, characterized by a Pearson correlation coefficient (r2) of 0.9736 for the log₂ normalized intensities (Figure 1D). The expression of known erythroid transcriptional regulators, including GATA1, FOG1, NF-E2, KLF1, SOX6, and MYB, was comparable between the groups. The most differentially expressed genes were mouse embryonic β-like and α-like globin genes (Figure 1D; Figure 9; Tables 2 and 3). Thus, BCL11A is highly selective in controlling targets in erythroid cells, and only expression of the globin genes is substantially affected in its absence.

These findings establish roles for BCL11A in HbF silencing, but fail to demonstrate whether γ-globin genes that are fully silenced during normal development can be reactivated upon loss of BCL11A. Thus, the inventors introduced the interferon-inducible Mx1-Cre allele into BCL11A floxed β-YAC mice (Figure 10). Efficient excision of floxed BCL11A alleles in adult mice was not associated with significant changes in blood counts (Figure 11) except for a decline in total B-cells, consistent with a role in lymphopoiesis (14). Developmentally silenced γ-globins were reexpressed to 13.8% of total β-like human globins one week following inactivation of BCL11A and sustained thereafter (Figure 1E). As this level of γ-derepression closely approximates that in EpoR-Cre BCL11A conditional mice, the substantial component of HbF silencing dependent on BCL11A is reversible. Similarly, the mouse embryonic εy- and βh1-globin genes were derepressed on BCL11A loss (Figure 12).

The partial silencing of γ-globin expression in BCL11A-null erythroid cells points to additional silencing pathways that act independently of BCL11A. Two epigenetic pathways, DNA methylation and histone deacetylation, have been implicated in HbF control (15-17). DNA methylation of the γ-globin promoters progressively increased in BCL11A-null erythroid cells in correlation with the gradual, but partial, silencing of γ-globin expression (Figure 2A). Administration of the DNA methylation inhibitor 5-aza-2'-deoxycytidine (5-azaD) to normal β-YAC mice led to a very small increment in γ-globin mRNA (Figure 2B). In contrast, 5-azaD treatment was synergistic to BCL11A loss, leading to 37.9% γ-globin mRNA. By ChIP-chip analysis, the inventors observed that histone deacetylase 1 (HDAC1) occupies the γ-globin promoters in primary adult human erythroid precursors (Figure 2C). Notably, the binding peaks of HDAC1 overlap peaks of trimethyl-H3 Lys27, consistent with the very low-level expression of γ-globin genes in adult erythroid cells. Administration of an HDAC inhibitor (SAHA) also synergized with BCL11A in derepression of γ-globin mRNA (Figure 2D). The level of histone H3 acetylation was elevated in bone marrow cells and within γ-promoter regions after treatment (Figure 13), this indicated that the enhanced transcription of γ-globin genes is related to the increase in histone acetylation. These results demonstrated that loss of BCL11A markedly enhances the effects of DNA demethylation and HDAC inhibitors in reactivating HbF expression, and indicated that a combination of BCL11A downregulation with known HbF inducers can cause efficient HbF augmentation, as demonstrated herein.

The above findings illustrate favorable features of BCL11A as a target for reactivation of HbF in the β-hemoglobinopathies. HbF silencing in the adult is strongly, though not exclusively, dependent on BCL11A. Red cell production is largely unaffected by its absence. Moreover, loss of BCL11A enhances effects of known HbF inducers. The inventors next explored whether impairment of BCL11A is sufficient to ameliorate disease symptoms in a mouse model of SCD. Transgenic knockout mice expressing exclusively human sickle hemoglobin faithfully recapitulate the principal hematologic and histopathologic features of the human disease and are widely used to evaluate pharmacologic and genetic therapies (18-22). "Berkeley" SCD mice (19), which contain targeted deletions of murine α and β globins plus human fetal (^{G}γ, ^{A}γ) and adult (δ, β^{s}) globin transgenes in a normal genomic configuration and express low levels of HbF in adults, have been employed in the inventors' analysis.

The inventors introduced BCL11A-null alleles into SCD mice (SCD *Bcl11a*^{fl/fl}EpoR-Cre+, hereafter called *SCD*/*Bcl11a*^{*-*/*-*} mice). As typical human patients, SCD mice exhibit severe hemolytic anemia, reticulocytosis, and shortened RBC survival (Figures 3A, 3B and Table 4). Hematologic parameters, including RBC counts and hemoglobin (Hb) content, were corrected in *SCD*/*Bcl11a*^{*-*/*-*} mice (Table 4). Reticulocyte counts, red cell distribution width, numbers of white blood cells (WBCs) and platelets were near control values (Table 4 and Figure 14). In stark contrast to SCD mice, sickled cells were absent in *SCD*/*Bcl11a*^{*-*/*-*} mice (Fig. 3A), and red cell survival was markedly improved (Figure 3B). Accordingly, spleen size, a measure of compensatory erythropoiesis, was dramatically reduced (Fig. 3C). In humans and SCD mice, RBC sickling leads to reduced medullary blood flow and impairs urine-concentrating ability. Urine osmolality of *SCD*/*Bcl11a*^{*-*/*-*} mice was normal, heralding improved renal function (Table 1). Organ histopathology present in SCD mice was also reversed in *SCD*/*Bcl11a*^{*-*/*-*} mice (Figure 15). In addition to hemolytic anemia, SCD in humans and SCD mice leads to severe tissue damage, a major source of morbidity in patients with SCD. Tissue damage in multiple organs, including lung (vascular congestion), liver (ischemic infarcts and focal areas of necrosis), spleen (expansion of red pulp and congested sinusoidal channels), and kidney (fibrosis, cysts and infarcts), was markedly improved in SCD/Bcl11a^{-/-} mice. Representative sections of each group are shown. For lung, spleen, and kidney, 200x magnification; for liver, 100x magnification. All sections were stained with hematoxylin-eosin (Figure 15).

To relate these phenotypic findings to HbF reactivation, the steady-state level of γ-globin expression and cellular distribution of HbF in *SCD*/*Bel11a*^{*-*/*-*} mice was determined. Expression of y-globin genes was greatly elevated (P = 0.00017) in adult *SCD*/*Bcl11a*^{*-*/*-*} mice compared to SCD mice (28.3% vs 1.3% of total β-like human globins; Figure 3D). The cellular distribution of HbF in red cells was assessed by staining for F-cells using antibodies against HbF and HbA (which also recognizes HbS). Peripheral blood of control and SCD mice contained few F cells (3.6% and 7.2%, respectively; Fig. 3E). In contrast, peripheral blood of *SCD*/*Bcl11a*^{*-*/*-*} mice exhibited strong pancellular staining of HbF, and F-cells accounted for 85.1% of total RBCs (P = 3.9 x 10⁻⁵; Figure 3E). The level of HbF expression achieved in the absence of BCL11A greatly exceeds the estimated ∼15-20% HbF believed to be necessary to virtually eliminate SCD phenotypes in patients (4). Further, BCL11A-null alleles were introduced into an independent SCD mouse strain (22). Similar to the "Berkeley" SCD mice, inactivation of BCL11A corrected the sickle cell phenotypes due to HbF reactivation. Thus, the findings described herein are not limited to a singleSCD mouse model.

The correction of SCD in a mouse model by genetic manipulation of a single component involved in globin gene regulation constitutes a requisite step in translating new insights in HbF silencing into mechanism-based, improved therapy for the major hemoglobin disorders.

Persistence of human fetal hemoglobin (HbF, α2γ2) in adults lessens the severity of sickle cell disease (SCD) and the β-thalassemias. Herein the inventors demonstrated that the repressor BCL11A is required *in vivo* for silencing of γ-globin expression in adult animals, yet is dispensable for red cell production. BCL11A serves as a barrier to HbF reactivation by known HbF inducing agents. In a proof of principle test of BCL11A as a therapeutic target, the inventors demonstrated that inactivation of BCL11A in SCD transgenic mice corrected the hematologic and pathologic defects associated with SCD through high-level pancellular HbF induction. Thus, interference with HbF silencing by manipulation of a single target protein is sufficient to reverse SCD.

### References

1. A. P. Kraus, B. Koch, L. Burckett, Br Med J 1, 1434 (1961).
2. C. L. Conley, D. J. Weatherall, S. N. Richardson, M. K. Shepard, S. Charache, Blood 21, 261 (1963).
3. V. M. Ingram, Nature 178, 792 (1956).
4. O. S. Platt et al., N Engl J Med 330, 1639 (1994).
5. S. Menzel et al., Nat Genet 39, 1197 (2007).
6. M. Uda et al., Proc Natl Acad Sci U S A 105, 1620 (2008).
7. G. Lettre et al., Proc Natl Acad Sci U S A 105, 11869 (2008).
8. V. G. Sankaran et al., Science 322, 1839 (2008).
9. V. G. Sankaran et al., Nature 460, 1093 (2009).
10. J. Xu et al., Genes Dev 24, 783 (2010).
11. A. Wilber et al., Blood (2010).
12. A. Wilber, A. W. Nienhuis, D. A. Persons, Blood 117, 3945 (2011).
13. A. C. Heinrich, R. Pelanda, U. Klingmuller, Blood 104, 659 (2004).
14. P. Liu et al., Nat Immunol 4, 525 (2003).
15. J. DeSimone, P. Heller, L. Hall, D. Zwiers, Proc Natl Acad Sci USA 79, 4428 (1982).
16. T. J. Ley et al., N Engl J Med 307, 1469 (1982).
17. J. E. Bradner et al., Proc Natl Acad Sci USA 107, 12617 (2010).
18. T. M. Ryan, D. J. Ciavatta, T. M. Townes, Science 278, 873 (1997).
19. C. Paszty et al., Science 278, 876 (1997).
20. R. Pawliuk et al., Science 294, 2368 (2001).
21. M. E. Fabry et al., Blood 97, 410 (2001).
22. J. Hanna et al., Science 318, 1920 (2007).
23. J. B. Herrick, Arch Intern Med 6, 517 (1910).
24. D. M. Dykxhoorn, J. Lieberman, Cell 126, 231 (2006).
25. R. E. Lanford et al., Science 327, 198 (2010).
26. C. Li, P. Xiao, S. J. Gray, M. S. Weinberg, R. J. Samulski, Proc Natl Acad Sci U S A (2011).
27. K. L. Shaw, D. B. Kohn, Science Transl Med 3, 97ps36 (2011).
28. R. E. Moellering et al., Nature 462, 182 (2009).
29. A. N. Koehler, Curr Opin Chem Biol 14, 331 (2010).
30. K. R. Peterson et al., Proc Natl Acad Sci U S A 92, 5655 (1995).
31. S. Porcu et al., Blood 90, 4602 (1997).
32. P. Georgiades et al., Genesis 34, 251 (2002).
33. R. Kuhn, F. Schwenk, M. Aguet, K. Rajewsky, Science 269, 1427 (1995).
34. J. W. Rupon, S. Z. Wang, K. Gaensler, J. Lloyd, G. D. Ginder, Proc Natl Acad Sci U S A 103, 6617 (2006).
35. G. Imre, V. Gekeler, A. Leja, T. Beckers, M. Boehm, Cancer Res 66, 5409 (2006).
36. N. Li et al., Proc Natl Acad Sci U S A 105, 4796 (2008).
37. R. P. Hebbel et al., Blood 115, 2483 (2010).
38. J. W. Choi, Y. Kim, M. Fujino, M. Ito, Int J Hematol 74, 277 (2001).
39. V. G. Sankaran, S. H. Orkin, C. R. Walkley, Genes Dev 22, 463 (2008).
40. J. Xu et al., Genes Dev 23, 2824 (2009).
41. C. Li, W. H. Wong, Proc Natl Acad Sci U S A 98, 31 (2001).
42. G. Dennis, Jr. et al., Genome Biol 4, P3 (2003).
43. K. M. Gaensler, M. Kitamura, Y. W. Kan, Proc Natl Acad Sci USA 90, 11381 (1993).

## Claims

1. A pharmaceutical composition for increasing fetal hemoglobin levels in a mammal in need thereof, the composition comprising at least one inhibitor of BCL11A and at least one DNA methylation inhibitor in a pharmaceutically acceptable carrier;
wherein the inhibitor of BCL11A is:
(i) an antibody specific for BCL11A or an antigen-binding fragment thereof;
(ii) a small molecule, wherein said small molecule is an organic or inorganic compound having a molecular weight of less than about 10,000 grams per mole or a salt, or ester or other pharmaceutically acceptable form of said compound, a peptide, a peptidomimetic, an amino acid, an amino acid analog, a nucleotide, or a nucleotide analog; or
(iii) a BCL11A specific nucleic acid that is selected from the group consisting of an aptamer that binds BCL11A, a BCL11A specific RNA interference agent, or a vector encoding a BCL11A specific RNA interference agent, wherein said RNA interference agent comprises one or more of the nucleotide sequences selected from the group consisting of SEQ ID NO: 3-8.

2. A pharmaceutical composition according to Claim 1, wherein the small molecule is a heterorganic compound or an organometallic compound.

3. A pharmaceutical composition according to Claim 1 or 2, wherein the at least one DNA methylation inhibitor is 5-aza-2'-deoxycytidine.

4. A pharmaceutical composition according to any of Claims 1-3, for use in increasing fetal hemoglobin levels in a mammal in need thereof.

5. A pharmaceutical composition for use according to Claim 4, wherein said mammal has been diagnosed with or is at risk for a hemoglobinopathy; such as wherein said hemoglobinopathy is a 3-hemoglobinopathy, sickle cell disease, or 3-thalassemia.

6. A pharmaceutical composition according to any of Claims 1-3, for use in treating a blood disorder in a mammal in need thereof, whereby fetal hemoglobin expression is increased in said mammal, relative to fetal hemoglobin expression prior to said administration.

7. A pharmaceutical composition for use according to Claim 6, wherein the blood disorder is a hemoglobinopathy; such as wherein said hemoglobinopathy is a β-hemoglobinopathy, sickle cell disease, or 3-thalassemia.

8. A pharmaceutical composition for use according to Claim 6 or 7, wherein said treatment further comprises administering an additional therapeutic agent for the blood disorder.

9. A pharmaceutical composition for use according to any one of Claims 4-8, wherein the composition is formulated for administration to the mammal by injection, infusion, instillation, or ingestion.

10. An *ex vivo* or *in vitro* method for increasing fetal hemoglobin levels in a cell, the method comprising the steps of contacting a hematopoietic progenitor cell with an effective amount of a composition according to any of Claims 1-3, whereby fetal hemoglobin expression is increased in said cell, or its progeny, relative to fetal hemoglobin expression in said cell prior to said contacting.

11. A method according to Claim 10, wherein the hematopoietic progenitor cell is a cell of the erythroid lineage.

## Patentansprüche

1. Pharmazeutische Zusammensetzung zur Erhöhung von fetalen Hämoglobinspiegeln in einem Säugetier, das dieser bedarf, wobei die Zusammensetzung mindestens einen Hemmer von BCL11A und mindestens einen DNA-Methylierungshemmer in einem pharmazeutisch akzeptablen Trägerstoff umfasst;
wobei der Hemmer von BCL11A Folgendes ist:
(i) ein Antikörper, der für BCL11A spezifisch ist, oder ein antigenbindendes Fragment davon;
(ii) ein kleines Molekül, wobei das kleine Molekül eine organische oder anorganische Verbindung mit einem Molekulargewicht von weniger als etwa 10.000 Gramm pro Mol oder ein Salz oder ein Ester oder eine andere pharmazeutisch akzeptable Form der Verbindung, ein Peptid, ein Peptidomimetikum, eine Aminosäure, ein Aminosäuren-Analogon, ein Nukleotid oder ein Nukleotid-Analogon ist; oder
(iii) eine BCL11A-spezifische Nukleinsäure, die aus der Gruppe bestehend aus einem Aptamer, das BCL11A bindet, einem BCL11A-spezifischen RNA-Interferenz-Agens oder einem Vektor, der ein BCL11A-spezifisches RNA-Interferenz-Agens kodiert, ausgewählt ist, wobei das RNA-Interferenz-Agens eine oder mehrere der Nukleotidsequenzen umfasst, die aus der Gruppe bestehend aus den SEQ ID Nrn. 3-8 ausgewählt sind.

2. Pharmazeutische Zusammensetzung nach Anspruch 1, wobei das kleine Molekül eine heteroorganische Verbindung oder eine organometallische Verbindung ist.

3. Pharmazeutische Zusammensetzung nach Anspruch 1 oder 2, wobei der mindestens eine DNA-Methylierungshemmer 5-Aza-2'-desoxycytidin ist.

4. Pharmazeutische Zusammensetzung nach einem der Ansprüche 1-3 zur Verwendung bei der Erhöhung von fetalen Hämoglobinspiegeln in einem Säugetier, das dieser bedarf.

5. Pharmazeutische Zusammensetzung zur Verwendung nach Anspruch 4, wobei das Säugetier mit einer Hämoglobinopathie diagnostiziert wurde und ein Risiko für eine Hämoglobinopathie aufweist; wie wobei die Hämoglobinopathie eine β-Hämoglobinopathie, Sichelzellkrankheit oder β-Thalassämie ist.

6. Pharmazeutische Zusammensetzung nach einem der Ansprüche 1-3 zur Verwendung bei der Behandlung einer Bluterkrankung in einem Säugetier, das dieser bedarf, wodurch die Expression von fetalem Hämoglobin in dem Säugetier im Vergleich zu der Expression von fetalem Hämoglobin vor der Verabreichung erhöht wird.

7. Pharmazeutische Zusammensetzung zur Verwendung nach Anspruch 6, wobei die Bluterkrankung eine Hämoglobinopathie ist; wie wobei die Hämoglobinopathie eine β-Hämoglobinopathie, Sichelzellkrankheit oder β-Thalassämie ist.

8. Pharmazeutische Zusammensetzung zur Verwendung nach Anspruch 6 oder 7, wobei die Behandlung weiterhin ein Verabreichen eines zusätzlichen Therapeutikums für die Bluterkrankung umfasst.

9. Pharmazeutische Zusammensetzung zur Verwendung nach einem der Ansprüche 4-8, wobei die Zusammensetzung zur Verabreichung an das Säugetier mittels Injektion, Infusion, Instillation oder Ingestion formuliert ist.

10. *Ex-vivo-* oder *In-vitro*-Verfahren zur Erhöhung von fetalen Hämoglobinspiegeln in einer Zelle, wobei das Verfahren die Schritte eines Inkontaktbringens einer hämatopoetischen Progenitorzelle mit einer wirksamen Menge einer Zusammensetzung nach einem der Ansprüche 1-3 umfasst, wodurch die Expression von fetalem Hämoglobin in der Zelle oder ihrem Abkömmling im Vergleich zu der Expression von fetalem Hämoglobin in der Zelle vor dem Inkontaktbringen erhöht wird.

11. Verfahren nach Anspruch 10, wobei die hämatopoetische Progenitorzelle eine Zelle der erythroiden Linie ist.

## Revendications

1. Composition pharmaceutique pour l'augmentation des niveaux d'hémoglobine foetale chez un mammifère qui en a besoin, la composition comprenant au moins un inhibiteur du BCL11A et au moins un inhibiteur de la méthylation de l'ADN dans un support pharmaceutiquement acceptable ;
dans laquelle l'inhibiteur du BCL11A est :
(i) un anticorps spécifique du BCL11A ou un fragment de liaison à un antigène de celui-ci ;
(ii) une petite molécule, dans laquelle ladite petite molécule est un composé organique ou inorganique ayant un poids moléculaire de moins d'environ 10 000 grammes par mole ou un sel, ou un ester ou une autre forme pharmaceutiquement acceptable dudit composé, un peptide, un peptidomimétique, un acide aminé, un analogue d'acide aminé, un nucléotide, ou un analogue de nucléotide ; ou
(iii) un acide nucléique spécifique du BCL11A qui est sélectionné parmi le groupe constitué par un aptamère qui lie le BCL11A, un agent d'interférence à ARN spécifique du BCL11A, ou un vecteur codant pour un agent d'interférence à ARN spécifique du BCL11A, dans laquelle ledit agent d'interférence à ARN comprend une ou plusieurs séquences de nucléotides sélectionnées dans le groupe constitué par les SEQ ID NO : 3 à 8.

2. Composition pharmaceutique selon la revendication 1, dans laquelle la petite molécule est un composé hétérorganique ou un composé organométallique.

3. Composition pharmaceutique selon la revendication 1 ou 2, dans laquelle l'au moins un inhibiteur de la méthylation de l'ADN est la 5-aza-2'-désoxycytidine.

4. Composition pharmaceutique selon l'une quelconque des revendications 1 à 3, destinée à une utilisation dans l'augmentation des niveaux d'hémoglobine foetale chez un mammifère qui en a besoin.

5. Composition pharmaceutique destinée à une utilisation selon la revendication 4, dans laquelle ledit mammifère a reçu un diagnostic d'hémoglobinopathie ou risque de souffrir d'hémoglobinopathie ; de sorte que dans laquelle ladite hémoglobinopathie est une β-hémoglobinopathie, une drépanocytose, ou une β-thalassémie.

6. Composition pharmaceutique selon l'une quelconque des revendications 1 à 3, destinée à une utilisation dans le traitement d'un trouble sanguin chez un mammifère qui en a besoin, par quoi l'expression de l'hémoglobine foetale est augmentée chez ledit mammifère, par rapport à l'expression de l'hémoglobine foetale avant ladite administration.

7. Composition pharmaceutique destinée à une utilisation selon la revendication 6, dans laquelle le trouble sanguin est une hémoglobinopathie ; de sorte que dans laquelle ladite hémoglobinopathie est une β-hémoglobinopathie, une drépanocytose, ou une β-thalassémie.

8. Composition pharmaceutique destinée à une utilisation selon la revendication 6 ou 7, dans laquelle ledit traitement comprend en outre l'administration d'un agent thérapeutique supplémentaire pour le trouble sanguin.

9. Composition pharmaceutique destinée à une utilisation selon l'une quelconque des revendications 4 à 8, dans laquelle la composition est présentée pour l'administration au mammifère par injection, perfusion, instillation, ou ingestion.

10. Procédé ex vivo ou in vitro pour l'augmentation des niveaux d'hémoglobine foetale dans une cellule, le procédé comprenant les étapes consistant à mettre en contact une cellule progénitrice hématopoïétique avec une quantité efficace d'une composition selon l'une quelconque des revendications 1 à 3, par quoi l'expression de l'hémoglobine foetale est augmentée dans ladite cellule, ou sa progéniture, par rapport à l'expression de l'hémoglobine foetale dans ladite cellule avant ladite mise en contact.

11. Procédé selon la revendication 10, dans lequel la cellule progénitrice hématopoïétique est une cellule du lignage érythroïde.
